# EUROPEAN PATENT APPLICATION

(11) **EP 1 897 879 A2**
(43) Date of publication of application: **12.03.2008**
(21) Application number: 07150020.1
(22) Date of filing: 30.03.2006
(51) Int. Cl.: C07D 413/12, C07D 413/14, A61K 31/445

(54) **2,4,5 substituted piperidines as renin inhibitors**

(30) Priority: 31.03.2005 US 666556 P; 16.12.2005 US 750853 P
(62) Divisional of application: 06725445.8
(71) Applicant: Speedel Experimenta AG, 4123 Allschwil (CH)
(72) Inventor: Herold, Peter, 4123, Allschwil (CH); Mah, Robert, 4123, Allschwil (CH); Tschinke, Vincenzo, 4123, Allschwil (CH); Marti, Christiane, 4123, Allschwil (CH); Stutz, Stefan, 4123, Allschwil (CH); Jelakovic, Stjepan, 4123, Allschwil (CH); Hollinger, Frank, Wayne, PA 19087 (US); Konteatis, Zenon D., Chatham Township, NJ 07928 (US); Ludington, Jennifer L., Blue Bell, PA 19422 (US); Quirmbach, Michael, 4054, Basel (CH); Stojanovic, Aleksandar, 4123, Allschwil (CH); Behnke, Dirk, 4123, Allschwil (CH)
(74) Representative: Maué, Paul Georg

(57) **Abstract**

Novel substituted piperidines of the general formulae (I) and (II) 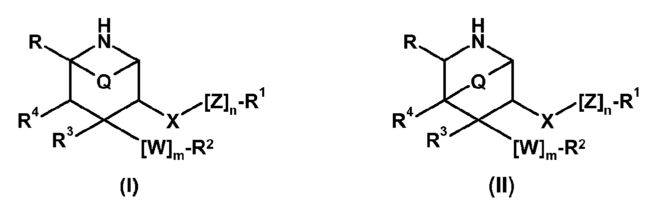 with the substituent definitions as explained in detail in the description are described. The compounds are suitable in particular as renin inhibitors and are highly potent.

## Description

### Field of the Invention

The present invention relates to novel substituted piperidines, to processes for their preparation and to the use of the compounds as medicaments, in particular as renin inhibitors.

### Background of the Invention

Piperidine derivatives for use as medicaments are disclosed, for example, by WO 97/09311. However, with regard especially to renin inhibition, there is still a need for highly potent active ingredients. In this context, the improvement of the pharmacokinetic properties is at the forefront. These properties directed to better bioavailability are, for example, absorption, metabolic stability, solubility or lipophilicity.

### Detailed Description of the Invention

The invention therefore provides substituted piperidines of the general formulae (I) and (II) 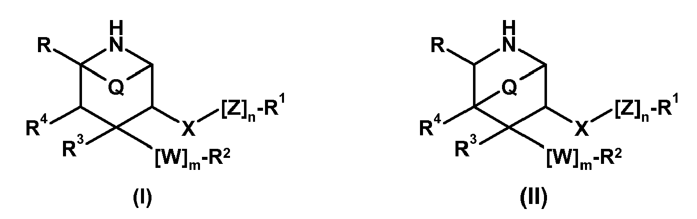 where
R is C₂₋₈-alkenyl, C₁₋₈-alkyl, C₂₋₈-alkynyl, C₀₋₈-alkyl-carbonyl-amino-C₁₋₈-alkyl, C₃₋₈-cycloalkyl-C₀₋₈-alkyl, C₁₋₈-alkyl-sulfonyl-C₁₋₈-alkyl, optionally N-mono- or N,N-di-C₁₋₈-alkylated carbamoyl-C₀₋₈-alkyl, optionally O-C₁₋₈-alkylated carboxyl-C₀₋₈-alkyl, optionally N and/or N' mono-, di- or tri-C₁₋₈-alkylated ureido-C₁₋₈-alkyl or heterocyclyl-carbonyl-C₀₋₈-alkyl, each of said radicals being substituted by 1-4 C₁₋₈-alkoxy, or hydroxyl;
R¹ is aryl or heterocyclyl;
R² is acenaphthyl, cyclohexyl, diazinyl, furyl, imidazolyl, naphthyl, oxadiazolyl, oxazolyl, phenyl, pyrazinyl, pyridyl, pyrimidinyl, pyrrolyl, oxopyridinyl, tetrazolyl, thienyl, or triazolyl, each of said radicals may be substituted by 1-3 C₁₋₈-alkanoyloxy-C₁₋₈-alkyl, C₂₋₈-alkenyloxy, C₁₋₈-alkoxy, C₁₋₈-alkoxy-C₁₋₈-alkoxy, C₁₋₈-alkoxy-C₁₋₈-alkoxy-C₁₋₈-alkyl, C₁₋₈-alkoxy-C₁₋₈-alkyl, C₁₋₈-alkoxycarbonyl, C₁₋₈-alkoxycarbonyloxy-C₁₋₈-alkyl, C₁₋₈-alkyl, carboxy-C₁₋₈-alkyl, C₁₋₈-alkoxy-C₁₋₈-alkylsulfanyl, C₁₋₈-alkylsulfanyl, C₁₋₈-alkylsulfanyl-C₁₋₈-alkoxy, C₁₋₈-alkylsulfanyl-C₁₋₈-alkoxy-C₁₋₈-alkyl, C₁₋₈-alkylsulfanyl-C₁₋₈-alkyl, cyano, cyano-C₁₋₈-alkyl, C₃₋₈-cycloalkyl-C₀₋₆-alkoxy-C₁₋₈-alkoxy, C₁₋₆-alkoxy-C₀₋₆-alkyl-C₃₋₈-cycloalkyl-C₀₋₆-alkoxy-C₁₋₈-alkyl, C₃₋₈-cycloalkyl-C₀₋₆-alkoxy-C₁₋₈-alkyl, halo-C₁₋₈-alkyl, halogen, hydroxy-C₁₋₈-alkyl, hydroxyl, oxide, trifluoromethoxy or trifluoromethyl groups, or a C₁₋₈-alkylenedioxy group, and/or by an L1-T1-L2-T2-L3-T3-L4-T4-L5-U radical;
L1, L2, L3, L4 and L5 are each independently a bond, C₁₋₈-alkylene, C₂₋₈-alkenylene or C₂₋₈-alkynylene, C₃₋₈-cycloalkene or are absent;
T1, T2, T3 and T4 are each independently
   (a) a bond, or are absent, or are one of the groups
   (b) -CH(OH)-
   (c) -CH(OR⁶)-
   (d) -CH(NR⁵R⁶)-
   (e) -CO-
   (f) -CR⁷R⁸-
   (g) -O- or -NR⁶-
   (h) -S(O)₀₋₂-
   (i) -SO₂NR⁶-
   (j) -NR⁶SO₂-
   (k) -CONR⁶-
   (l) -NR⁶CO-
   (m) -O-CO-
   (n) -CO-O-
   (o) -O-CO-O-
   (p) -O-CO-NR⁶-
   (q) -N(R⁶)-CO-N(R⁶)-
   (r) -N(R⁶)-CO-O-
   (s) pyrrolidinylene, piperidinylene or piperazinylene
   (t) -C(R¹¹)(R¹²)-,
where the bonds starting from (b)-(t) lead to a saturated or aromatic carbon atom of the adjacent group if the bond starts from a heteroatom, and where not more than two (b)-(f) groups, three (g)-(h) groups and one (i)-(t) group are present;
R³ is hydrogen, hydroxyl, C₁₋₈-alkoxy or C₂₋₈-alkenyloxy;
R⁴ is hydrogen, C₂₋₈-alkenyl, C₁₋₈-alkoxy, C₁₋₈-alkoxy-C₁₋₈-alkyl, C₁₋₈-alkoxy-C₁₋₈-alkoxy, C₁₋₈-alkyl, optionally (N-C₁₋₈-alkyl)-C₁₋₈-alkoxycarbonyl-amino-C₁₋₈-alkoxy, optionally (N-C₁₋₈-alkyl)-C₁₋₈-alkylcarbonyl-amino-C₁₋₈-alkoxy, optionally (N-mono- or N,N-di-C₁-C₈-alkyl)-amino-C₁₋₈-alkoxy, benzyl, C₃₋₈-cycloalkyloxy, C₃₋₈-cycloalkyloxy-C₁₋₈-alkoxy, heterocyclyl-C₀₋₈-alkoxy, heterocyclyloxy-C₁₋₈-alkoxy, hydroxy, hydroxy-C₁₋₈-alkoxy-C₁₋₈-alkoxy, hydroxy-C₁₋₈-alkyl, oxo or a
R^{4a}-Z1-X1- group where R^{4a} is
   (a) H-
   (b) C₁₋₈-alkyl-
   (c) C₂₋₈-alkenyl-
   (d) hydroxy-C₁₋₈-alkyl-
   (e) polyhydroxy-C₁₋₈-alkyl-
   (f) C₁₋₈-alkyl-O-C₁₋₈-alkyl-
   (g) aryl-
   (h) heterocyclyl-
   (i) arylalkyl-
   (j) heterocyclylalkyl-
   (k) aryloxyalkyl-
   (l) heterocyclyloxyalkyl-
   (m) (R⁵,R⁶)N-(CH₂)₁₋₃-
   (n) (R⁵,R⁶)N-
   (o) Cₗ-₈-alkyl-S(0)₀-₂-
   (p) aryl-S(0)₀-₂-
   (q) heterocyclyl-S(O)₀₋₂-
   (r) HO-SO₃- or salts thereof
   (s) H₂N-C(NH)-NH-
   (t) NC-
and the bonds starting from (n)-(t) lead to a carbon atom of the adjacent group and this carbon atom is saturated if the bond starts from a heteroatom;
Z1
   (a) is a bond, is absent, or is one of the groups
   (b) -C₁₋₈-alkylene-
   (c) -C₂₋₈-alkenylene-
   (d) -O-, -N(R¹¹)-, -S(O)₀₋₂-
   (e) -CO-
   (f) -O-CO-
   (g) -O-CO-O-
   (h) -O-CO-N(R¹¹)-
   (i) -N(R¹¹)-CO-O-
   (j) -CO-N(R¹¹)-
   (k) -N(R¹¹)-CO-
   (l) -N(R¹¹)-CO-N(R¹¹)-
   (m) -CH(OR⁹)-
   and the bonds starting from (d) and (f)-(m) lead to a carbon atom of the adjacent group and this carbon atom is saturated if the bond starts from a heteroatom;
X1
   (a) is a bond, is absent, or is one of the groups
   (b) -O-
   (c) -N(R¹¹)-
   (d) -S(O)₀₋₂-
   (e) -(CH₂)₁₋₃-;
   or R³ and R⁴ in formula (I) together are a bond;
   R⁵ and R⁶ are each independently hydrogen, C₁₋₈-alkyl, C₂₋₈-alkenyl, aryl-C₁₋₈-alkyl or acyl, or, together with the nitrogen atom to which they are bonded, are a 5- or 6-membered heterocyclic ring which may contain an additional nitrogen, oxygen or sulfur atom or a -SO- or -SO₂- group, and the additional nitrogen atom may optionally be substituted by C₁₋₈-alkyl radicals;
   R⁷ and R⁸, together with the carbon atom to which they are bonded, are a 3-7-membered ring which may contain one or two -O- or -S - atoms or -SO- or -SO₂-groups;
   R⁹ is hydrogen, C₁₋₈-alkyl, C₁₋₈-alkoxy-C₁₋₈-alkyl, acyl or arylalkyl;
   R¹⁰ is carboxyalkyl, alkoxycarbonylalkyl, alkyl or hydrogen;
   R¹¹ is hydrogen or C₁₋₈-alkyl;
   R¹² is hydrogen or C₁₋₈-alkyl;
   Q is ethylene or is absent (formula I) or is ethylene or methylene (formula II);
   U is hydrogen, C₁₋₈-alkyl, cyano, optionally substituted C₃₋₈-cycloalkyl, aryl or heterocyclyl; W is oxygen or sulfur;
   X is a bond, oxygen or sulfur, or is a >CH-R¹¹, >CHOR⁹, -O-CO-, >CO, >C=NOR¹⁰,
   -O-CHR¹¹- or -O-CHR¹¹-CO-NR⁹- group and the bond starting from an oxygen or sulfur atom leads to a saturated carbon atom of the Z group or to R¹;
   Z is C₁₋₈-alkylene, C₂₋₈-alkenylene, hydroxy-C₁₋₈-alkylidene, -O-, -S-, -O-alk-, -S-alk-, -alk-O-, -alk-S- or -alk-NR⁹-, where alk is C₁₋₈-alkylene; and where
      (a) if Z is -O- or -S-, X is >CH-R¹¹ and either R² contains an L1-T1-L2-T2-L3-T3-L4-T4-L5-U substituent or R⁴ is a substituent other than hydrogen as defined above;
      (b) if Z is -O-alk- or -S-alk-, X is >CH-R¹¹; and
      (c) if X is a bond, Z is C₂₋₈-alkenylene, -alk-O- or -alk-S-;

m is 0 or 1;
n is 0 or 1;
and salts thereof.

Examples of alkyl and alkoxy radicals, which may be linear or branched, are C₁₋₈-alkyl and C₁₋₈-alkoxy radicals such as methyl, ethyl, n-propyl, isopropyl, n-butyl, isobutyl, sec-butyl, tert-butyl, pentyl, hexyl, and methoxy, ethoxy, propoxy, isopropoxy, butoxy, isobutoxy, sec-butoxy and tert-butoxy respectively, in addition C₀-alkoxy designates -O-. C₁₋₈-alkylenedioxy radicals are preferably methylenedioxy, ethylenedioxy and propylenedioxy. Examples of C₁₋₈-alkanoyl radicals, which may be linear or branched, are acetyl, propionyl and butyryl. Cycloalkyl is a saturated, cyclic hydrocarbon radical having 3-12 carbon atoms, i.e. cyclopropyl, cyclobutyl, cyclopentyl, cyclohexyl, cycloheptyl, bicyclo[2.2.1]heptyl, cyclooctyl, bicyclo[2.2.2]octyl and adamantyl. C₁₋₈-alkylene radicals, which may be linear or branched, are, for example, methylene, ethylene, propylene, 2-methylpropylene, 2-methylbutylene, 2-methylbutyl-2-ene, butyl-2-ene, butyl-3-ene, propyl-2-ene, tetra-, penta- and hexamethylene. C₂₋₈-alkenylene radicals, which may be linear or branched, are, for example, vinylene and propenylene. C₂₋₈-alkynylene radicals, which may be linear or branched, are, for example, ethynylene; acyl radicals are alkanoyl radicals, preferably C₁₋₈-alkanoyl radicals, or aroyl radicals such as benzoyl. Aryl denotes mono- or polycyclic aromatic radicals which may be mono- or polysubstituted, for example phenyl, substituted phenyl, naphthyl, substituted naphthyl, tetrahydronaphthyl or substituted tetrahydronaphthyl. Examples of substituents on such aryl radicals or on heterocyclyl radicals are C₁₋₈-alkyl, trifluoromethyl, trifluoromethoxy, nitro, amino, C₂₋₆-alkenyl, C₁₋₈-alkoxy, C₁₋₈-alkylsulfinyl, C₁₋₈-alkylcarbonyloxy, hydroxyl, halogen, cyano, carbamoyl, carboxyl and C₁₋₈-alkylenedioxy, and also phenyl, phenoxy, phenylthio, phenyl-C₁₋₈-alkyl or phenyl-C₁₋₈-alkoxy each optionally substituted by halogen, C₁₋₈-alkyl, C₁₋₈-alkoxy or dihydroxy-C₁₋₈-alkylaminocarbonyl. Further examples of substituents on aryl or on heterocyclyl radicals are oxo, C₁₋₈-alkoxycarbonylphenyl, hydroxy-C₁₋₈-alkylphenyl, benzyloxy, pyridylcarbonylamino-C₁₋₈-alkyl, C₂₋₆-alkenyloxy, C₁₋₈-alkoxy-C₁₋₈-alkoxy, C₁₋₈-alkoxy-C₁₋₈-alkoxy-C₁₋₈-alkyl, hydroxy-C₁₋₈-alkoxy, di-C₁₋₈-alkylamino, 2,3-dihydroxypropoxy, 2,3-dihydroxypropoxy-C₁₋₈-alkoxy, 2,3-dimethoxypropoxy, methoxybenzyloxy, hydroxybenzyloxy, phenethyloxy, methylenedioxybenzyloxy, dioxolanyl-C₁₋₈-alkoxy, cyclopropyl-C₁₋₈-alkyl, cyclopropyl-C₁₋₈-alkoxy, hydroxy-C₁₋₈-alkoxy, carbamoyloxy-C₁₋₈-alkoxy, pyridylcarbamoyloxy-C₁₋₈-alkoxy, benzoyloxy-C₁₋₈-alkoxy, picolyloxy, C₁₋₈-alkoxycarbonyl, C₀₋₆-alkylcarbonylamino, C₀₋₆-alkylcarbonylamino-C₁₋₈-alkyl, C₀₋₆-alkylcarbonylamino-C₁₋₈-alkoxy, (N-C₁₋₈-alkyl)-C₀₋₆-alkylcarbonylamino-C₁₋₈-alkyl, (N-C₁₋₈-alkyl)-C₀₋₆-alkylcarbonylamino-C₁₋₈-alkoxy, C₃₋₈-cycloalkylcarbonylamino-C₁₋₈-alkyl, C₃₋₈-cycloalkylcarbonylamino-C₁₋₈-alkoxy, C₁₋₈-alkoxy-C₁₋₈-alkyl, hydroxy-C₁₋₈-alkyl, hydroxy-C₁₋₈-alkoxy-C₁₋₈-alkyl, hydroxy-C₁₋₈-alkoxy-C₁₋₈-alkoxy, C₁₋₈-alkoxycarbonylamino-C₁₋₈-alkyl, C₁₋₈-alkoxycarbonylamino-C₁₋₈-alkoxy, C₁₋₈-alkylaminocarbonylamino-C₁₋₈-alkyl, C₁₋₈-alkylaminocarbonylamino-C₁₋₈-alkoxy, C₁₋₈-alkylaminocarbonyl-C₁₋₈-alkyl, C₁₋₈-alkylaminocarbonyl-C₁₋₈-alkoxy, C₁₋₈-alkylaminocarbonyl-C₁₋₈-alkoxy-C₁₋₈-alkyl, di-C₁₋₈-alkylaminocarbonyl-C₁₋₈-alkyl, di-C₁₋₈-alkylaminocarbonyl-C₁₋₈-alkoxy, C₁₋₈-alkoxycarbonyloxy-C₁₋₈-alkyl, C₁₋₈-alkylcarbonyloxy-C₁₋₈-alkyl, C₁₋₈-alkylcarbonyloxy-C₁₋₈-alkoxy, cyano-C₁₋₈-alkyl, cyano-C₁₋₈-alkoxy, 2-oxooxazolidinyl-C₁₋₈-alkyl, 2-oxooxazolidinyl-C₁₋₈-alkoxy, C₁₋₈-alkoxycarbonyl-C₁₋₈-alkyl, C₁₋₈-alkoxycarbonyl-C₁₋₈-alkoxy, C₁₋₈-alkylsulfonylamino-C₁₋₈-alkyl, C₁₋₈-alkylsulfonylamino-C₁₋₈-alkoxy, (N-C₁₋₈-alkyl)-C₁₋₈-alkylsulfonylamino-C₁₋₈-alkyl, (N-C₁₋₈-alkyl)-C₁₋₈-alkylsulfonylamino-C₁₋₈-alkoxy, amino-C₁₋₈-alkyl, amino-C₁₋₈-alkoxy, C₁₋₈-alkylamino-C₁₋₈-alkyl, C₁₋₈-alkylamino-C₁₋₈-alkoxy, di-C₁₋₈-alkylamino-C₁₋₈-alkyl, di-C₁₋₈-alkylamino-C₁₋₈-alkoxy, C₁₋₈-alkylsulfonyl-C₁₋₈-alkyl, C₁₋₈-alkylsulfonyl-C₁₋₈-alkoxy, carboxy-C₁₋₈-alkyl, carboxy-C₁₋₈-alkoxy, carboxy-C₁₋₈-alkoxy-C₁₋₈-alkyl, C₁₋₈-alkoxy-C₁₋₈-alkylcarbonyl, acyl-C₁₋₈-alkoxy-C₁₋₈-alkyl, (N-C₁₋₈-alkyl)-C₁₋₈-alkoxycarbonylamino, (N-hydroxy)-C₁₋₈-alkylaminocarbonyl-C₁₋₈-alkyl, (N-hydroxy)-C₁₋₈-alkylaminocarbonyl-C₁₋₈-alkoxy, (N-hydroxy)aminocarbonyl-C₁₋₈-alkyl, (N-hydroxy)aminocarbonyl-C₁₋₈-alkoxy, C₁₋₈-alkoxyaminocarbonyl-C₁₋₈-alkyl, C₁₋₈-alkoxyaminocarbonyl-C₁₋₈-alkoxy, (N-C₁₋₈-alkoxy)-C₁₋₈-alkylaminocarbonyl-C₁₋₈-alkyl, (N-C₁₋₈-alkoxy)-C₁₋₈-alkylaminocarbonyl-C₁₋₈-alkoxy, (N-acyl)-C₁₋₈-alkoxy-C₁₋₈-alkylamino, C₁₋₈-alkoxy-C₁₋₈-alkylcarbamoyl, (N-C₁₋₈-alkyl)-C₁₋₈-alkoxy-C₁₋₈-alkylcarbamoyl, C₁₋₈-alkoxy-C₁₋₈-alkylcarbonyl, C₁₋₈-alkoxy-C₁₋₈-alkylcarbonylamino, (N-C₁₋₈-alkyl)-C₁₋₈-alkoxy-C₁₋₈-alkylcarbonylamino, 1-C₁₋₈-alkoxy-C₁₋₈-alkylimidazol-2-yl, 1-C₁₋₈-alkoxy-C₁₋₈-alkyltetrazol-5-yl, 5-C₁₋₈-alkoxy-C₁₋₈-alkyltetrazol-1-yl, 2-C₁₋₈-alkoxy-C₁₋₈-alkyl-4-oxo-imidazol-1-yl, carbamoyl-C₁₋₈-alkyl, carbamoyl-C₁₋₈-alkoxy, C₁₋₈-alkylcarbamoyl, di-C₁₋₈-alkylcarbamoyl, C₁₋₈-alkylsulfonyl, C₁₋₈-alkylamidinyl, acetamidinyl-C₁₋₈-alkyl, O-methyloximyl-C₁₋₈-alkyl, O,N-dimethylhydroxylamino-C₁₋₈-alkyl, C₃₋₈-cycloalkyl-C₁₋₈-alkanoyl, aryl-C₁₋₈-alkanoyl, heterocyclyl-C₁₋₈-alkanoyl; and also pyridyl, pyridyloxy, pyridylthio, pyridylamino, pyridyl-C₁₋₈-alkyl, pyridyl-C₁₋₈-alkoxy, pyrimidinyl, pyrimidinyloxy, pyrimidinylthio, pyrimidinylamino, pyrimidinyl-C₁₋₈-alkyl, pyrimidinyl-C₁₋₈-alkoxy, thienyl, thienyl-C₁₋₈-alkyl, thienyl-C₁₋₈-alkoxy, furyl, furyl-C₁₋₈-alkyl, furyl-C₁₋₈-alkoxy each optionally substituted by halogen, C₁₋₈-alkyl, C₁₋₈-alkoxy or dihydroxy-C₁₋₈-alkylaminocarbonyl.

The term heterocyclyl denotes a saturated or unsaturated, 4-8-membered, particularly preferably 5- or 6-membered, monocyclic ring system, a saturated or unsaturated, 7-12-membered, particularly preferably 9- or 10-membered, bicyclic ring system and also a saturated or unsaturated, 7-12-membered tricyclic ring system, in each case having from 1 to 4 nitrogen and/or 1 or 2 sulfur or oxygen atoms and comprising an N, O or S atom in at least one ring, it also being possible for additional N, O or S atoms to be present in the same ring. Said radicals may be unsubstituted or substituted one or more times, e.g. once or twice. It also being possible for a plurality of identical or different substituents to be present. Preferred substituents are (in the case of unsaturated heterocyclyl radicals) alkyl, hydroxyl, alkoxy, cyano, oxide, nitrogen. halogen, and substituents as defined above for aryl radicals, or (in the case of saturated heterocyclyl radicals) alkyl and alkoxy. Examples of heterocyclyl radicals are pyridyl, thienyl, pyrazinyl, triazolyl, imidazolyl, benzothiazolyl, furyl, pyranyl, tetrahydropyranyl, azetidinyl, pyrimidinyl, morpholinyl, quinazolinyl, quinolyl, quinoxalinyl, isoquinolyl, tetrahydroquinolyl, tetrahydroisoquinolyl, benzo[b]thienyl, isobenzofuranyl, benzoimidazolyl, 2-oxobenzoimidazolyl, oxazolyl, thiazolyl, indolyl, 2-oxo-1H-quinolinyl, 2H-chromenyl, 2-oxo-2H-chromenyl, 1,1a,2,7b-tetrahydrocyclopropa[c]chromenyl, 2-oxo-1a,7b-dihydro-1H-cyclopropa[c]chromenyl, pyrrolyl, 2-oxodihydrobenzo[d][1,3]oxazinyl, 4-oxodihydroimidazolyl, 5-oxo-4H-[1,2,4]triazinyl, 3-oxo-4H-benzo[1,4]thiazinyl, tetrahydroquinoxalinyl, 1,1,3-trioxodihydro-2H-1λ⁶-benzo[1,4]thiazinyl, 1-oxopyridyl, dihydro-2H-benzo[1,4]oxazinyl, 2-oxotetrahydro-benzo[e][1,4]diazepinyl, 2-oxodihydrobenzo[e][1,4]diazepinyl, 1H-pyrrolizinyl, phthalazinyl, 1-oxo-3H-isobenzofuranyl, 4-oxo-3H-thieno[2,3-d]pyrimidinyl, 3-oxo-4H-benzo[1,4]oxazinyl, [1,5]naphthyridyl, dihydro-2H-benzo[1,4]thiazinyl, 1,1-dioxodihydro-2H-benzo[1,4]thiazinyl, 2-oxo-1 H-pyrido[2,3-b][1,4]oxazinyl, dihydro-1 H-pyrido[2,3-b][1,4]oxazinyl, 1H-pyrrolo[2,3-b]pyridyl, benzo[1,3]dioxolyl, benzooxazolyl, 2-oxobenzooxazolyl, 2-oxo-1,3-dihydroindolyl, 2,3-dihydroindolyl, 2-oxodihydro-1 H-quinazolinyl, indazolyl or benzofuranyl. Examples of substituted heterocyclyl radicals are nitrobenzothiazolyl, phenyltetrazolyl, phenyloxadiazolyl, phenylpiperidinyl, phenylpiperazinyl, phenylpyrrolidinyl, thienyloxadiazolyl, furanyloxadiazolyl, benzyloxadiazolyl or phenyloxazolyl. Examples of substituted heterocyclyl radicals are dioxolanyl, dioxanyl, dithiolanyl, dithianyl, pyrrolidinyl, piperidinyl, piperazinyl, 4-methylpiperazinyl, morpholinyl, thiomorpholinyl, 2-hydroxymethylpyrrolidinyl, 3-hydroxypyrrolidinyl, 3,4-dihydroxypyrrolidinyl, 4-hydroxypiperidinyl, 4-oxopiperidinyl, 3,5-dimethylmorpholinyl, 4,4-dioxothiomorpholinyl, 4-oxothiomorpholinyl, 2,6-dimethylmorpholinyl, tetrahydropyranyl, 2-oxoimidazolidinyl, 2-oxooxazolidinyl, 2-oxopiperidinyl, 2-oxopyrrolidinyl, 2-oxo-[1,3]oxazinyl, 2-oxoazepanyl, 2-oxotetrahydropyrimidinyl and the like.

In the case of R¹, R^{4a} and R⁹, the aryl, aroyl and heterocyclyl radicals may additionally be substituted by heterocyclylalkyl, heterocyclylalkoxy, heterocyclylalkoxyalkyl or heterocyclyl for example piperidinoalkyl, piperidinoalkoxy, piperidinoalkoxyalkyl, morpholinoalkyl, morpholinoalkoxy, morpholinoalkoxyalkyl, piperazinoalkyl, piperazinoalkoxy, piperazinoalkoxyalkyl, [1,2,4]-triazol-1-ylalkyl, [1,2,4]-triazol-1-ylalkoxy, [1,2,4]-triazol-4-ylalkyl, [1,2,4]-triazol-4-ylalkoxy, [1,2,4]-oxadiazol-5-ylalkyl, [1,2,4]-oxadiazol-5-ylalkoxy, 3-methyl-[1,2,4]-oxadiazol-5-ylalkyl, 3-methyl-[1,2,4]-oxadiazol-5-ylalkoxy, 5-methyl-[1,2,4]-oxadiazol-3-ylalkyl, 5-methyl-[1,2,4]-oxadiazol-3-ylalkoxy, tetrazol-1-ylalkyl, tetrazol-1-ylalkoxy, tetrazol-2-ylalkyl, tetrazol-2-ylalkoxy, tetrazol-5-ylalkyl, tetrazol-5-ylalkoxy, 5-methyltetrazol-1-ylalkyl, 5-methyltetrazol-1-ylalkoxy, thiazol-4-ylalkyl, thiazol-4-ylalkoxy, oxazol-4-ylalkyl, oxazol-4-ylalkoxy, 2-oxopyrrolidinylalkyl, 2-oxopyrrolidinylalkoxy, imidazolylalkyl, imidazolylalkoxy, 2-methylimidazolylalkyl, 2-methylimidazolylalkoxy, N-methylpiperazinoalkyl, N-methylpiperazinoalkoxy, N-methylpiperazinoalkoxyalkyl, and also alkylaminoalkyl, alkylaminoalkoxy, alkylaminoalkoxyalkyl, mono- and polyhydroxyalkyl, -alkoxy, -alkoxyalkyl and -alkoxyalkoxy, carbamoylalkyloxy, C₁₋₈-alkoxy, amino-C₁₋₈-alkoxy, hydroxy-C₁₋₈-alkoxy, dioxolanyl, dioxanyl, dithiolanyl, dithianyl, pyrrolidinyl, piperidinyl, piperazinyl, pyrrolyl, 4-methylpiperazinyl, morpholinyl, thiomorpholinyl, 2-hydroxymethylpyrrolidinyl, 3-hydroxypyrrolidinyl, 3,4-dihydroxypyrrolidinyl, 3-acetamidomethylpyrrolidinyl, 3-C₁₋₈-alkoxy-C₁₋₈-alkylpyrrolidinyl, 4-hydroxypiperidinyl, 4-oxopiperidinyl, 3,5-dimethylmorpholinyl, 4,4-dioxothiomorpholinyl, 4-oxothiomorpholinyl, 2,6-dimethylmorpholinyl, 2-oxoimidazolidinyl, 2-oxooxazolidinyl, 2-oxopyrrolidinyl, 2-oxo[1,3]oxazinyl, 2-oxotetrahydropyrimidinyl and the like, or by the -O-CH₂CH(OH)CH₂NRx radical where NRx is a mono- or di-C₁₋₈-alkylamino, piperidino, morpholino, piperazino or N-methylpiperazino radical. Examples of 5- and 6-membered heterocyclic rings represented by NR⁵R⁶ are pyrrolidinyl, piperidinyl, piperazinyl, morpholinyl, thiomorpholinyl, 2-hydroxymethylpyrrolidinyl, 3-hydroxypyrrolidinyl, 3,4-dihydroxypyrrolidinyl, 4-hydroxypiperidinyl, 4-oxopiperidinyl, 3,5-dimethylmorpholinyl, 4,4-dioxothiomorpholinyl, 4-oxothiomorpholinyl, 2,6-dimethylmorpholinyl, 2-oxoimidazolidinyl, 2-oxooxazolidinyl, 2-oxopyrrolidinyl, 2-oxo-[1,3]oxazinyl, 2-oxotetrahydropyrimidinyl and the like. Examples of 3-7-membered rings represented by CR⁷R⁸ are cyclopentyl, cyclohexyl, cycloheptyl, 1,3-dioxolanyl, 1,3-dioxanyl, 1,3-dithiolanyl and 1,3-dithianyl. The term polyhydroxyalkyl denotes C₁₋₇-alkyl radicals which may be substituted by 2-6 hydroxyl groups, for example glyceryl, arabityl, sorbityl, etc. Halogen or halo denotes, for example, fluorine, chlorine or bromine, or a radical singly, multiply or fully substituted by fluorine, chlorine or bromine.

Salts are primarily the pharmaceutically usable or nontoxic salts of compounds of the formula (I) or formula (II). The term "pharmaceutically useable salts" encompasses salts with inorganic or organic acids, such as hydrochloric acid, hydrobromic acid, nitric acid, sulfuric acid, phosphoric acid, citric acid, formic acid, maleic acid, acetic acid, succinic acid, tartaric acid, methanesulfonic acid, p-toluenesulfonic acid and the like.

Salts of compounds having salt-forming groups are in particular acid addition salts, salts with bases, or, in the presence of a plurality of salt-forming groups, in some cases also mixed salts or internal salts.

Such salts are formed, for example, from compounds of the formula (I) or formula (II) with an acidic group, for example a carboxyl or sulfo group, and are, for example, the salts thereof with suitable bases such as non-toxic metal salts derived from metals of group Ia, Ib, IIa and IIb of the Periodic Table of the Elements, for example alkali metal, in particular lithium, sodium, or potassium, salts, alkaline earth metal salts, for example magnesium or calcium salts, and also zinc salts and ammonium salts, including those salts which are formed with organic amines, such as optionally hydroxy-substituted mono-, di- or trialkylamines, in particular mono-, di- or tri(lower alkyl)amines, or with quaternary ammonium bases, e.g. methyl-, ethyl-, diethyl- or triethylamine, mono-, bis- or tris(2-hydroxy(lower alkyl))amines, such as ethanol-, diethanol- or triethanolamine, tris(hydroxymethyl)methylamine or 2-hydroxy-tert-butylamine, N,N-di(lower alkyl)-N-(hydroxy(lower alkyl))amine, such as N,N-di-N-dimethyl-N-(2-hydroxyethyl)amine, or N-methyl-D-glucamine, or quaternary ammonium hydroxides such as tetrabutylammonium hydroxide. The compounds of the formula I having a basic group, for example an amino group, may form acid addition salts, for example with suitable inorganic acids, e.g. hydrohalic acid such as hydrochloric acid, hydrobromic acid, sulfuric acid with replacement of one or both protons, phosphoric acid with replacement of one or more protons, e.g. orthophosphoric acid or metaphosphoric acid, or pyrophosphoric acid with replacement of one or more protons, or with organic carboxylic, sulfonic or phosphonic acids or N-substituted sulfamic acids, e.g. acetic acid, propionic acid, glycolic acid, succinic acid, maleic acid, hydroxymaleic acid, methylmaleic acid, fumaric acid, malic acid, tartaric acid, gluconic acid, glucaric acid, glucuronic acid, citric acid, benzoic acid, cinnamic acid, mandelic acid, salicylic acid, 4-aminosalicylic acid, 2-phenoxybenzoic acid, 2-acetoxybenzoic acid, embonic acid, nicotinic acid, isonicotinic acid, and also amino acids, for example the alpha-amino acids mentioned above, and also methanesulfonic acid, ethanesulfonic acid, 2-hydroxyethanesulfonic acid, ethane-1,2-disulfonic acid, benzenesulfonic acid, 4-methylbenzenesulfonic acid, naphthalene-2-sulfonic acid, 2- or 3-phosphoglycerate, glucose 6-phosphate, N-cyclohexylsulfamic acid (with formation of the cyclamates) or with other acidic organic compounds such as ascorbic acid. Compounds of the formula (I) or formula (II) having acidic and basic groups may also form internal salts.

Salts obtained may be converted to other salts in a manner known per se, acid addition salts, for example, by treating with a suitable metal salt such as a sodium, barium or silver salt, of another acid in a suitable solvent in which an inorganic salt which forms is insoluble and thus separates out of the reaction equilibrium, and base salts by release of the free acid and salt reformation.

The compounds of the formula (I) and (II), including their salts, may also be obtained in the form of hydrates or include the solvent used for the crystallization.

For the isolation and purification, pharmaceutically unsuitable salts may also find use.

The compounds of the formulae (I) and (II) also include those compounds in which one or more atoms are replaced by their stable, non-radioactive isotopes; for example a hydrogen atom by deuterium.

The compounds of the formulae (I) or (II) may be prepared in a similar manner to the preparation processes disclosed in the literature. Preferred procedures for the preparation of optically pure compounds of the formula (I) or (II) consist of the construction of the requisite multiply-substituted piperidine scaffolds and their precursors via either (i) a chiral aza-Diels-Alder reaction of an imine derivative with a diene (see Chemistry-A European Journal 2000, 6(13), 2435-2448 and references cited therein) [Scheme 1] or (ii) condensation of a chiral amino acid derivative with Meldrum's acid followed by cyclisation (see Journal of Organic Chemistry 2004, 69(1), 130-141 and references cited therein) [Scheme 2]. In the case of the former approach, the presence of non-stoichiometric amounts of an amine (e.g. triethylamine) is crucial for obtaining products of high optical purities. This amine component may either be present in the commercially available diene (e.g. Danishefsky's diene from Aldrich) or added exogenously. Details on the specific preparation variants can be taken from the examples.

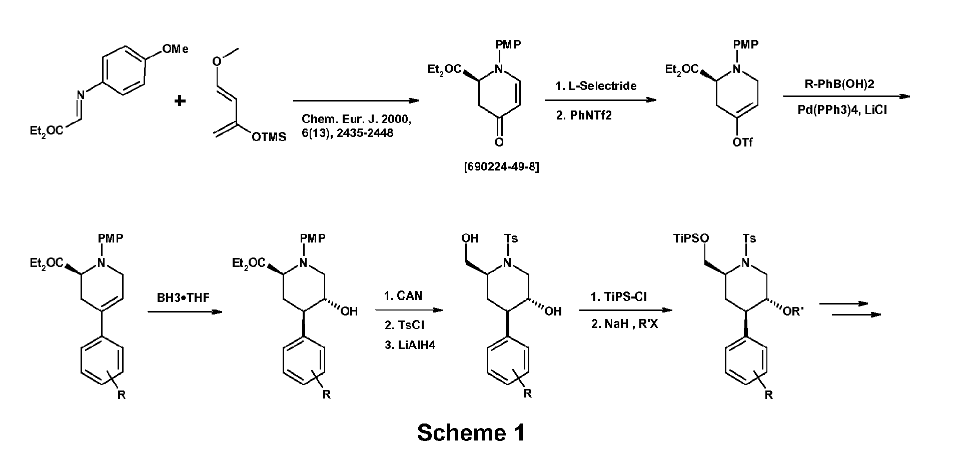

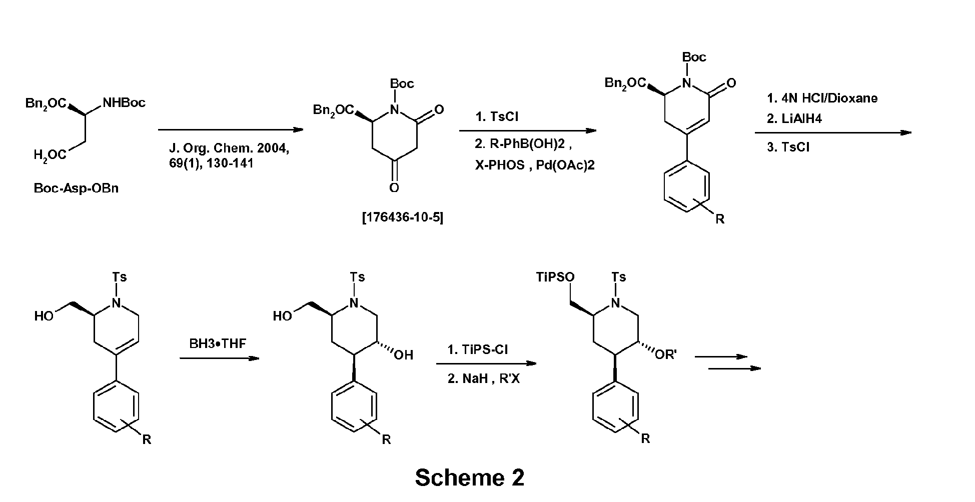

The compounds of the formula (I) or formula (II) have at least three asymmetric carbon atoms and may therefore be in the form of optically pure diastereomers, diastereomeric mixtures, diastereomeric racemates, mixtures of diastereomeric racemates or as meso compounds. The invention encompasses all of these forms. Diastereomeric mixtures, diastereomeric racemates or mixtures of diastereomeric racemates may be separated by customary procedures, for example by column chromatography, thin-layer chromatography, HPLC and the like.

The compounds of the formulae (I) or (II) may also be prepared in optically pure form. The separation into antipodes can be effected by procedures known per se, either preferably at an earlier synthetic stage by salt formation with an optically active acid, for example (+)- or (-)-mandelic acid and separation of the diastereomeric salts by fractional crystallization, or preferably at a relatively late stage by derivatizing with a chiral auxiliary building block, for example (+)- or (-)-camphanoyl chloride, and separation of the diastereomeric products by chromatography and/or crystallization and subsequent cleavage of the bonds to give the chiral auxiliary. The pure diastereomeric salts and derivatives may be analysed to determine the absolute configuration of the piperidine present with common spectroscopic procedures, and X-ray spectroscopy on single crystals constitutes a particularly suitable procedure.

It is possible for the configuration at individual chiral centres in a compound of the formulae (I) or (II) to be inverted selectively. For example, the configuration of asymmetric carbon atoms which bear nucleophilic substituents, such as amino or hydroxyl, may be inverted by second-order nucleophilic substitution, if appropriate after conversion of the bonded nucleophilic substituent to a suitable nucleofugic leaving group and reaction with a reagent which introduces the original substituents, or the configuration at carbon atoms having hydroxyl groups can be inverted by oxidation and reduction, analogously to the process in the European patent application EP-A-0 236 734. Also advantageous is the reactive functional modification of the hydroxyl group and subsequent replacement thereof by hydroxyl with inversion of configuration.

The compounds of the formulae (I) or (II) also include compounds where one or more atoms are replaced by their stable, non-radioactive isotopes (for example hydrogen by deuterium).

Prodrug derivatives of the compounds described in the present context are derivatives thereof which, on *in vivo* application, release the original compound by a chemical or physiological process. A prodrug may be converted to the original compound, for example, when a physiological pH is attained or by enzymatic conversion. Prodrug derivatives may, for example, be esters of freely available carboxylic acids, S- and O-acyl derivatives of thiols, alcohols or phenols, and the acyl group is as defined in the present context. Preference is given to pharmaceutically useable ester derivatives which are converted by solvolysis in physiological medium to the original carboxylic acid, for example lower alkyl esters, cycloalkyl esters, lower alkenyl esters, benzyl esters, mono- or disubstituted lower alkyl esters such as lower ω-(amino, mono- or dialkylamino, carboxyl, lower alkoxycarbonyl)-alkyl esters or such as lower α-(alkanoyloxy, alkoxycarbonyl or dialkylaminocarbonyl)-alkyl esters; as such, pivaloyloxymethyl esters and similar esters are utilized in a conventional manner.

Owing to the close relationship between a free compound, a prodrug derivative and a salt compound, a certain compound in this invention also encompasses its prodrug derivative and salt form, where these are possible and appropriate.

The compound groups mentioned below are not to be regarded as closed, but rather parts of these compound groups may be exchanged with one another or with the definitions given above or omitted in a sensible manner, for example to replace general by more specific definitions. The definitions are valid in accordance with general chemical principles, such as, for example, the common valences for atoms.

Preferred inventive compounds are those of the general formulae (IA) or (IIA) 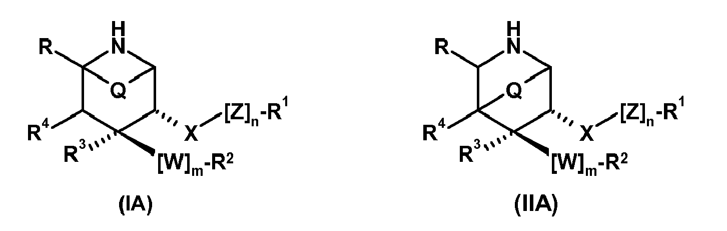 where R, R¹, R², R³, R⁴, Q, W, X and Z, n and m are each as defined above for the compounds of the formulae (I) and (II).

A further, preferred group of compounds of the formula (I) or (II), or more preferably of the formula (IA) or (IIA), are compounds where
R is C₂₋₈-alkenyl, C₁₋₈-alkyl, C₂₋₈-alkynyl, C₀₋₈-alkyl-carbonyl-amino-C₁₋₈-alkyl, C₃₋₈-cycloalkyl-C₀₋₈-alkyl, C₁₋₈-alkyl-sulfonyl-C₀₋₈-alkyl, optionally N-mono- or N,N-di-C₁₋₈-alkylated carbamoyl-C₀₋₈-alkyl, optionally O-C₁₋₈-alkylated carboxyl-C₀₋₈-alkyl, optionally N and/or N' mono-, di- or tri-C₁₋₈-alkylated ureido-C₀₋₈-alkyl or heterocyclylcarbonyl-C₀₋₈-alkyl, each of said radicals being substituted by 1-4 C₁₋₈-alkoxy or hydroxyl;
R¹ is aryl or heterocyclyl;
R² is phenyl, cyclohexyl, tetrazolyl, naphthyl or acenaphthyl, each of said radicals may be unsubstituted or substituted, preferably by 1-3 C₁₋₈-alkanoyloxy-C₁₋₈-alkyl, C₂₋₈-alkenyloxy, C₁₋₈-alkoxy, C₁₋₈-alkoxy-C₁₋₈-alkoxy, C₁₋₈-alkoxy-C₁₋₈-alkoxy-C₁₋₈-alkyl, C₁₋₈-alkoxy-C₁₋₈-alkyl, C₁₋₈-alkoxycarbonyl, C₁₋₈-alkoxycarbonyloxy-C₁₋₈-alkyl, C₁₋₈-alkyl, carboxy-C₁₋₈-alkyl, C₁₋₈-alkoxy-C₁₋₈-alkylsulfanyl, C₁₋₈-alkylsulfanyl, C₁₋₈-alkylsulfanyl-C₁₋₈-alkoxy, C₁₋₈-alkylsulfanyl-C₁₋₈-alkoxy-C₁₋₈-alkyl, C₁₋₈-alkylsulfanyl-C₁₋₈-alkyl, cyano, cyano-C₁₋₈-alkyl, C₃₋₈-cycloalkyl-C₀₋₆-alkoxy-C₁₋₈-alkoxy, C₁₋₆-alkoxy-C₀₋₆-alkyl-C₃₋₈-cycloalkyl-C₀₋₆-alkoxy-C₁₋₈-alkyl, C₃₋₈-cycloalkyl-C₀₋₆-alkoxy-C₁₋₈-alkyl, halo-C₁₋₈-alkyl, halogen, hydroxy-C₁₋₈-alkyl, hydroxyl, oxide, trifluoromethoxy or trifluoromethyl groups, or a C₁₋₈-alkylenedioxy group, and/or by an L1-T1-L2-T2-L3-T3-L4-T4-L5-U radical;
L1, L2, L3, L4 and L5 are each independently a bond, C₁₋₈-alkylene, C₂₋₈-alkenylene or C₂₋₈-alkynylene, C₃₋₈-cydoalkene or are absent;
T1, T2, T3 and T4 are each independently
   (a) a bond, or are absent, or are one of the groups
   (b) -CH(OH)-
   (c) -CH(OR⁶)-
   (d) -CH(NR⁵R⁶)-
   (e) -CO-
   (f) -CR⁷R⁸-
   (g) -O- or -NR⁶-
   (h) -S(O)₀₋₂-
   (i) -SO₂NR⁶-
   (j) -NR⁶SO₂-
   (k) -CONR⁶-
   (l) -NR⁶CO-
   (m) -O-CO-
   (n) -CO-O-
   (o) -O-CO-O-
   (p) -O-CO-NR⁶-
   (q) -N(R⁶)-CO-N(R⁶)-
   (r) -N(R⁶)-CO-O-
   (s) pyrrolidinylene, piperidinylene or piperazinylene
   (t) -C(R¹¹)(R¹²)-,
   where the bonds starting from (b)-(t) lead to a saturated or aromatic carbon atom of the adjacent group if the bond starts from a heteroatom, and where not more than two (b)-(f) groups, three (g)-(h) groups and one (i)-(t) group are present;
   R³ is hydrogen, hydroxyl, C₁₋₈-alkoxy or C₂₋₈-alkenyloxy;
   R⁴ is hydrogen, C₁₋₈-alkoxy, C₁₋₈-alkoxy-C₁₋₈-alkoxy, C₁₋₈-alkoxy-C₁₋₈-alkyl, C₁₋₈-alkyl, optionally (N-C₁₋₈-alkyl)-C₁₋₈-alkoxycarbonyl-amino-C₁₋₈-alkoxy, optionally (N-C₁₋₈-alkyl)-C₁₋₈-alkylcarbonyl-amino-C₁₋₈-alkoxy, optionally (N-mono- or N,N-di-C₁-C₈-alkyl)-amino-C₁₋₈-alkoxy, C₃₋₈-cycloalkyloxy, C₃₋₈-cycloalkyloxy-C₁₋₈-alkoxy, heterocyclyl-C₀₋₈-alkoxy, heterocyclyloxy-C₁₋₈-alkoxy, hydroxy, oxo or hydroxy-C₁₋₈-alkoxy-C₁₋₈-alkoxy;
   R⁵ and R⁶ are each independently hydrogen, C₁₋₈-alkyl or acyl, or, together with the nitrogen atom to which they are bonded, are a 5- or 6-membered heterocyclic ring which may contain an additional nitrogen, oxygen or sulfur atom;
   R⁷ and R⁸, together with the carbon atom to which they are bonded, are a 3-7-membered ring which may contain one or two -O- or -S- atoms;
   R⁹ is hydrogen, C₁₋₈-alkyl, acyl or arylalkyl;
   U is hydrogen, C₁₋₈-alkyl, C₃₋₈-cycloalkyl, cyano, aryl or heterocyclyl;
   Q is ethylene or is absent (formula (I)) and is ethylene or methylene (formula (II));
   X is a bond, oxygen, sulfur or is a >CHR¹¹, >CHOR⁹, -O-CO-, >CO or -O-CH-R¹¹-CO-NR⁹- group;
   W is oxygen or sulfur if R³ is hydrogen;
   Z is C₁₋₈-alkylene or -alk-O-;
   where, if X is a bond, Z is -alk-O-;
   n is 0 or 1;
   m is 0 or 1;
and pharmaceutically useable salts thereof.
Preference is further given to compounds of the formulae (I), (lA), (II) and (IIA) in which W is absent (m is 0), and to those of the formulae (I) and (IA) in which Q is absent.

Preferred R radicals are C₁₋₈-alkyl, C₀₋₈-alkyl-carbonyl-amino-C₁₋₈-alkyl, C₁₋₈-alkylsulfonyl-C₁₋₈-alkyl, optionally N-mono- or N,N-di-C₁₋₈-alkylated carbamoyl-C₀₋₈-alkyl, optionally N and/or N' mono-, di- or tri-C₁₋₈-alkylated ureido-C₀₋₈-alkyl, heterocyclyl-C₀₋₈-alkyl, or heterocyclylcarbonyl-C₀₋₈-alkyl, each of said radicals being substituted by 1-4 C₁₋₈-alkoxy or hydroxyl.

Preferred R¹ radicals are phenyl and phenyl substituted by 1-3 radicals selected from C₁₋₈-alkyl, C₁₋₈-alkoxy, halogen, hydroxyl, hydroxy-C₁₋₈-alkoxy, C₁₋₈-alkoxy-C₁₋₈-alkoxy, C₁₋₈-alkylsulfinyl, carbamoyl, cyano, trifluoromethyl, trifluoromethoxy, carboxyl, C₁₋₈-alkoxycarbonyl, C₁₋₈-alkoxy-C₁₋₈-alkoxy-C₁₋₈-alkyl, C₀₋₆-alkylcarbonylamino, C₀₋₆-alkylcarbonylamino-C₁₋₈-alkyl, C₀₋₆-alkylcarbonylamino-C₁₋₈-alkoxy, (N-C₁₋₈-alkyl)-C₀₋₆-alkylcarbonylamino-C₁₋₈-alkyl, (N-C₁₋₈-alkyl)-C₀₋₆-alkylcarbonylamino-C₁₋₈-alkoxy, C₃₋₈-cycloalkylcarbonylamino-C₁₋₈-alkyl, C₃₋₈-cycloalkylcarbonyl-amino-C₁₋₈-alkoxy, C₁₋₈-alkoxy-C₁₋₈-alkyl, hydroxy-C₁₋₈-alkyl, hydroxy-C₁₋₈-alkoxy-C₁₋₈-alkyl, hydroxy-C₁₋₈-alkoxy-C₁₋₈-alkoxy, C₁₋₈-alkoxycarbonylamino-C₁₋₈-alkyl, C₁₋₈-alkoxycarbonylamino-C₁₋₈-alkoxy, C₁₋₈-alkylaminocarbonylamino-C₁₋₈-alkyl, C₁₋₈-alkylaminocarbonylamino-C₁₋₈-alkoxy, C₁₋₈-alkylaminocarbonyl-C₁₋₈-alkyl, C₁₋₈-alkylaminocarbonyl-C₁₋₈-alkoxy, C₁₋₈-alkylaminocarbonyl-C₁₋₈-alkoxy-C₁₋₈-alkyl, di-C₁₋₈-alkylaminocarbonyl-C₁₋₈-alkyl, di-C₁₋₈-alkylaminocarbonyl-C₁₋₈-alkoxy, C₁₋₈-alkylcarbonyloxy-C₁₋₈-alkyl, C₁₋₈-alkylcarbonyloxy-C₁₋₈-alkoxy, cyano-C₁₋₈-alkyl, cyano-C₁₋₈-alkoxy, 2-oxooxazolidinyl-C₁₋₈-alkyl, 2-oxooxazolidinyl-C₁₋₈-alkoxy, C₁₋₈-alkoxycarbonyl-C₁₋₈-alkyl, C₁₋₈-alkoxycarbonyl-C₁₋₈-alkoxy, C₁₋₈-alkylsulfonylamino-C₁₋₈-alkyl, C₁₋₈-alkylsulfonylamino-C₁₋₈-alkoxy, (N-C₁₋₈-alkyl)-C₁₋₈-alkylsulfonylamino-C₁₋₈-alkyl, (N-C₁₋₈-alkyl)-C₁₋₈-alkylsulfonylamino-C₁₋₈-alkoxy, amino-C₁₋₈-alkyl, amino-C₁₋₈-alkoxy, C₁₋₈-alkylamino-C₁₋₈-alkyl, C₁₋₈-alkylamino-C₁₋₈-alkoxy, di-C₁₋₈-alkylamino-C₁₋₈-alkyl, di-C₁₋₈-alkylamino-C₁₋₈-alkoxy, C₁₋₈-alkylsulfonyl-C₁₋₈-alkyl, C₁₋₈-alkylsulfonyl-C₁₋₈-alkoxy, carboxy-C₁₋₈-alkyl, carboxy-C₁₋₈-alkoxy, carboxy-C₁₋₈-alkoxy-C₁₋₈-alkyl, C₁₋₈-alkoxy-C₁₋₈-alkylcarbonyl, acyl-C₁₋₈-alkoxy-C₁₋₈-alkyl, (N-C₁₋₈-alkyl)-C₁₋₈-alkoxycarbonylamino, (N-hydroxy)-C₁₋₈-alkylaminocarbonyl-C₁₋₈-alkyl, (N-hydroxy)-C₁₋₈-alkylaminocarbonyl-C₁₋₈-alkoxy, (N-hydroxy)aminocarbonyl-C₁₋₈-alkyl, (N-hydroxy)aminocarbonyl-C₁₋₈-alkoxy, C₁₋₈-alkoxyaminocarbonyl-C₁₋₈-alkyl, C₁₋₈-alkoxyaminocarbonyl-C₁₋₈-alkoxy, (N-C₁₋₈-alkoxy)-C₁₋₈-alkylaminocarbonyl-C₁₋₈-alkyl, (N-C₁₋₈-alkoxy)-C₁₋₈-alkylaminocarbonyl-C₁₋₈-alkoxy, (N-acyl)-C₁₋₈-alkoxy-C₁₋₈-alkylamino, C₁₋₈-alkoxy-C₁₋₈-alkylcarbamoyl, (N-C₁₋₈-alkyl)-C₁₋₈-alkoxy-C₁₋₈-alkylcarbamoyl, C₁₋₈-alkoxy-C₁₋₈-alkylcarbonyl, C₁₋₈-alkoxy-C₁₋₈-alkylcarbonylamino, (N-C₁₋₈-alkyl)-C₁₋₈-alkoxy-C₁₋₈-alkylcarbonylamino, 1-C₁₋₈-alkoxy-C₁₋₈-alkylimidazol-2-yl, 1-C₁₋₈-alkoxy-C₁₋₈-alkyltetrazol-5-yl, 5-C₁₋₈-alkoxy-C₁₋₈-alkyltetrazol-1-yl, 2-C₁₋₈-alkoxy-C₁₋₈-alkyl-4-oxoimidazol-1-yl, carbamoyl-C₁₋₈-alkyl, carbamoyl-C₁₋₈-alkoxy, C₁₋₈-alkylcarbamoyl, di-C₁₋₈-alkylcarbamoyl, C₁₋₈-alkylsulfonyl, piperidinoalkyl, piperidinoalkoxy, piperidinoalkoxyalkyl, morpholinoalkyl, morpholinoalkoxy, morpholinoalkoxyalkyl, piperazinoalkyl, piperazinoalkoxy, piperazinoalkoxyalkyl, [1,2,4]-triazol-1-ylalkyl, [1,2,4]-triazol-1-ylalkoxy, [1,2,4]-triazol-4-ylalkyl, [1,2,4]-triazol-4-ylalkoxy, [1,2,4]-oxadiazol-5-ylalkyl, [1,2,4]-oxadiazol-5-ylalkoxy, 3-methyl-[1,2,4]-oxadiazol-5-ylalkyl, 3-methyl-[1,2,4]-oxadiazol-5-ylalkoxy, 5-methyl-[1,2,4]-oxadiazol-3-ylalkyl, 5-methyl-[1,2,4]-oxadiazol-3-ylalkoxy, tetrazol-1-ylalkyl, tetrazol-1-ylalkoxy, tetrazol-2-ylalkyl, tetrazol-2-ylalkoxy, tetrazol-5-ylalkyl, tetrazol-5-ylalkoxy, 5-methyltetrazol-1-ylalkyl, 5-methyltetrazol-1-ylalkoxy, thiazol-4-ylalkyl, thiazol-4-ylalkoxy, oxazol-4-ylalkyl, oxazol-4-ylalkoxy, 2-oxopyrrolidinylalkyl, 2-oxopyrrolidinylalkoxy, imidazolylalkyl, imidazolylalkoxy, 2-methylimidazolylalkyl, 2-methylimidazolylalkoxy, N-methylpiperazinoalkyl, N-methylpiperazinoalkoxy, N-methylpiperazinoalkoxyalkyl, pyrrolidinyl, piperidinyl, piperazinyl, pyrrolyl, 4-methylpiperazinyl, morpholinyl, thiomorpholinyl, 2-hydroxymethylpyrrolidinyl, 3-hydroxypyrrolidinyl, 3,4-dihydroxypyrrolidinyl, 3-acetamidomethylpyrrolidinyl, 3-C₁₋₈-alkoxy-C₁₋₈-alkylpyrrolidinyl, 4-hydroxypiperidinyl, 4-oxopiperidinyl, 3,5-dimethylmorpholinyl, 4,4-dioxothiomorpholinyl, 4-oxothiomorpholinyl, 2,6-dimethylmorpholinyl, 2-oxoimidazolidinyl, 2-oxooxazolidinyl, 2-oxopyrrolidinyl, 2-oxo[1,3]oxazinyl and 2-oxotetrahydropyrimidinyl.

R¹ radicals which are likewise preferred are benzofuranyl, benzoimidazolyl, 4H-benzo[1,4]oxazinyl, benzooxazolyl, 2- and 5-benzo[b]thienyl, 3,4-dihydro-2H-benzo[1,4]oxazinyl, 1,3-dihydroindolyl, 2,3-dihydroindolyl, indazolyl, indolyl,1H-quinolinyl, 2H-chromenyl, 1,1a,2,7b-tetrahydro-cyclopropa[c]chromenyl, 1a,7b-dihydro-1 H-cyclopropa[c]chromenyl, 6- and 7-isoquinolyl, pyridyl, pyrimidinyl, 6- and 7-quinazolinyl, 6- and 7-quinolyl, 6-quinoxalinyl, 6- and 7-tetrahydroisoquinolyl, 6-and 7-tetrahydroquinolyl, each of which is substituted by 1-3 radicals selected from hydroxyl, oxo, oxide, halogen, cyano, trifluoromethyl, trifluoromethoxy, carbamoyl, carboxyl, C₁₋₈-alkyl, C₁₋₈-alkoxy, hydroxy-C₁₋₈-alkoxy, C₁₋₈-alkoxy-C₁₋₈-alkoxy, di-C₁₋₈-alkylamino, 2,3-dihydroxypropoxy, 2,3-dihydroxypropoxy-C₁₋₈-alkoxy, 2,3-dimethoxypropoxy, methoxybenzyloxy, hydroxybenzyloxy, phenethyloxy, methylenedioxybenzyloxy, dioxolanyl-C₁₋₈-alkoxy, cyclopropyl-C₁₋₈-alkoxy, hydroxy-C₁₋₈-alkoxy, pyridylcarbamoyloxy-C₁₋₈-alkoxy, 3-morpholino-2-hydroxypropoxy, benzyloxy-C₁₋₈ alkoxy, picolyloxy, C₁₋₈-alkoxycarbonyl, C₁₋₈-alkoxy-C₁₋₈-alkoxy-C₁₋₈-alkyl, C₀₋₆-alkylcarbonylamino, C₀₋₆-alkylcarbonylamino-C₁₋₈-alkyl, C₀₋₆-alkylcarbonylamino-C₁₋₈-alkoxy, (N-C₁₋₈-alkyl)-C₀₋₆-alkylcarbonylamino-C₁₋₈-alkyl, (N-C₁₋₈-alkyl)-C₀₋₆-alkl-carbonylamino-C₁₋₈-alkoxy, C₃₋₈-cycloalkylcarbonylamino-C₁₋₈-alkyl, C₃₋₈-cyclo-alkylcarbonylamino-C₁₋₈-alkoxy, C₁₋₈-alkoxy-C₁₋₈-alkyl, hydroxy-C₁₋₈-alkyl, hydroxy-C₁₋₈-alkoxy-C₁₋₈-alkyl, hydroxy-C₁₋₈-alkoxy-C₁₋₈-alkoxy, C₁₋₈-alkoxycarbonylamino-C₁₋₈-alkyl, C₁₋₈-alkoxycarbonylamino-C₁₋₈-alkoxy, C₁₋₈-alkylaminocarbonylamino-C₁₋₈-alkyl, C₁₋₈-alkylaminocarbonylamino-C₁₋₈-alkoxy, C₁₋₈-alkylaminocarbonyl-C₁₋₈-alkyl, C₁₋₈-alkylaminocarbonyl-C₁₋₈-alkoxy, C₁₋₈-alkylaminocarbonyl-C₁₋₈-alkoxy-C₁₋₈-alkyl, di-C₁₋₈-alkylaminocarbonyl-C₁₋₈-alkyl, di-C₁₋₈-alkylaminocarbonyl-C₁₋₈-alkoxy, C₁₋₈-alkylcarbonyloxy-C₁₋₈-alkyl, C₁₋₈-alkylcarbonyloxy-C₁₋₈-alkoxy, cyano-C₁₋₈-alkyl, cyano-C₁₋₈-alkoxy, 2-oxooxazolidinyl-C₁₋₈-alkyl, 2-oxooxazolidinyl-C₁₋₈-alkoxy, C₁₋₈-alkoxycarbonyl-C₁₋₈-alkyl, C₁₋₈-alkoxycarbonyl-C₁₋₈-alkoxy, C₁₋₈-alkylsulfonylamino-C₁₋₈-alkyl, C₁₋₈-alkylsulfonylamino-C₁₋₈-alkoxy, (N-C₁₋₈-alkyl)-C₁₋₈-alkylsulfonylamino-C₁₋₈-alkyl, (N-C₁₋₈-alkyl)-C₁₋₈-alkylsulfonylamino-C₁₋₈-alkoxy, amino-C₁₋₈-alkyl, amino-C₁₋₈-alkoxy, C₁₋₈-alkylamino-C₁₋₈-alkyl, C₁₋₈-alkylamino-C₁₋₈-alkoxy, di-C₁₋₈-alkylamino-C₁₋₈-alkyl, di-C₁₋₈-alkylamino-C₁₋₈-alkoxy, C₁₋₈-alkylsulfonyl-C₁₋₈-alkyl, C₁₋₈-alkylsulfonyl-C₁₋₈-alkoxy, carboxy-C₁₋₈-alkyl, carboxy-C₁₋₈-alkoxy, carboxy-C₁₋₈-alkoxy-C₁₋₈-alkyl, C₁₋₈-alkoxy-C₁₋₈-alkylcarbonyl, acyl-C₁₋₈-alkoxy-C₁₋₈-alkyl, (N-C₁₋₈-alkyl)-C₁₋₈-alkoxycarbonylamino, (N-hydroxy)-C₁₋₈-alkylaminocarbonyl-C₁₋₈-alkyl, (N-hydroxy)-C₁₋₈-alkylaminocarbonyl-C₁₋₈-alkoxy, (N-hydroxy)-aminocarbonyl-C₁₋₈-alkyl, (N-hydroxy)aminocarbonyl-C₁₋₈-alkoxy, C₁₋₈-alkoxyaminocarbonyl-C₁₋₈-alkyl, C₁₋₈-alkoxyaminocarbonyl-C₁₋₈-alkoxy, (N-C₁₋₈-alkoxy)-C₁₋₈-alkylaminocarbonyl-C₁₋₈-alkyl, (N-C₁₋₈-alkoxy)-C₁₋₈-alkylaminocarbonyl-C₁₋₈-alkoxy, (N-acyl)-C₁₋₈-alkoxy-C₁₋₈-alkylamino, C₁₋₈-alkoxy-C₁₋₈-alkylcarbamoyl, (N-C₁₋₈-alkyl)-C₁₋₈-alkoxy-C₁₋₈-alkylcarbamoyl, C₁₋₈-alkoxy-C₁₋₈-alkylcarbonyl, C₁₋₈-alkoxy-C₁₋₈-alkylcarbonylamino, (N-C₁₋₈-alkyl)-C₁₋₈-alkoxy-C₁₋₈-alkylcarbonylamino, 1-C₁₋₈-alkoxy-C₁₋₈-alkylimidazol-2-yl, 1-C₁₋₈-alkoxy-C₁₋₈-alkyltetrazol-5-yl, 5-C₁₋₈-alkoxy-C₁₋₈-alkyltetrazol-1-yl, 2-C₁₋₈-alkoxy-C₁₋₈-alkyl-4-oxoimidazol-1-yl, carbamoyl-C₁₋₈-alkyl, carbamoyl-C₁₋₈-alkoxy, C₁₋₈-alkylcarbamoyl, di-C₁₋₈-alkylcarbamoyl, C₁₋₈-alkylsulfonyl, piperidinoalkyl, piperidinoalkoxy, piperidinoalkoxyalkyl, morpholinoalkyl, morpholinoalkoxy, morpholinoalkoxyalkyl, piperazinoalkyl, piperazinoalkoxy, piperazinoalkoxyalkyl, [1,2,4]-triazol-1-ylalkyl, [1,2,4]-triazol-1-ylalkoxy, [1,2,4]-triazol-4-ylalkyl, [1,2,4]-triazol-4-ylalkoxy, [1,2,4]-oxadiazol-5-ylalkyl, [1,2,4]-oxadiazol-5-ylalkoxy, 3-methyl-[1,2,4]-oxadiazol-5-ylalkyl, 3-methyl-[1,2,4]-oxadiazol-5-ylalkoxy, 5-methyl-[1,2,4]-oxadiazol-3-ylalkyl, 5-methyl-[1,2,4]-oxadiazol-3-ylalkoxy, tetrazol-1-ylalkyl, tetrazol-1-ylalkoxy, tetrazol-2-ylalkyl, tetrazol-2-ylalkoxy, tetrazol-5-ylalkyl, tetrazol-5-ylalkoxy, 5-methyltetrazol-1-ylalkyl, 5-methyltetrazol-1-ylalkoxy, thiazol-4-ylalkyl, thiazol-4-ylalkoxy, oxazol-4-ylalkyl, oxazol-4-ylalkoxy, 2-oxopyrrolidinylalkyl, 2-oxopyrrolidinylalkoxy, imidazolylalkyl, imidazolylalkoxy, 2-methylimidazolylalkyl, 2-methylimidazolylalkoxy, N-methylpiperazinoalkyl, N-methylpiperazinoalkoxy, N-methylpiperazinoalkoxyalkyl, pyrrolidinyl, piperidinyl, piperazinyl, pyrrolyl, 4-methylpiperazinyl, morpholinyl, thiomorpholinyl, 2-hydroxymethylpyrrolidinyl, 3-hydroxypyrrolidinyl, 3,4-dihydroxypyrrolidinyl, 3-acetamidomethylpyrrolidinyl, 3-C₁₋₈-alkoxy-C₁₋₈-alkylpyrrolidinyl, 4-hydroxypiperidinyl, 4-oxopiperidinyl, 3,5-dimethylmorpholinyl, 4,4-dioxothiomorpholinyl, 4-oxothiomorpholinyl, 2,6-dimethylmorpholinyl, 2-oxoimidazolidinyl, 2-oxooxazolidinyl, 2-oxopyrrolidinyl, 2-oxo-[1,3]oxazinyl and 2-oxotetrahydropyrimidinyl.

Examples of particularly preferred R¹ radicals are phenyl, pyridyl, 3-C₁₋₈-alkylindolyl, benzofuranyl, 4H-benzo[1,4]oxazin-3-onyl, 3,4-dihydro-2H-benzo[1,4]oxazinyl, 3,4-dihydro-2H-benzo[1,4]thiazinyl, 3,3-di-C₁₋₈-alkyl-1,3-dihydroindol-2-onyl, 3,3-di-C₁₋₈-alkyl-1,3-dihydroindolyl, indazolyl, indolyl, 1H-quinolinyl, 2H-chromenyl, 1,1a,2,7b-tetrahydro-cyclopropa[c]chromenyl, 1a,7b-dihydro-1H-cyclopropa[c]chromenyl, and spiro[cyclopropane-1,3']-2,3-dihydro-1 H-indolyl which may be substituted as specified above, especially by at least one substituent selected from halogen, oxide, oxo, C₁₋₈-alkoxy, C₁₋₈-alkoxy-C₁₋₈-alkoxy, C₁₋₈-alkoxy-C₁₋₈-alkyl, C₁₋₈-alkoxycarbonylamino-C₁₋₈-alkoxy, C₁₋₈-alkoxycarbonylamino-C₁₋₈-alkyl, C₁₋₈-alkyl, C₀₋₆-alkylcarbonylamino-C₁₋₈-alkoxy, C₀₋₆-alkylcarbonylamino-C₁₋₈-alkyl, N-acetyl-C₁₋₈-alkoxy-C₁₋₈-alkylamino, C₁₋₈-alkanoylamido-C₁₋₈-alkyl, N-C₁₋₈-alkyl-C₁₋₈-alkanoylamido-C₁₋₈-alkyl, C₁₋₈-alkoxy-C₁₋₈-alkoxy-C₁₋₈-alkyl, triazol-1-yl-C₁₋₈-alkyl, tetrazol-1-yl-C₁₋₈-alkyl, tetrazol-2-yl-C₁₋₈-alkyl, tetrazol-5-yl-C₁₋₈-alkyl, C₁₋₈-alkoxycarboxyl-C₁₋₈-alkyl, pyrrolidinonyl-C₁₋₈-alkyl, imidazolyl-C₁₋₈-alkyl, cyano-C₁₋₈-alkyl, carboxy-C₁₋₈-alkyl, carboxy-C₁₋₈-alkoxy, C₁₋₈-alkoxycarbonyl-C₀₋₆-alkyl, C₁₋₈-alkylsulfonamidyl-C₁₋₈-alkyl, C₁₋₈-alkoxy-C₁₋₈-alkanoylamido, C₁₋₈-alkoxy-C₁₋₈-alkanoylamido-C₁₋₈-alkyl, N-(C₁₋₈-alkyl)-C₁₋₈-alkoxy-C₁₋₈-alkanoylamido, N-C₁₋₈-alkylcarbamoyl-C₁₋₈-alkyl, C₃₋₈-cyclo-alkanoylamido-C₁₋₈-alkyl, C₁₋₈-alkylaminocarbonylamino-C₁₋₈-alkyl, C₁₋₈-alkanoyl-amidomethylpyrrolidinyl, N-(C₁₋₈-alkoxy-C₁₋₈-alkyl)carbamoyl, N-(C₁₋₈-alkoxy-C₁₋₈-alkyl)-N-(C₁₋₈-alkyl)carbamoyl, N-(C₁₋₈-alkoxy-C₁₋₈-alkyl)imidazol-2-yl, hydroxy-C₁₋₈-alkyl, hydroxy-C₁₋₈-alkoxy, hydroxy-C₁₋₈-alkoxy-C₁₋₈-alkyl, C₁₋₈-alkoxycarbonylamido-C₁₋₈-alkyl, amino-C₁₋₈-alkyl and C₁₋₈-alkylamino-C₁₋₈-alkyl.

Examples of very particularly preferred R¹ radicals are phenyl, pyridyl, or a bicyclic heterocyclyl, consisting of a phenyl-ring annulated to a 5-6 membered, saturated or unsaturated, heteocyclic ring, having from 1 to 3 nitrogen and/or 1 sulfur or oxygen atoms, such as 4H-benzo[1,4]oxazin-3-on-6-yl, 3,4-dihydro-2H-benzo[1,4]oxazin-6-yl, 3,4-dihydro-2H-benzo[1,4]thiazin-6-yl, 2,2-dimethyl-3,4-dihydro-2H-benzo[1,4]oxazin-6-yl, 2,2-dimethyl-4H-benzo[1,4]oxazin-3-on-6-yl, 4,4-dimethyl-1,4-dihydro-benzo[d][1,3]oxazin-2-on-8-yl, 3,3-dimethyl-1,3-dihydroindol-2-on-6-yl, 3,3-dimethyl-1,3-dihydroindol-2-on-7-yl, 3,3-dimethyl-1,3-dihydroindol-6-yl, 3-methylindol-6-yl, 1-methyl-indazol-5-yl, 3,4-dihydro-1H-quinolin-2-on-7-yl, 4,4-dimethyl-3,4-dihydro-1H-quinolin-2-on-8-yl, 1H-quinolinyl, 2H-chromenyl, 1,1a,2,7b-tetrahydrocyclo-propa[c]chromenyl, 1a,7b-dihydro-1H-cyclopropa[c]chromenyl, and spiro[cyclopropane-1,3']-2,3-dihydro-1H-indol-6-yl, which may be substituted as specified above, especially by at least one substituent selected from C₁₋₈-alkoxy, C₁₋₈-alkoxy-C₁₋₈-alkoxy, C₁₋₈-alkoxy-C₁₋₈-alkoxy-C₁₋₈-alkyl, C₁₋₈-alkoxy-C₁₋₈-alkyl, C₁₋₈-alkoxycarbonylamino-C₁₋₈-alkoxy, C₁₋₈-alkoxycarbonylamino-C₁₋₈-alkyl, C₁₋₈-alkyl, C₀₋₆-alkylcarbonylamino-C₁₋₈-alkoxy, C₀₋₆-alkylcarbonylamino-C₁₋₈-alkyl, halogen, oxide, triazol-1-yl-C₁₋₈-alkyl, and oxo, and, preferably, where, in the case that R¹ is a bicyclic heterocyclyl, the phenyl-ring of the bicyclic system is bonded to Z or, in case n is 0, is bonded to X and at least the non-phenyl-ring is substituted as specified.

Preferred R² radicals are phenyl and phenyl substituted by halogen, hydroxyl, cyano, trifluoromethoxy, trifluoromethyl, C₂₋₈-alkenyloxy, C₁₋₈-alkoxy, C₁₋₈-alkoxy-C₁₋₈-alkoxy, C₁₋₈-alkoxy-C₁₋₈-alkoxy-C₁₋₈-alkyl, C₁₋₈-alkoxy-C₁₋₈-alkyl, C₁₋₈-alkyl, C₁₋₈-alkoxy-C₁₋₈-alkylsulfanyl, C₁₋₈-alkylsulfanyl, C₁₋₈-alkylsulfanyl-C₁₋₈-alkyl, C₁₋₈-alkylsulfanyl-C₁₋₈-alkoxy, C₁₋₈-alkylsulfanyl-C₁₋₈-alkoxy-C₁₋₈-alkyl, C₃₋₈-cycloalkylsulfanyl-C₁₋₈-alkyl, C₃₋₈-cycloalkyl-C₀₋₆-alkoxy, C₃₋₈-cycloalkyl-C₀₋₆-alkoxy-C₁₋₈-alkyl, C₃₋₈-cycloalkyl-C₀₋₆-alkoxy-C₁₋₈-alkoxy, heterocyclyl-C₀₋₆-alkoxy, heterocyclyl-C₀₋₆-alkoxy-C₁₋₈-alkyl, halo-C₁₋₈-alkyl, hydroxy-C₁₋₈-alkyl , cyano-C₁₋₈-alkyl, carboxy-C₁₋₈-alkyl, C₁₋₈-alkylcarbonyl-oxy-C₁₋₈-alkyl, C₁₋₈-alkoxycarbonyloxy-C₁₋₈-alkyl, C₁₋₈-alkoxycarbonyl or C₁₋₈-alkylene-dioxy.

R² radicals which are likewise preferred are phenyl and halophenyl substituted by an L1-T1-L2-T2-L3-T3-L4-T4-L5-U radical where L1 and L2 are preferably absent or C₁₋₈-alkylene and L3 is absent and U is hydrogen, C₁₋₈-alkyl, C₃₋₈-cycloalkyl, phenylpiperidinyl, phenylpiperazinyl, phenylpyrrolidinyl, phenyl, is phenyl or phenylpyrrolidinyl each substituted by C₁₋₈-alkyl, C₁₋₈-alkoxy, C₁₋₈-alkylthio, C₁₋₈-alkylsulfinyl, C₁₋₈-alkylenedioxy, halogen, benzoyl-C₁₋₈-alkyl, halogen-C₁₋₈-alkyl, C₁₋₈-alkanoyloxy or hydroxyl, or is naphthyl, pyridyl, thienyl, pyrazinyl, triazolyl, imidazolyl, phenyloxadiazolyl, thienyloxadiazolyl, furyloxadiazolyl, phenyloxazolyl, benzthiazolyl, furyl, pyrimidinyl, nitrobenzthiazolyl, phenyltetrazolyl, piperidinyl, tetrahydropyranyl, morpholinyl or indolyl.
In the groups T1-T4, preference is given to the definitions (a)-(c), (e)-(h), (k)-(n) and (r)-(t).

Examples of particularly preferred R² radicals are unsubstituted phenyl and phenyl and halophenyl each substituted by C₂₋₈-alkenyloxy, C₁₋₈-alkoxy, C₁₋₈-alkoxy-C₁₋₈-alkoxy, C₁₋₈-alkoxy-C₁₋₈-alkoxy-C₁₋₈-alkyl, C₁₋₈-alkoxy-C₁₋₈-alkyl, C₁₋₈-alkoxy-C₁₋₈-alkylamino-C₁₋₈-alkyl, C₁₋₈-alkyl, C₁₋₈-alkoxy-C₁₋₈-alkylsulfanyl, C₁₋₈-alkylsulfanyl, C₁₋₈-alkylsulfanyl-C₁₋₈-alkyl, C₁₋₈-alkylsulfanyl-C₁₋₈-alkoxy, C₁₋₈-alkylsulfanyl-C₁₋₈-alkoxy-C₁₋₈-alkyl, C₃₋₈-cycloalkylsulfanyl-C₁₋₈-alkyl, C₁₋₈-alkylsulfonyl-C₁₋₈-alkoxy-C₁₋₈-alkyl, C₃₋₈-cycloalkyl-C₀₋₆-alkoxy, C₃₋₈-cycloalkyl-C₀₋₆-alkoxy-C₁₋₈-alkoxy, C₃₋₈-cycloalkyl-C₀₋₆-alkoxy-C₁₋₈-alkyl, C₁₋₆-alkoxy-C₀₋₆-alkyl-C₃₋₈-cycloalkyl-C₀₋₆-alkoxy-C₁₋₈-alkyl, C₃₋₈-cycloalkyl-C₁₋₈-alkylamino-C₁₋₈-alkyl, halogen, heterocyclyl-C₀₋₆-alkoxy, heterocyclyl-C₀₋₆-alkoxy-C₁₋₈-alkyl, C₁₋₈-alkoxy-C₁₋₈-alkylamino-C₁₋₈-alkyl, N-(halophenyl)pyrrolidinyloxy, N-(halo-phenyl)pyrrolidinyloxy-C₁₋₈-alkyl, C₁₋₈-alkoxy-benzyloxy-C₁₋₈-alkoxy, C₁₋₈-alkoxyphenoxy-C₁₋₈-alkoxy, C₁₋₈-alkoxyphenoxy-C₁₋₈-alkyl, C₁₋₈-alkoxyphenyl-C₁₋₈-alkoxy-C₁₋₈-alkoxy, halobenzyloxy-C₁₋₈-alkoxy, halophenoxy-C₁₋₈-alkoxy, halophenoxy-C₁₋₈-alkoxy-C₁₋₈-alkyl, halophenoxy-C₁₋₈-alkyl, halophenyl-C₁₋₈-alkoxy-C₁₋₈-alkoxy or C₁₋₈-alkylbenzyloxy-C₁₋₈-alkoxy.

Examples of very particularly preferred R² radicals are phenyl and halophenyl each substituted by C₂₋₈-alkenyloxy, C₁₋₈-alkoxy, C₁₋₈-alkoxy-C₁₋₈-alkoxy, C₁₋₈-alkoxy-C₁₋₈-alkoxy-C₁₋₈-alkyl, C₁₋₈-alkoxy-C₁₋₈-alkyl, C₁₋₈-alkoxy-C₁₋₈-alkyl-amino-C₁₋₈-alkyl, C₁₋₈-alkyl, C₁₋₈-alkoxy-C₁₋₈-alkylsulfanyl, C₁₋₈-alkylsulfanyl, C₁₋₈-alkylsulfanyl-C₁₋₈-alkyl, C₁₋₈-alkylsulfanyl-C₁₋₈-alkoxy, C₁₋₈-alkylsulfanyl-C₁₋₈-alkoxy-C₁₋₈-alkyl, C₃₋₈-cyclo-alkylsulfanyl-C₁₋₈-alkyl, C₁₋₈-alkylsulfonyl-C₁₋₈-alkoxy-C₁₋₈-alkyl, C₃₋₈-cycloalkyl-C₀₋₆-alkoxy, C₃₋₈-cycloalkyl-C₀₋₆-alkoxy-C₁₋₈-alkoxy, C₃₋₈-cycloalkyl-C₀₋₆-alkoxy-C₁₋₈-alkyl, C₁₋₆-alkoxy-C₀₋₆-alkyl-C₃₋₈-cycloalkyl-C₀₋₆-alkoxy-C₁₋₈-alkyl, C₃₋₈-cycloalkyl-C₁₋₈-alkylamino-C₁₋₈-alkyl, halogen, heterocyclyl-C₀₋₆-alkoxy, heterocyclyl-C₀₋₆-alkoxy-C₁₋₈-alkyl, C₁₋₈-alkoxy-C₁₋₈-alkylamino-C₁₋₈-alkyl, N-(halo-phenyl)pyrrolidinyloxy, N-(halophenyl)pyrrolidinyloxy-C₁₋₈-alkyl, C₁₋₈-alkoxybenzyloxy-C₁₋₈-alkoxy, C₁₋₈-alkoxy-phenoxy-C₁₋₈-alkoxy, C₁₋₈-alkoxyphenoxy-C₁₋₈-alkyl, C₁₋₈-alkoxyphenyl-C₁₋₈-alkoxy-C₁₋₈-alkoxy, halobenzyloxy-C₁₋₈-alkoxy, halophenoxy-C₁₋₈-alkoxy, halophenoxy-C₁₋₈-alkoxy-C₁₋₈-alkyl, halophenoxy-C₁₋₈-alkyl, halophenyl-C₁₋₈-alkoxy-C₁₋₈-alkoxy or C₁₋₈-alkylbenzyloxy-C₁₋₈-alkoxy.

Also very particularly preferred R² radicals are phenyl and halophenyl each substituted as defined above whereby at least one substituent is in the para-position relative to the bond of R² to the rest of the molecule. Preferred substituents --in the para-position relative to the bond of R² to the rest of the molecule-- on R² are fluoro, ethyl, propyl, butyl, isobutyl, pentyl, methylsulfanyl, 2-methoxyethylsulfanyl, 3-methoxypropylsulfanyl, 4-methoxybutylsulfanyl, 4-methoxy-3-methyl-butylsulfanyl, allyloxy, methoxy, ethoxy, propoxy, 4-methoxybutoxy, 3-methoxypropoxy, 2-methoxyethoxy, 3-cyclopropyloxypropoxy, (2-methoxy-cyclopropyl)methoxymethyl, (2-methoxymethyl-cyclopropyl)methoxymethyl, 3-methylsulfanylpropoxy, methoxymethyl, 2-methoxyethoxymethyl, 3-methoxypropoxymethyl, 2-methyl-3-methylsulfanyl-propoxymethyl, cyclopropylmethoxymethyl, ethoxymethyl, 2-methoxyethyl, 3-methoxy-2-methyl-propoxymethyl, 2-methylsulfanylethoxymethyl, methylsulfanylmethyl, 2-methoxyethylsulfanylmethyl, 3-methoxypropylsulfanylmethyl and cyclopropylsulfanylmethyl.

Examples of preferred X radicals are oxygen, methylene, -O-CH₂-CO-NH-, -O-CH₂-CO-N(CH₃)- and -O-CH(CH₃)-CO-NH-.
Particularly preferred for X are, a bond, methylene and oxygen.
Particularly preferred Z radicals are alkylene, -(CH₂)₁₋₂-O- and -CH(CH₃)-;
where, if X is a bond, Z is -(CH₂)₁₋₂-O-

A group of very particularly preferred compounds of the formula (I) or (II), or more preferably of the formula (IA) or (IIA), are compounds where
R is C₁₋₈-alkyl, C₀₋₈-alkyl-carbonyl-amino-C₁₋₈-alkyl, C₁₋₈-alkyl-sulfonyl-C₁₋₈-alkyl,optionally N-mono- or N,N-di-C₁₋₈-alkylated carbamoyl-C₀₋₈-alkyl, optionally N and/or N' mono-, di- or tri-C₁₋₈-alkylated ureido-C₀₋₈-alkyl, heterocyclyl-C₀₋₈-alkyl, or heterocyclylcarbonyl-C₀₋₈-alkyl, each of said radicals being substituted by 1-4 C₁₋₈-alkoxy or hydroxyll;
R¹ is phenyl, pyridyl, 4H-benzo[1,4]oxazin-3-on-6-yl, 3,4-dihydro-2H-benzo[1,4]oxazin-6-yl, 3,4-dihydro-2H-benzo[1,4]thiazin-6-yl, 2,2-dimethyl-3,4-dihydro-2H-benzo[1,4]oxazin-6-yl, 2,2-dimethyl-4H-benzo[1,4]oxazin-3-on-6-yl, 4,4-dimethyl-1,4-dihydrobenzo[d][1,3]oxazin-2-on-8-yl, 3,3-dimethyl-1,3-dihydroindol-2-on-6-yl, 3,3-dimethyl-1,3-dihydroindol-2-on-7-yl, 3,3-dimethyl-1,3-dihydroindol-6-yl, 3-methylindol-6-yl, 1-methyl-indazol-5-yl, 3,4-dihydro-1H-quinolin-2-on-7-yl, 4,4-dimethyl-3,4-dihydro-1H-quinolin-2-on-8-yl, 1H-quinolinyl, 2H-chromenyl, 1,1 a,2,7b-tetrahydro-cyclopropa[c]chromenyl, 1a,7b-dihydro-1H-cyclopropa[c]chromenyl, or spiro[cyclopropane-1,3']-2,3-dihydro-1H-indol-6-yl which may be substituted as specified above, especially by at least one substituent selected from C₁₋₈-alkoxy, C₁₋₈-alkoxy-C₁₋₈-alkoxy, C₁₋₈-alkoxy-C₁₋₈-alkoxy-C₁₋₈-alkyl, C₁₋₈-alkoxy-C₁₋₈-alkyl, C₁₋₈-alkoxycarbonylamino-C₁₋₈-alkoxy, C₁₋₈-alkoxycarbonylamino-C₁₋₈-alkyl, C₁₋₈-alkyl, C₀₋₆-alkylcarbonylamino-C₁₋₈-alkoxy, C₀₋₆-alkylcarbonylamino-C₁₋₈-alkyl, halogen, oxide, triazol-1-yl-C₁₋₈-alkyl, or oxo;
R² is phenyl or halophenyl each substituted by C₂₋₈-alkenyloxy, C₁₋₈-alkoxy, C₁₋₈-alkoxy-C₁₋₈-alkoxy, C₁₋₈-alkoxy-C₁₋₈-alkoxy-C₁₋₈-alkyl, C₁₋₈-alkoxy-C₁₋₈-alkyl, C₁₋₈-alkoxy-C₁₋₈-alkyl-amino-C₁₋₈-alkyl, C₁₋₈-alkyl, C₁₋₈-alkoxy-C₁₋₈-alkylsulfanyl, C₁₋₈-alkylsulfanyl, C₁₋₈-alkylsulfanyl-C₁₋₈-alkyl, C₁₋₈-alkylsulfanyl-C₁₋₈-alkoxy, C₁₋₈-alkylsulfanyl-C₁₋₈-alkoxy-C₁₋₈-alkyl, C₃₋₈-cycloalkylsulfanyl-C₁₋₈-alkyl, C₁₋₈-alkylsulfonyl-C₁₋₈-alkoxy-C₁₋₈-alkyl, C₃₋₈-cycloalkyl-C₀₋₆-alkoxy, C₃₋₈-cycloalkyl-C₀₋₆-alkoxy-C₁₋₈-alkoxy, C₃₋₈-cycloalkyl-C₀₋₆-alkoxy-C₁₋₈-alkyl, C₁₋₆-alkoxy-C₀₋₆-alkyl-C₃₋₈-cycloalkyl-C₀₋₆-alkoxy-C₁₋₈-alkyl, C₃₋₈-cycloalkyl-C₁₋₈-alkylamino-C₁₋₈-alkyl, halogen, heterocyclyl-C₀₋₆-alkoxy, heterocyclyl-C₀₋₆-alkoxy-C₁₋₈-alkyl, C₁₋₈-alkoxy-C₁₋₈-alkylamino-C₁₋₈-alkyl, N-(halo-phenyl)pyrrolidinyloxy, N-(halo-phenyl)pyrrolidinyloxy-C₁₋₈-alkyl , C₁₋₈-alkoxybenzyloxy-C₁₋₈-alkoxy, C₁₋₈-alkoxyphenoxy-C₁₋₈-alkoxy, C₁₋₈-alkoxyphenoxy-C₁₋₈-alkyl, C₁₋₈-alkoxyphenyl-C₁₋₈-alkoxy-C₁₋₈-alkoxy, halobenzyloxy-C₁₋₈-alkoxy, halophenoxy-C₁₋₈-alkoxy, halophenoxy-C₁₋₈-alkoxy-C₁₋₈-alkyl, halophenoxy-C₁₋₈-alkyl, halophenyl-C₁₋₈-alkoxy-C₁₋₈-alkoxy or C₁₋₈-alkylbenzyloxy-C₁₋₈-alkoxy, whereby at least one substituent is in the para-position relative to the bond of R² to the rest of the molecule. Preferred substituents --in the para-position relative to the bond of R² to the rest of the molecule-- on R² are fluoro, ethyl, propyl, butyl, isobutyl, pentyl, methylsulfanyl, 2-methoxyethylsulfanyl, 3-methoxypropylsulfanyl, 4-methoxybutylsulfanyl, 4-methoxy-3-methyl-butylsulfanyl, allyloxy, methoxy, ethoxy, propoxy, 4-methoxybutyloxy, 3-methoxypropoxy, 2-methoxyethoxy, 3-cyclopropyloxypropoxy, (2-methoxy-cyclopropyl)methoxymethyl, (2-methoxymethylcyclopropyl)methoxymethyl, 3-methylsulfanylpropoxy, methoxymethyl, 2-methoxyethoxymethyl, 3-methoxypropoxymethyl, 2-methyl-3-methylsulfanyl-propoxymethyl, cyclopropylmethoxymethyl, ethoxymethyl, 2-methoxyethyl, 3-methoxy-2-methylpropoxymethyl, 2-methylsulfanylethoxymethyl, methylsulfanylmethyl, 2-methoxyethylsulfanylmethyl, 3-methoxypropylsulfanylmethyl and cyclopropylsulfanylmethyl;
R³ is hydrogen;
_{R}⁴ is hydrogen;
Q is absent;
X is a bond, methylene or oxygen;
Z is alkylene, -(CH₂)₁₋₂-O- and -CH(CH₃)-;
where, if X is a bond, Z is -(CH₂)₁₋₂-O-
m = 0 and
n = 1.

The compounds of the formulae (I) and (II), or preferably of the formula (IA) or (IIA), and their pharmaceutically useable salts have inhibiting action on the natural enzyme renin. The latter passes from the kidneys into the blood and there brings about the cleavage of angiotensinogen to form the decapeptide angiotensin I which is then cleaved in the lung, the kidneys and other organs to the octapeptide angiotensin II. Angiotensin II increases the blood pressure both directly by arterial constriction and indirectly by the release of the hormone aldosterone which inhibits the release of the sodium ion from the adrenal glands, which is associated with a rise in the extracellular liquid volume. This rise can be attributed to the action of angiotensin II itself or of the heptapeptide angiotensin III formed therefrom as a cleavage product. Inhibitors of the enzymatic activity of renin bring about a reduction in the formation of angiotensin I and, as a consequence thereof, the formation of a smaller amount of angiotensin II. The reduced concentration of this active peptide hormone is the immediate cause of the hypotensive action of renin inhibitors.

One experimental procedure of detecting the action of renin inhibitors is by means of in vitro tests, in which the reduction of the formation of angiotensin I in different systems (human plasma, purified human renin together with synthetic or natural renin substrate) is measured. One in vitro test which is used is the one according to Nussberger et. al (1987) J. Cardiovascular Pharmacol., Vol. 9, p. 39-44 which follows. This test measures the formation of angiotensin I in human plasma. The amount of angiotensin I formed is determined in a subsequent radioimmunoassay. Which action inhibitors have on the formation of angiotensin I is tested in this system by the addition of different concentrations of these substances. The IC₅₀ refers to that concentration of the particular inhibitor which reduces the formation of angiotensin I by 50%. The compounds of the present invention exhibit inhibiting actions in the in vitro systems at minimum concentrations of about 10⁻³ to about 10⁻¹⁰ mol/I.

Illustrative of the invention, the compounds of examples 10, 12, 21, 33, 40, 53, and 120 inhibit the formation of angiotensin I with IC₅₀ values in the range of about 10⁻⁵ to about 10⁻⁷ mol/I.

In salt-depleted animals, renin inhibitors bring about a blood pressure decrease. Human renin differs from renin of other species. To test inhibitors of human renin, primates (marmosets, Callithrixjacchus) are used, because human renin and primate renin are substantially homologous in the enzymatically active region. One in vivo test which is used is as follows: the test compounds are tested on normotensive marmosets of both genders and having a body weight of about 350 g which are conscious, able to move freely and in their normal cages. Blood pressure and heart rate are measured using a catheter in the descending aorta and recorded radiometrically. The endogenous release of renin is stimulated by the combination of a 1-week low-salt diet with a single intra-muscular injection of furosemide (5-(aminosulfonyl)-4-chloro-2-[(2-furanylmethyl)amino]benzoic acid) (5 mg/kg). 16 hours after the injection of furosemide, the test substances are administered either directly into the femoral artery by means of an injection cannular or into the stomach by gavage as a suspension or solution, and their effect on blood pressure and heart rate was evaluated. The compounds of the present invention effectively reduce blood pressure in the in vivo test described at doses of about 0.003 to about 0.3 mg/kg i.v. and at doses of about 0.3 to about 30 mg/kg p.o.

The compounds of the formulae (I) or (II), or preferably of the formula (IA) or (IIA), and their pharmaceutically useable salts may find use as medicaments, for example in the form of pharmaceutical preparations. The pharmaceutical preparations may be administered enterally, such as orally, for example in the form of tablets, coated tablets, sugar-coated tablets, hard and soft gelatine capsules, solutions, emulsions or suspensions, nasally, for example in the form of nasal sprays, rectally, for example in the form of suppositories, or transdermally, for example in the form of ointments or patches. The administration may also be parenteral, such as intramuscular or intravenous, for example in the form of injection solutions.

To prepare tablets, coated tablets, sugar-coated tablets and hard gelatine capsules, the compounds of the formulae (I) or (II), or preferably of the formula (IA) or (IIA), and pharmaceutically useable salts thereof, may be processed with pharmaceutically inert, inorganic or organic excipients. Such excipients used, for example for tablets, coated tablets and hard gelatine capsules, may be lactose, corn starch, or derivatives thereof, talc, stearic acid or salts thereof etc.
Suitable excipients for soft gelatine capsules are, for example, vegetable oils, waxes, fats, semisolid and liquid polyols, etc.
Suitable excipients for preparing solutions and syrups are, for example, water, polyols, sucrose, invert sugar, glucose, etc.
Suitable excipients for injection solutions are, for example, water, alcohols, polyols, glycerol, vegetable oils, bile acids, lecithin, etc.
Suitable excipients for suppositories are, for example, natural or hardened oils, waxes, fats, semisolid or liquid polyols, etc.
The pharmaceutical preparations may additionally also comprise preservatives, solubilizers, viscosity-increasing substances, stabilizers, wetting agents, emulsifiers, sweeteners, colorants, flavourings, salts for altering the osmotic pressure, buffers, coatings or antioxidants. They may also comprise other therapeutically valuable substances.

The present invention further provides the use of the compounds of the formulae (I) or (II), or preferably of the formula (IA) or (IIA), and the pharmaceutically useable salts thereof, in the treatment or prevention of hypertension and heart failure, and also glaucoma, cardiac infarction, kidney failure and restenoses of mammals, especially of human beings.

The compounds of the formulae (I) or (II), or preferably of the formula (IA) or (IIA), and the pharmaceutically useable salts thereof, may also be administered in combination with one or more agents having cardiovascular action, for example α-and β-blockers such as phentolamine, phenoxybenzamine, prazosin, terazosin, tolazine, atenolol, metoprolol, nadolol, propranolol, timolol, carteolol etc.; vasodilators such as hydralazine, minoxidil, diazoxide, nitroprusside, flosequinan etc.; calcium antagonists such as amrinone, bencyclan, diltiazem, fendiline, flunarizine, nicardipine, nimodipine, perhexilene, verapamil, gallopamil, nifedipine etc.; ACE inhibitors such as cilazapril, captopril, enalapril, lisinopril etc.; potassium activators such as pinacidil; anti-serotoninergics such as ketanserin; thromboxane-synthetase inhibitors; neutral endopeptidase inhibitors (NEP inhibitors); angiotensin II antagonists; and also diuretics such as hydrochlorothiazide, chlorothiazide, acetazolamide, amiloride, bumetanide, benzthiazide, ethacrynic acid, furosemide, indacrinone, metolazone, spironolactone, triamteren, chlorthalidone etc.; sympatholytics such as methyldopa, clonidine, guanabenz, reserpine; and other agents which are suitable for the treatment of hypertension, heart failure or vascular diseases in humans and animals which are associated with diabetes or renal disorders such as acute or chronic renal failure. Such combinations may be employed separately or in preparations which comprise a plurality of components.

Further substances which can be used in combination with the compounds of the formulae (I) or (II), or preferably of the formula (IA) or (IIA), are the compounds of classes (i) to (ix) on page 1 of WO 02/40007 (and also the preferences and examples further listed therein) and the substances specified on pages 20 and 21 of WO 03/027091.

The dose may vary within wide limits and has of course to be adapted to the individual circumstances in each individual case. In general, for oral administration, a daily dose of about 3 mg to about 3 g, preferably about 10 mg to about 1 g, for example about 300 mg, per adult (70 kg), divided into preferably 1-3 individual doses which may, for example, be of equal size, may be appropriate, although the upper limit specified may also be exceeded if this should be found to be appropriate; typically, children receive a lower dose according to their age and body weight.

### Examples

The examples which follow illustrate the present invention. All temperatures are reported in degrees Celsius, pressures in mbar. Unless stated otherwise, the reactions take place at room temperature. The abbreviation "Rf = xx (A)" means, for example, that the Rf value xx is obtained in the solvent system A. The ratio of the solvents relative to one another is always reported in parts by volume. Chemical names of end products and intermediates were obtained with the aid of the program AutoNom 2000 (Automatic Nomenclature).

HPLC gradient on Hypersil BDS C-18 (5 µm); column: 4 x 125 mm 90% water*/10% acetonitrile* to 0% water*/100% acetonitrile* in 5 minutes + 2.5 minutes (1.5 ml/min); *: containing 0.1% trifluoroacetic acid

The following abbreviations are used:

| | |
|---|---|
| Rf | ratio of distance which a substance travels to distance of the eluent front from the start point in thin layer chromatography |
| Rt | retention time of a substance in HPLC (in minutes) |
| m.p. | melting point (temperature) |

### General procedure A (N-Tos-deprotection)

To a stirred solution of 0.09 mmol "tosylamide" in 10 ml of methanol are added 0.44 mmol sodiumdihydrogenphosphate and 0.90 mmol of sodium amalgam (10% Na) at room temperature. The reaction mixture is stirred for 2-18 hours, diluted with water and extracted with ethyl acetate. The organic phases are combined, washed with brine and dried over sodium sulfate. The solvent is concentrated under reduced pressure and the residue is purified by flash chromatography (SiO₂ 60F) to afford the title compound.

### General procedure B: (N-p-Methoxyphenyl deprotection)

To a stirred solution of 0.5 mmol "p-methoxyanilin" in 10 ml of acetonitrile/water (1:1) is added a solution of 1.43 mol of ceric ammonium nitrate in 5.0 ml of water at 0°C. The mixture is stirred for 30 min, followed by addition of 1.0 g of sodium sulfite. After additional 30 min, the mixture is diluted with water and extracted with tert-butyl methyl ether. The organic phase is dried and concentrated under reduced pressure. The residue is purified by flash chromatography (SiO₂ 60F) to afford the title compound.

### General procedure C: (BH₃-Reduction)

To a stirred solution of 1 mmol of "lactam" in 3 ml of tetrahydrofuran is admixed with 2-4 mmol of borane tetrahydrofuran (1 M in tetrahydrofuran) and heated to 50°C for 2-8 hours.

The reaction mixture is quenched by addition of 10 ml of methanol and concentrated under reduced pressure. The title compound is obtained from the residue by means of flash chromatography (SiO₂ 60F).

### General procedure D: (Amide-Formation)

To a stirred solution of 1.0 mmol of "acid" and "1.0 mmol of "amin" in 20 ml of dichloromethane are added 5.0 mmol of triethylamin and 1.0 mmol of tripropylphosphonic acid cyclic anhydride [68957-94-8] (50% in ethyl acetate) at room temperature. The reaction mixture is stirred for 1-3 hours, diluted with dichloromethane, washed with 1 N hydrochloric acid and brine. The organic phases are combined, dried over sodium sulfate and the solvent is concentrated under reduced pressure. The residue is purified by flash chromatography (SiO₂ 60F) to afford the title compound.

### General procedure E: (Hydrogenation)

To a stirred solution of 1 mmol of "substrate" in 15 ml of tetrahydrofuran are added 100-200 mg Pd/C 10% and the reaction mixture is hydrogenated at 15-20°C. The reaction mixture is filtered and concentrated under reduced pressure. The residue is purified by flash chromatography (SiO₂ 60F) to afford the title compound.

### General procedure F: (Mesylation)

To a stirred solution of 1 mmol of "alcohol" in 10 ml of dichloromethane are added 5 mmol of triethylamine and 2 mmol of methansulfonyl chloride at 0°C. The reaction mixture is allowed to stir for 1 hour, diluted with dichloromethane, washed with 1 N hydrochloric acid ,and dried over sodium sulfate. The solvent is concentrated under reduced pressure and the residue is purified by flash chromatography (SiO₂ 60F) to afford the title compound or is directly used in the next step without any further purification.

### General procedure G: (substitution of mesylate by nitrile)

A solution of 0.5 mmol of "mesylate" and 5.5 mmol of sodium cyanide in 3 ml of dimethyl sulphoxide is stirred at 60°C for 20 hours. Subsequently, the mixture is diluted with ethyl acetate and washed with brine. The aqueous phase is extracted with ethyl acetate (2X). The combined organic phases are dried with sodium sulphate and concentrated by evaporation. The crude title compound is obtained from the residue.

### General Method H: (nitrile hydrolysis)

A suspension of 3.5 mmol of "nitrile" in 55 ml of ethanol and 55 ml of 2M NaOH is stirred at 80°C for 21 hours. Subsequently, the mixture is concentrated and the residue is adjusted to pH 2-3 with 1 M HCI. The mixture is extracted with ethyl acetate (2X). The residue is diluted with dichloromethane, and washed with 1 M HCI and finally with brine. The combined organic phases are dried over sodium sulphate and concentrated by evaporation. The crude title compound is obtained from the residue.

### General Method I: (alcohol alkylation)

A solution of 235 mmol of "alcohol"in 800 ml of tetrahydrofuran are added 239 mmol of sodium hydride (60% dispersion in oil) in portions. The reaction mixture is stirred for 30 minutes and 235 mmol of "halide" and 282 mmol of tetrabutylammonium iodide are added. The reaction is stirred at 70°C for 5 hours. Ice-water is added and the mixture is extracted with ethyl acetate (2X). The combined organic phases are washed with brine, dried over sodium sulphate and concentrated by evaporation. The title compound is obtained from the residue by means of flash chromatography (SiO₂ 60F).

### General Method J: (methoxyphenol demethylation)

A solution of 3.99 mmol of "methoxyphenol" in 35 ml of N,N-dimethyl-formamide is treated with 39.9 mmol of sodium ethanethiolate and the resulting suspension is heated at 120°C for 21 hours. The reaction mixture is cooled to 0°C and adjusted to pH 2 using 2M HCl. The mixture is diluted with water and extracted with tert-butyl methyl ether (2X). The combined organic phases are dried using sodium sulphate and evaporated. The title compound is obtained from the residue by means of flash chromatography (SiO₂ 60F).

### General Method K: (phenol alkylation)

A suspension of 1 mmol of "phenol", 1.0-1.5 mmol of "tosylate" or "bromide", 1.5 mmol of caesium carbonate and 2 ml of acetonitrile is stirred at 80°C for 2 hours. The reaction mixture is cooled, poured into water and extracted with ethyl acetate (2x). The organic phases are washed with brine, dried with sodium sulphate and evaporated. The title compound is obtained from the residue by flash chromatography (SiO₂ 60F).

### Example 1

{(2S,4R,5R)-4-(4-Methoxy-phenyl)-5-[4-(3-methoxy-propyl)-3,4-dihydro-2H-benzo[1,4]oxazin-6-ylmethoxy]-piperidin-2-yl}-methanol To a stirred solution of 0.10 g of (2S,4R,5R)-4-(4-methoxy-phenyl)-5-[4-(3-methoxy-propyl)-3,4-dihydro-2H-benzo[1,4]oxazin-6-ylmethoxy]-piperidine-2-carboxylic acid ethyl ester in 10 ml of tetrahydrofuran are added 17.0 mg of lithium aluminium hydride at 0°C. The reaction mixture is stirred for 1 hour at this temperature, diluted with tert-butyl methyl ether and quenched by addition of 2.0 ml of aqueous saturated sodium sulfate solution. The mixture is allowed to warm to room temperature, filtered and dried over sodium sulfate. The solvent is concentrated under reduced pressure and the residue is purified by flash chromatography (SiO₂ 60F) to afford the title compound.

The starting materials are prepared as follows:
a) (2S,4R,5R)-4-(4-Methoxy-phenyl)-5-[4-(3-methoxy-propyl)-3,-oxo-3,4-dihydro-2H-benzo[1,4]oxazin-6-ylmethoxyl-piperidine-2-carboxylic acid ethyl ester
   According to general procedure B, 0.3 g of (2S,4R,5R)-1,4-bis-(4-methoxy-phenyl)-5-[4-(3-methoxy-propyl)-3-oxo-3,4-dihydro-2H-benzo[1,4]oxazin-6-ylmethoxy]-piperidine-2-carboxylic acid ethyl ester are used to afford the title compound as a yellow oil. Rf = 0.15 (dichlormethane-methanol-25% ammonia conc. 200:10:1); Rt = 3.53.
b) (2S,4R,5R)-1,4-Bis-(4-methoxy-phenyl)-5-[4-(3-methoxy-propyl)-3-oxo-3,4-dihydro-
   2H-benzo[1,4]oxazin-6-ylmethoxy]-piperidine-2-carboxylic acid ethyl ester To a stirred solution of 5.20 g of (2S,4R,5R)-5-hydroxy-1,4-bis(4-methoxyphenyl)piperidine-2-carboxylic acid ethyl ester and 5.62 g of 2,2,2-trichloro-acetimidic acid 4-(3-methoxy-propyl)-3-oxo-3,4-dihydro-2H-benzo[1,4]oxazin-6-ylmethyl ester in 150 ml of dichloromethane is added 1.1 ml of trifluoromethanesulfonic acid at -30°C. The reaction mixture is allowed to stir for another hour at this temperature and then allowed to warm to 0°C within 2 hours. The mixture is quenched by addition of 2N sodium hydroxide, extracted with dichlormethane, dried over sodium sulfate and concentrated under reduced pressure. The residue is purified by flash chromatography (SiO₂ 60F) to afford the title compound as a brown oil. Rf = 0.15 (EtOAc-heptane 1:1); Rt = 4.73.
c) (2S,4R,5R)-5-Hydroxy-1,4-bis(4-methoxy-phenyl)piperidine-2-carboxylic acid ethyl ester
   To a stirred solution of 19.5 g of (S)-1,4-bis-(4-methoxy-phenyl)-1,2,3,6-tetrahydropyridine-2-carboxylic acid ethyl ester in 400 ml of tetrahydrofuran are added 56 ml of a solution of borane-tetrahydrofuran (1 M) at room temperature. The reaction mixture is heated to 40°C for 2-6 hours, cooled to room temperature, and quenched by the addition of 50 ml of 2N sodium hydroxide solution and 100 ml of 30% hydrogen peroxide solution. The organic phase is separated, dried over sodium sulfate and concentrated under reduced pressure. The residue is purified by flash chromatography (SiO₂ 60F) to afford the title compound as a yellow solid. Rf = 0.33 (EtOAc-heptane 1:1); Rt = 3.58.
d) (S)-1,4-Bis-(4-methoxy-phenyl)-1,2,3,6-tetrahydro-pyridine-2-carboxylic acid ethyl ester
   To a suspension of 38.0 g of (S)-1-(4-methoxy-phenyl)-4-trifluoromethanesulfonyloxy-1,2,3,6-tetrahydropyridine-2-carboxylic acid ethyl ester, 18.1 g of 4-methoxyphenylboronic acid, 6.45 g of lithiumchloride in 300 ml of dimethoxyethan are added 100 ml of ethanol, 160 ml of 2N sodium carbonat solution and 2.22 g of tetrakis(triphenylphosphine)palladium. The reaction mixture is heated to 75°C for 3-6 hours and allowed to cool to room temperature overnight. The reaction mixture is filtered and the filtercake is redissolved in dichloromethane and water. The suspension is filtered again and the organic phase is concentrated under reduced pressure to afford the title compound as a colourless solid. Rf = 0.25 (EtOAc-heptane 1:4); Rt = 5.00.
e) (S)-1(4-Methoxy-phenyl)-4-trifluoromethanesulfonyloxy-1,2,3,6-tetrahydropyridine-2-carboxylic acid ethyl ester
   To a stirred solution of 0.94 g (S)-1-(4-methoxy-phenyl)-4-oxo-1,2,3,4-tetrydropyridine-2-carboxylic acid ethyl ester [690224-49-8] in 20 ml tetrahydrofuran is added a solution of 3.7 ml of L-selectride (0.5M, Aldrich 17,849-7) at -78°C. The mixture is stirred for 1 hour and a solution of 1.18 g N-phenyltrifluoromethanesulfonimide (Fluka 78175) in 8 ml of tetrahydrofuran is added and the mixture is allowed to warm to room temperature overnight. The reaction mixture is concentrated under reduced pressure and the residue is purified by flash chromatography (aluminium oxide, Fluka 06290) to afford the title compound as a brown solid. Rf = 0.2 (EtOAc-heptane 1:9); Rt = 5.14.
f) 2,2,2-Trichloro-acetimidic acid 4-(3-methoxy-propyl)-3-oxo-3,4-dihydro-2H-benzo[1,4]oxazin-6-ylmethyl ester
   To a stirred solution of 8.50 g of 6-hydroxymethyl-4-(3-methoxy-propyl)-4H-benzo[1,4]oxazin-3-one in 200 ml of diethylether is added 1.28 g of sodium hydride at 0°C. The mixture is stirred for 1 hour and concentrated to dryness under reduced pressure to afford a brown oil, which is not further purified and directly used in the next step. Rf = 0.5 (EtOAc-heptane 1:1).
g) 6-Hydroxymethyl-4-(3-methoxy-propyl)-4H-benzo[1,4]oxazin-3-one
   A stirred suspension of 1.79 g of 6-hydroxymethyl-4H-benzo[1,4]oxazin-3-one, 2.20 ml of 1-chlor-3-methoxypropane, 10 g of potassium fluoride on aluminium oxide and 0.033 g potassium iodide in 150 ml of acetonitrile is heated to reflux for 72 hours. The reaction mixture is allowed to cool to room temperature, filtered and concentrated under reduced pressure. The residue is purified by flash chromatography (SiO₂ 60F) to afford the title compound. Rf = 0.60 (dichlormethane - methanol 9:1); Rt = 2.74.
h) 6-Hydroxymethyl-4H-benzo[1,4]oxazin-3-on
   To a mixture of 6.9 g of 3-oxo-3,4-dihydro-2H-benzo[1,4]oxazin-6-carboxylic acid methyl ester [604756-32-3] in 230 ml of tetrahydrofuran are added 88.9 ml of diisobutylaluminiumhydride (1.5M in toluene) at -40°C. The reaction mixture is stirred for 1.5 hours at -40°C to -20°C and poured into 150 ml of 2N hydrochloric acid .The organic phase is separated, washed with brine, dried over sodium sulfate and concentrated under reduced pressure. The residue is crystallized from ethanol to afford the title compound as a beige solid. Rf = 0.16 (EtOAc-heptan 2:1); Rt = 2.23, m.p.: 186 - 187°C.

### Example 15

2-{(2S,4R,5R)-4-(4-Methoxy-phenyl)-5-[4-(3-methoxy-propyl)-3,4-dihydro-2H-benzo[1,4]oxazin-6-ylmethoxy]-piperidin-2-yl}-ethanol According to general procedure E, 70.0 mg of (2S,4R,5R)-2-(2-hydroxy-ethyl)-4-(4-methoxy-phenyl)-5-[4-(3-methoxy-propyl)-3,4-dihydro-2H-benzo[1,4]oxazin-6-ylmethoxy]-piperidine-1-carboxylic acid benzyl ester are used to afford the title compound.

The starting material is prepared as follows:
a) (2S,4R,5R)-2-(2-Hydroxy-ethyl)-4-(4-methoxy-phenyl)-5-[4-(3-methoxy-propyl)-3,4-dihydro-2H-benzo[1,4]oxazin-6-ylmethoxy]-piperidine-1-carboxylic acid benzyl ester
   To a solution of 0.10 g of (2S,4R,5R)-2-carboxymethyl-4-(4-methoxy-phenyl)-5-[4-(3-methoxy-propyl)-3,4-dihydro-2H-benzo[1,4]oxazin-6-ylmethoxy]-piperidine-1-carboxylic acid benzyl ester in 8.0 ml of tetrahydrofuran are added 0.31 ml of a solution of borane tetrahydrofuran (1 M) at 50°C. The reaction mixture is stirred for 2 hours at this temperature, allowed to cool to room temperature and quenchd by addition of methanol. The mixture is concentrated under reduced pressure and the obtained residue is purified by flash chromatography (SiO₂ 60F) to afford the title compound as a colourless oil. Rf = 0.50 (EtOAc-heptane 5:1); Rt = 4.95.
b) (2S,4R,5R)-2-Carboxymethyl-4-(4-methoxy-phenyl)-5-[4-(3-methoxy-propyl)-3,4-dihydro-2H-benzo[1,4]oxazin-6-ylmethoxy]-piperidine-1-carboxylic acid benzyl ester
   To a solution of 45 mg of (2S,4R,5R)-2-(2-diazo-acetyl)-4-(4-methoxy-phenyl)-5-[4-(3-methoxy-propyl)-3,4-dihydro-2H-benzo[1,4]oxazin-6-ylmethoxy]-piperidin-1-carboxylic acid benzyl ester in 10 ml of tetrahydrofuran and 1 ml of water are added 0.027 ml of triethylamine und 8.0 mg of silver trifluoroacetate at -15°C. The reaction mixture is allowed to warm to room temperature, stirred for another 2 hours, diluted with tert-butyl methyl ether and dried over sodium sulfate. The organic phase is concentrated under reduced pressure and the residue is purified by flash chromatography (SiO₂ 60F) to afford the title compound as a brown oil. Rf = 0.25 (EtOAc); Rt = 4.82.
c) (2S,4R,5R)-2-(2-Diazo-acetyl)-4-(4-methoxy-phenyl)-5-[4-(3-methoxy-propyl)-3,4-dihydro-2H-benzo[1,4]oxazin-6-ylmethoxy]-piperidine-1-carboxylic acid benzyl ester
   To a solution of 0.1 g of (2S,4R,5R)-4-(4-methoxy-phenyl)-5-[4-(3-methoxy-propyl)-3,4-dihydro-2H-benzo[1,4]oxazin-6-ylmethoxy]-piperidine-1,2-carboxylic acid 1-benzyl ester in 10 ml of tetrahydrofuran are added 0.023 ml of triethylamine and 0.014 ml of ethyl chloroformate at -15°C. The reaction mixture is allowed to warm to 0°C, stirred for 30 minutes at this temperature and cooled to -10°C, followed by the addition of 20 ml of a solution of diazomethane in diethyl ether (1.5%). The reaction mixture is allowed to warm to room temperature overnight, diluted with tert.-butyl methyl ether and the organic phase is washed with an aqueous solution of saturated sodium bicarbonate, dried over sodium sulfate and concentrated under reduced pressure to afford the title compound after flash chromatography (SiO₂ 60F) as a yellow oil. Rf = 0.16 (EtOAc-heptane 1:1); Rt = 5.12.
d) (2S,4R,5R)-4-(4-Methoxy-phenyl)-5-[4-(3-methoxy-propyl)-3,4-dihydro-2H-benzo[1,4]oxazin-6-ylmethoxylpiperidine-1,2-dicarboxylic acid 1-benzyl ester
   To a solution of 1.5 g of (2S,4R,5R)-4-(4-methoxy-phenyl)-5-[4-(3-methoxy-propyl)-3,4-dihydro-2H-benzo[1,4]oxazin-6-ylmethoxy]-piperidine-2-carboxylic acid ethyl ester in 60 ml of methanol-tetrahydrofuran-water 1:1:1 are added 2.85 ml of a solution of 1 N lithiumhydroxide at 0°C. Stirring is continued for an additional 2 hours at room temperature, followed by addition of 40 ml of ethyl acetate, 20 ml of saturated aqueous sodium carbonate solution and 0.42 ml of benzyl chloroformate. The reaction mixture is stirred for 1 hour, diluted with ethyl acetate and the organic phase is dried over sodium sulfate. The organic phase is concentrated under reduced pressure and the residue is purified by flash chromatography (SiO₂ 60F) to afford the title compound as a yellow oil. Rf = 0.13 (EtOAc); Rt = 4.77.
e) (2S,4R,5R)-4-(4-Methoxy-phenyl)-5-[4-(3-methoxy-propyl)-3,4-dihydro-2H-benzo[1,4]oxazin-6-ylmethoxyl-piperidine-2-carboxylic acid ethyl ester
   According to general procedure B, 0.1 g of (2S,4R,5R)-1,4-bis-(4-methoxy-phenyl)-5-[4-(3-methoxy-propyl)-3-oxo-3,4-dihydro-2H-benzo[1,4]oxazin-6-ylmethoxy]-piperidine-2-carboxylic acid ethyl ester (from example 1 b) are used. The residue is purified by flash chromatography (SiO₂ 60F) to afford the title compound.

### Example 18

6-[(3R,4R,6S)-6-(2-Methoxy-ethyl)-4-(4-methoxy-phenyl)-piperidin-3-yloxymethyl]-4-(3-methoxy-propyl)-3,4-dihydro-2H-benzo[1,4]oxazine According to general procedure E, 47.0 mg of (2S,4R,5R)-2-(2-methoxy-ethyl)-4-(4-methoxy-phenyl)-5-[4-(3-methoxy-propyl)-3,4-dihydro-2H-benzo[1,4]oxazin-6-ylmethoxy]-piperidine-1-carboxylic acid benzyl ester are used to afford the title compound.

The starting materials are prepared as follows:
a) (2S,4R,5R)-2-(2-Methoxy-ethyl)-4-(4-methoxy-phenyl)-5-[4-(3-methoxy-propyl)-3,4-dihydro-2H-benzo[1,4]oxazin-6-ylmethoxy]-piperidine-1-carboxylic acid benzyl ester
   To a solution of 0.10 g of (2S,4R,5R)-2-(2-methanesulfonyloxy-ethyl)-4-(4-methoxyphenyl)-5-[4-(3-methoxy-propyl)-3,4-dihydro-2H-benzo[1,4]oxazin-6-ylmethoxy]-piperidine-1-carboxylic acid benzyl ester in 2.0 ml of N,N-dimethylformamide are added 0.060 ml of a 5M solution of sodium methoxide in methanol at room temperature. The reaction mixture is stirred for 5 hours, diluted with tert-butyl methyl ether, washed with 1 N hydrochloric acid and the organic phase is dried over sodium sulfate. The organic phase is concentrated under reduced pressure and the residue is purified by flash chromatography (SiO₂ 60F) to afford the title compound as a slightly brown oil. Rf = 0.25 (EtOAc-heptane 1:1); Rt = 5.47.
b) (2S,4R,5R)-2-(2-Methanesulfonyloxy-ethyl)-4-(4-methoxy-phenyl)-5-[4-(3-methoxy-propyl)-3,4-dihydro-2H-benzo[1,4]oxazin-6-ylmethoxy]-piperidine-1-carboxylic acid benzyl ester
   According to general procedure F, 0.35 g of (2S,4R,5R)-2-(2-hydroxy-ethyl)-4-(4-methoxy-phenyl)-5-[4-(3-methoxy-propyl)-3,4-dihydro-2H-benzo[1,4]oxazin-6-ylmethoxy]-piperidine-1-carboxylic acid benzyl ester (from example 15a) are used to afford the title compound as a slightly brown oil. Rf = 0.15 (dichlormethane-methanol 10:1); Rt = 5.16.

### Example 29

(R,S)-1-{(2R,4R,5R)-4-(4-Methoxy-phenyl)-5-[4-(3-methoxy-propyl)-3,4-dihydro-2H-benzo[1,4]oxazin-6-ylmethoxy]-piperidin-2-yl]-propan-2-ol (distereomeric mixture) According to general procedure A, 54 mg of (R,S)-1-[(2R,4R,5R)-4-(4-methoxyphenyl)-5-[4-(3-methoxy-propyl)-3,4-dihydro-2H-benzo[1,4]oxazin-6-ylmethoxy]-1-(toluene-4-sulfonyl)-piperidin-2-yl]-propan-2-ol (diastereomeric mixture) are used to afford the title compound as mixture of diastereomers.

The starting materials are prepared as follows:
a) (R,S)-1-[(2R,4R,5R)-4-(4-methoxy-phenyl)-5-[4-(3-methoxy-propyl)-3,4-dihydro-2H-benzo[1,4]oxazin-6-ylmethoxyl-1-(toluene-4-sulfonyl)-piperidin-2-yl]-propan-2-ol (diastereomeric mixture)
   To a stirred solution of 0.5 g of 1-[(2R,4R,5R)-4-(4-methoxy-phenyl)-5-[4-(3-methoxy-propyl)-3,4-dihydro-2H-benzo[1,4]oxazin-6-ylmethoxy]-1-(toluene-4-sulfonyl)-piperidin-2-yl]-propan-2-one in 8.0 ml of tetrahydrofuran are added 1.5 ml of a 1 M solution of borane tetrahydrofuran complex at room temperature. The reaction mixture is stirred for 3 hours, quenched with 60 ml of methanol and concentrated under reduced pressure. The residue is purified by flash chromatography (SiO₂ 60F) to afford the title compound as a yellow oil. Rf = 0.53 (EtOAc-heptane 5:1); Rt = 4.95 and 5.06.
b) 1-[(2R,4R,5R)-4-(4-methoxy-phenyl)-5-[4-(3-methoxy-propyl)-3,4-dihydro-2H-benzo[1,4]oxazin-6-ylmethoxy]-1-(toluene-4-sulfonyl)-piperidin-2-yl]-propan-2-one
   To a stirred solution of 0.79 g of N-methoxy-2-[(2R,4R,5R)-4-(4-methoxy-phenyl)-5-[4-(3-methoxy-propyl)-3,4-dihydro-2H-benzo[1,4]oxazin-6-ylmethoxy]-1-(toluene-4-sulfonyl)-piperidin-2-yl]-N-methyl-acetamide in 10 ml of tetrahydrofuran are added 1.3 ml of a 3M solution of methyl magnesium bromide in tetrahydrofuran at 0°C. The reaction mixture is stirred for 1 hour, diluted with an aqueous solution of 1 N potassium hydrogen sulfate, extracted with tert-butyl methyl ether. The organic phases are combined and dried over sodium sulfate. The residue is purified by flash chromatography (SiO₂ 60F) to afford the title compound as a yellow oil. Rf = 0.20 (EtOAc-heptane 1:1); Rt = 5.14.
c) N-Methoxy-2-[(2R,4R,5R)-4-(4-methoxy-phenyl)-5-[4-(3-methoxy-propyl)-3,4-dihydro-2H-benzo[1,4]oxazin-6-ylmethoxy]-1-(toluene-4-sulfonyl)-piperidin-2-yl]-N-methyl-acetamide
   According to general procedure D, 0.79 g of [(2R,4R,5R)-4-(4-methoxy-phenyl)-5-[4-(3-methoxy-propyl)-3,4-dihydro-2H-benzo[1,4]oxazin-6-ylmethoxy]-1-(toluene-4-sulfonyl)-piperidin-2-yl]-acetic acid are reacted with N,O-dimethylhydroxylamine hydrochloride to afford the title compound as a colourless foam. Rf = 0.44 (EtOAc-heptane 5:1); Rt = 5.00.
d) [(2R,4R,5R)-4-(4-Methoxy-phenyl)-5-[4-(3-methoxy-propyl)-3,4-dihydro-2H-benzo[1,4]oxazin-6-ylmethoxyl-1-(toluene-4-sulfonyl)-piperidin-2-yl]-acetic acid
   According to general procedure H, 0.5 g of [(2S,4R,5R)-4-(4-methoxy-phenyl)-5-[4-(3-methoxy-propyl)-3,4-dihydro-2H-benzo[1,4]oxazin-6-ylmethoxy]-1-(toluene-4-sulfonyl)-piperidin-2-yl]-acetonitrile are used to afford the title compound as a green foam. Rf = 0.33 (EtOAc-heptane 5:1); Rt = 4.76.
e) [(2S,4R,5R)-4-(4-Methoxy-phenyl)-5-[4-(3-methoxy-propyl)-3,4-dihydro-2H-benzo[1,4]oxazin-6-ylmethoxyl-1-(toluol-4-sulfonyl)-piperidin-2-yl]-acetonitril
   According to general procedure G, 0.5 g of (2S,4R,5R)-methanesulfonic acid 4-(4-methoxy-phenyl)-5-[4-(3-methoxy-propyl)-3,4-dihydro-2H-benzo[1,4]oxazin-6-ylmethoxy]-1-(toluene-4-sulfonyl)-piperidin-2-ylmethyl ester are used to afford the title compound. Rf = 0.26 (EtOAc-heptane 1:1); Rt = 5.20.
f) (2S,4R,5R)-Methanesulfonic acid 4-(4-methoxy-phenyl)-5-[4-(3-methoxy-propyl)-3,4-dihydro-2H-benzo[1,4]oxazin-6-ylmethoxy]-1-(toluene-4-sulfonyl)-piperidin-2-ylmethyl ester
   According to general procedure F, 225 mg of [(2S,4R,5R)-4-(4-methoxy-phenyl)-5-[4-(3-methoxy-propyl)-3,4-dihydro-2H-benzo[1,4]oxazin-6-ylmethoxy]-1-(toluene-4-sulfonyl)-piperidin-2-yl]-methanol are used to afford the title compound. Rf = 0.45 (EtOAc-heptane 1:1); Rt = 5.15.
g) [(2S,4R,5R)-4-(4-Methoxy-phenyl)-5-[4-(3-methoxy-propyl)-3,4-dihydro-2H-benzo[1,4]oxazin-6-ylmethoxy]-1-(toluene-4-sulfonyl)-piperidin-2-yl]-methanol
   Similar to example 1, 0.45 g of (2S,4R,5R)-4-(4-methoxy-phenyl)-5-[4-(3-methoxy-propyl)-3,4-dihydro-2H-benzo[1,4]oxazin-6-ylmethoxy]-1-(toluene-4-sulfonyl)-piperidine-2-carboxylic acid ethyl ester are reacted with lithium aluminium hydride to afford the title compound as a yellow oil. Rf = 0.3 (EtOAc-heptane 2:1); Rt = 4.82.
h) (2S,4R,5R)-4-(4-Methoxy-phenyl)-5-[4-(3-methoxy-propyl)-3,4-dihydro-2H-benzo[1,4]oxazin-6-ylmethoxy]-1-(toluene-4-sulfonyl)-piperidine-2-carboxylic acid ethyl ester
   To a solution of 1.51 g of (2S,4R,5R)-4-(4-methoxy-phenyl)-5-[4-(3-methoxy-propyl)-3,4-dihydro-2H-benzo[1,4]oxazin-6-ylmethoxy]-piperidine-2-carboxylic acid ethyl ester (from example 1d) in 25 ml of dichlormethane are added 1.97 ml of triethylamine, 3.5 mg of 4-dimethylaminopyridine and 0.57 g of 4-toluenesulfonylchloride at 0°C. The reaction mixture is allowed to warm to room temperature overnight, diluted with water and 1 N hydrochloric acid and the organic phase is separated and dried over sodium sulfate. The organic phase is concentrated under reduced pressure and the residue is purified by flash chromatography (SiO₂ 60F) to afford the title compound as a colourless oil. Rf = 0.50 (EtOAc-heptane 1:1); Rt = 5.44.

### Alternative synthesis for example 29g:

[(2S,4R,SR)-4-(4-Methoxy-phenyl)-5-[4-(3-methoxy-propyl)-3,4-dihydro-2H-benzo[1,4]oxazin-6-ylmethoxyl-1-(toluene-4-sulfonyl)-piperidin-2-yl]-methanol To a stirred solution of 17.09 g of 6-[(3R,4R,6S)-4-(4-methoxy-phenyl)-1-(toluene-4-sulfonyl)-6-triisopropylsilanyloxymethyl-piperidin-3-yloxymethyl]-4-(3-methoxy-propyl)-3,4-dihydro-2H-benzo[1,4]oxazine in 200 ml of tetrahydrofuran are added 42 ml of a 1N solution of tetrabutylammonium fluoride in tetrahydrofuran at room temperature. The reaction mixture is stirred for 2 hours at room temperature, diluted with water and extracted with tert-butyl methyl ether. The combined organic phases are dried over sodium sulfate and concentrated under reduced pressure. The crude product is purified by flash chromatography (SiO₂ 60F) to afford the title compound as a clear yellow oil. Rf = 0.30 (EtOAc-heptane 2:1); Rt = 4.82.

The starting materials are prepared as follows:
Aa) 6-[(3R,4R,6S)-4-(4-Methoxy-phenyl)-1-(toluene-4-sulfonyl)-6-triisopropylsilanyloxymethyl-piperidin-3-yloxymethyll-4-(3-methoxy-propyl)-3,4-dihydro-2H-benzo[1,4]oxazine
   According to general procedure C, 19.0 g of 6-[(3R,4R,6S)-4-(4-methoxy-phenyl)-1-(toluene-4-sulfonyl)-6-triisopropylsilanyloxymethyl-piperidin-3-yloxymethyl]-4-(3-methoxy-propyl)-4H-benzo[1,4]oxazin-3-one are used to afford the title compound after flash chromatography as a gold yellow oil. Rf = 0.50 (EtOAc-heptane 1:2); Rt = 6.44.
Ab) 6-[(3R,4R,6S)-4-(4-Methoxy-phenyl)-1-(toluene-4-sulfonyl)-6-triisopropylsilanyloxymethyl-piperidin-3-yloxymethyl]-4-(3-methoxy-propyl)-4H-benzo[1,4]Oxazin-3-one
   To a stirred solution of 22.65 g of (3R,4R,6S)-4-(4-methoxy-phenyl)-1-(toluene-4-sulfonyl)-6-triisopropylsilanyloxymethyl-piperidin-3-ol, 11.6 g of 6-bromomethyl-4-(3-methoxy-propyl)-4H-benzo[1,4]oxazin-3-one and 16.56 g of tetrabutyl ammonium iodide in 200 ml of N,N-dimethylformamide are added 1.63 g of sodium hydride at room temperature. The reaction mixture is stirred for 2 hours at room temperature, poured into ice water and extracted with tert-butyl methyl ether. The organic phases are combined, dried over sodium sulfate and concentrated under reduced pressure. The crude product is purified by flash chromatography (SiO₂ 60F) to afford the title compound as a clear yellow oil. Rf = 0.50 (EtOAc-heptane 1:1); Rt = 6.74.
Ac) (3R,4R,6S)-4-(4-Methoxy-phenyl)-1-(toluene-4-sulfonyl)-6-triisopropylsilanyloxymethyl-piperidin-3-ol
   To a stirred solution of 19.3 g of (3R,4R,6S)-6-hydroxymethyl-4-(4-methoxy-phenyl)-1-(toluene-4-sulfonyl)-piperidin-3-ol in 400 ml of N,N-dimethylformamide is added 10.9 g of imidazole and 6.79 g of triisopropylchlorsilane at room temperature. The reaction mixture is stirred overnight, diluted with 1 N hydrochloric acid and extracted with tert-buty methyl ether. The organic phases are combined, dried over sodium sulfate and concentrated under reduced pressure. The crude product is purified by flash chromatography (SiO₂ 60F) to afford the title compound as a light yellow oil. Rf = 0.60 (EtOAc-heptane 1:1); Rt = 6.39.
Ad) (3R,4R,6S)-6-Hydroxymethyl-4-(4-methoxy-phenyl)-1-(toluene-4-sulfonyl)-piperidin-3-ol
   To a stirred solution of 108.1 g of [(S)-4-(4-methoxy-phenyl)-1-(toluene-4-sulfonyl)-1,2,3,6-tetrahydro-pyridin-2-yl]-methanol in 1000 ml of tetrahydrofuran are added 504 ml of a solution of borane-tetrahydrofuran (1 M) at room temperature. The reaction mixture is heated to 40°C for 2-6 hours, cooled to room temperature, and quenched by the addition of 150 ml of 2N sodium hydroxide solution and 150 ml of 30% hydrogen peroxide solution. The organic phase is separated, dried over sodium sulfate and concentrated under reduced pressure. The residue is purified by flash chromatography (SiO₂ 60F) to afford the title compound as a white solid. Rf = 0.19 (EtOAc-heptane 1:1); Rt = 3.67.
Ae) [(S)-4-(4-methoxy-phenyl)-1-(toluene-4-sulfonyl)-1,2,3,6-tetrahydro-pyridin-2-yl]-methanol
   To a solution of 72.0 g of [(S)-4-(4-methoxy-phenyl)-1,2,3,6-tetrahydro-pyridin-2-yl]-methanol in 1.5 I of ethyl actetate and 1.5 I of 2N aqueous sodium carbonate are added 57.7 g of p-toluenesulfonyl chloride at 0°C. The reaction mixture is stirred overnight, extracted with ethyl acetate and the combined organic phases are dried over sodium sulfate and concentrated under reduced pressure. The crude product is used for the next step without any further purification. Rf = 0.1 (EtOAc-heptane 1:1); Rt = 4.20.
Af) [(S)-4-(4-Methoxy-phenyl)-1,2,3,6-tetrahydro-pyridin-2-yl]-methanol
   To a stirred suspension of 10.9 g of lithium aluminium hydride in 1l of tetrahydrofuran is added a solution of 43 g of (S)-4-(4-methoxy-phenyl)-6-oxo-1,2,3,6-tetrahydropyridine-2-carboxylic acid benzyl ester in 1l of tetrahydrofuran at 70°C. The reaction mixture is stirred for another 3-4 hours at this temperature, cooled to 0°C and quenched by the slow addition of 12.4 ml of water, 12.4 ml of 2N sodium hydroxide and 37 ml of water. The mixture is filtered and the filtrate is concentrated under reduced pressure. The residue is purified by flash chromatography (SiO₂ 60F) to afford the title compound as a white solid. Rf = 0.17 (dichloromethane-methanol-25% ammonia conc. 90:10:1); Rt = 2.55.
Ag) (S)-4-(4-Methoxy-phenyl)-6-oxo-1,2,3,6-tetrahydro-pyridine-2-carboxylic acid benzyl ester
   To a solution of 1.8 g of (S)-4-(4-methoxy-phenyl)-6-oxo-3,6-dihydro-2H-pyridine-1,2-dicarboxylic acid 2-benzyl ester 1-tert-butyl ester in 15 ml of 1,4 dioxane are added 1.2 ml of 4N hydrochloric acid in 1,4 dioxane at room temperature. The reaction mixture is stirred for 1 hour and concentrated under reduced pressure to afford the title compound as an off white solid. Rf = 0.1 (EtOAc-heptane 1:1); Rt = 3.94.
Ah) (S)-4-(4-Methoxy-phenyl)-6-oxo-3,6-dihydro-2H-pyridine-1,2-dicarboxylic acid 2-benzyl ester 1-tert-butyl ester
   To a solution of 2.75 g of 6-oxo-4-(toluene-4-sulfonyloxy)-3,6-dihydro-2H-pyridine-1,2-dicarboxylic acid 2-benzyl ester 1-tert-butyl ester in 18 ml of tetrahydrofuran are added 1.4 g of 4-methoxyphenylboronic acid, 3.15 g of potassium phosphate, 0.12 g of 'ic'cl'hexyl-(2',4',6'-triisopropyl-biphenyl-2-yl)-phosphane (X-PHOS) and 22 mg of palladium acetate under argon. The reaction mixture is heated to 80°C for 2 hours, cooled to room temperature, diluted with ethyl acetate and the organic phase is washed with water and dried over sodium sulfate. The residue is purified by flash chromatography (SiO₂ 60F) to afford the title compound as a brown solid. Rf = 0.35 (EtOAc-heptane 1:2); Rt = 4.95.
Ai) (S)-6-oxo-4-(toluene-4-sulfonyloxy)-3,6-dihydro-2H-pyridine-1,2-dicarboxylic acid 2-benzyl ester 1-tert-butyl ester
   Similar to example 29h, 9.43 g of (2S)-4,6-dioxo-piperidine-1,2-dicarboxylic acid 2-benzyl ester 1-tert-butyl ester [176436-10-5] are reacted with p-toluene sulfonyl chloride to afford the title compound as a yellow oil Rf = 0.64 (EtOAc-heptane 1:1); Rt = 5.20.
j) 6-Bromomethyl-4-(3-methoxy-propyl)-4H-benzo[1,4]oxazin-3-one
   To a solution of 74.0 g of 6-hydroxymethyl-4-(3-methoxy-propyl)-4H-benzo[1,4]oxazin-3-one (from example 1 g) in 600 ml of chloroform are added 58 ml of bromotrimethylsilane at room temperature. The reaction mixture is stirred for 30 minutes and concentrated under reduced pressure. The residue is purified by flash chromatography (SiO₂ 60F) to afford the title compound as an off white solid. Rf = 0.46 (EtOAc-heptane 1:1); Rt = 4.03.

### Example 30

6-[((3R,4R,6R)-4-(4-Methoxy-phenyl)-6-((R,S)-2-methoxy-propyl)-piperidin-3-yloxymethyl]-4-(3-methoxy-propyl)-3,4-dihydro-2H-benzo[1,4]oxazine (diastereomeric mixture)
According to general procedure A, 77.0 mg of 6-[(3R,4R,6R)-4-(4-methoxy-phenyl)-6-((R,S)-2-methoxy-propyl)-1-(toluene-4-sulfonyl)-piperidin-3-yloxymethyl]-4-(3-methoxy-propyl)-3,4-dihydro-2H-benzo[1,4]oxazine are used to afford the title compound.

The starting material is prepared as follows:
a) 6-[(3R,4R,6R)-4-(4-methoxy-phenyl)-6-((R,S)-2-methoxy-propyl)-1-(toluene-4-sulfonyl)-piperidin-3-yloxymethyl]-4-(3-methoxy-propyl)-3,4-dihydro-2H-benzo[1,4]oxazine (diastereomeric mixture)
   To a solution of 87 mg of (R,S)-1-[(2R,4R,5R)-4-(4-methoxy-phenyl)-5-[4-(3-methoxy-propyl)-3,4-dihydro-2H-benzo[1,4]oxazin-6-ylmethoxy]-1-(toluene-4-sulfonyl)-piperidin-2-yl]-propan-2-ol (diastereomeric mixture) (from example 29a) in 2.0 ml of N,N-dimethylformamide are added 59 mg of sodium hydride and 0.110 ml of methyl iodide at 0°C. The reaction mixture is stirred for 1 hour, diluted with 1 N hydrochloric acid and extracted with tert-butyl methyl ether. The organic phases are combined, dried over sodium sulfate and concentrated under reduced pressure. The residue is purified by flash chromatography (SiO₂ 60F) to afford the title compound as a yellow oil. Rf = 0.30 (EtOAc-heptane 1:1); Rt = 5.50.

### Example 34

6-[(3R,4R,6R)-4-(4-Methoxy-phenyl)-6-((R)-2-methoxy-propyl)-piperidin-3-yloxymethyl]-4-(3-methoxy-propyl)-3,4-dihydro-2H-benzo[1,4]oxazine According to general procedure A, 61 mg of 6-[(3R,4R,6R)-4-(4-methoxy-phenyl)-6-((R)-2-methoxy-propyl)-1-(toluene-4-sulfonyl)-piperidin-3-yloxymethyl]-4-(3-methoxy-propyl)-3,4-dihydro-2H-benzo[1,4]oxazine are used to afford the title compound.

The starting materials are prepared as follows:
a) 6-[(3R,4R,6R)-4-(4-Methoxy-phenyl)-6-((R)-2-methoxy-propyl)-1-(toluene-4-sulfonyl)-piperidin-3-yloxymethyl]-4-(3-methoxy-propyl)-3,4-dihydro-2H-benzo[1,4]oxazine
   According to example 30a, 63 mg of 1-[4-(4-methoxy-phenyl)-5-[4-(3-methoxy-propyl)-3,4-dihydro-2H-benzo[1,4]oxazin-6-ylmethoxy]-1-(toluene-4-sulfonyl)-piperidin-2-yl]-propan-(R)-2-ol (diastereomer 1) are used to afford the title compound as a yellow oil. Rt = 5.58.
b) (R)-1-[(2R,4R,5R)-4-(4-Methoxy-phenyl)-5-[4-(3-methoxy-propyl)-3,4-dihydro-2H-benzo[1,4]oxazin-6-ylmethoxyl-1-(toluene-4-sulfonyl)-piperidin-2-yl]-propan-2-ol (diastereomer 1)
   and
   (S)-1-[(2R,4R,5R)-4-(4-Methoxy-phenyl)-5-[4-(3-methoxy-propyl)-3,4-dihydro-2H-benzo[1,4]oxazin-6-ylmethoxy]-1-(toluene-4-sulfonyl)-piperidin-2-yl]-propan-2-ol (diastereomer 2)
   Similiar to example 29a, 1-[(2R,4R,5R)-4-(4-methoxy-phenyl)-5-[4-(3-methoxy-propyl)-3,4-dihydro-2H-benzo[1,4]oxazin-6-ylmethoxy]-1-(toluene-4-sulfonyl)-piperidin-2-yl]-propan-2-one (from example 29b) are used to afford the title compound. The diastereomers are separated by flash chromatography (SiO₂ 60F).
   Diastereomer 1: yellow oil; Rf = 0.18 (EtOAc-heptan 1:1); Rt = 5.10.
   Diastereomer 2: yellow oil; Rf = 0.09 (EtOAc-heptan 1:1); Rt = 4.98.

### Example 35

6-[(3R,4R,6R)-4-(4-Methoxy-phenyl)-6-((S)-2-methoxy-propyl)-piperidin-3-yloxymethyl]-4-(3-methoxy-propyl)-3,4-dihydro-2H-benzo[1,4]oxazine According to general procedure A, 6-[(3R,4R,6R)-4-(4-methoxy-phenyl)-6-((S)-2-methoxy-propyl)-1-(toluene-4-sulfonyl)-piperidin-3-yloxymethyl]-4-(3-methoxy-propyl)-3,4-dihydro-2H-benzo[1,4]oxazine is used to afford the title compound.

The starting material is prepared as follows:
a) 6-[(3R,4R,6R)-4-(4-Methoxy-phenyl)-6-((S)-2-methoxy-propyl)-1-(toluene-4-sulfonyl)-piperidin-3-yloxymethyl]-4-(3-methoxy-propyl)-3,4-dihydro-2H-benzo[1,4]oxazine
   Similar to example 30a, 1-[(3R,4R,6R)-4-(4-methoxy-phenyl)-5-[4-(3-methoxy-propyl)-3,4-dihydro-2H-benzo[1,4]oxazin-6-ylmethoxy]-1-(toluene-4-sulfonyl)-piperidin-2-yl]-propan-(S)-2-ol (diastereomer 2) (from example 34b) is used to afford the title compound as a yellow oil. Rt = 5.54.

### Example 51

1-{(2R,4R,5R)-4-(4-Methoxy-phenyl)-5-[4-(3-methoxy-propyl)-3,4-dihydro-2H-benzo[1,4]oxazin-6-ylmethoxy]-piperidin-2-yl}-2-methyl-propan-2-ol According to general procedure A, 0.095 g of 1-[(2R,4R,5R)-4-(4-methoxy-phenyl)-5-[4-(3-methoxy-propyl)-3,4-dihydro-2H-benzo[1,4]oxazin-6-ylmethoxy]-1-(toluene-4-sulfonyl)-piperidin-2-yl]-2-methyl-propan-2-ol are used to afford the title compound.

The starting materials are prepared as follows:
a) 1-[(2R,4R,5R)-4-(4-Methoxy-phenyl)-5-[4-(3-methoxy-propyl)-3,4-dihydro-2H-benzo[1,4]oxazin-6-ylmethoxy]-1-(toluene-4-sulfonyl)-piperidin-2-yl]-2-methyl-propan-2-ol
   0.34 ml of a methyl magnesium bromide solution (3M in ether) are added to a solution of 0.17 g of [(2R,4R,5R)-4-(4-methoxy-phenyl)-5-[4-(3-methoxy-propyl)-3,4-dihydro-2H-benzo[1,4]oxazin-6-ylmethoxy]-1-(toluene-4-sulfonyl)-piperidin-2-yl]-acetic acid methyl ester in 3 ml of tetrahydrofuran at 0°C. The reaction mixture is worked up after 3 hours by quenching with 1 N aqueous potassium bisulfate solution and extracting with tert-butyl methyl ether (3X). The combined organic layers are washed with brine, dried over sodium sulfate and concentrated under reduced pressure. The residue is purified by flash chromatography (Si0₂ 60F) to afford the title compound as a turbid yellow oil. Rf = 0.12 (EtOAc-heptane 1:1); Rt = 5.11.
b) [(2R,4R,5R)-4-(4-Methoxv-ₗphenyl)-5-[4-(3-methoxv-ₗpro_{lD}yl)-3,4-dihvdro-2H-benzof1,41oxazin-6-ylmethoxyl-1-(toluene-4-sulfonyl)-piperidin-2-yll-acetic acid methyl ester
   4 ml of a solution of diazomethane in ether (0.2M) are added dropwise to a solution of 0.12 g of [(2R,4R,5R)-4-(4-methoxy-phenyl)-5-[4-(3-methoxy-propyl)-3,4-dihydro-2H-benzo[1,4]oxazin-6-ylmethoxy]-1-(toluene-4-sulfonyl)-piperidin-2-yl]-acetic acid in 2 ml of methanol at 0°C. The reaction mixture is worked up after 3.5 hours by quenching with solid magnesium sulfate to decompose any excess diazomethane, filtering and concentrating under reduced pressure to afford the crude title compound as a pale yellow solid with was used in the next step without any further purification. Rf = 0.78 (EtOAc); Rt = 5.26.
c) [(2R,4R,5R)-4-(4-Methoxy-phenyl)-5-[4-(3-methoxy-propyl)-3,4-dihydro-2H-benzo[1,4]oxazin-6-ylmethoxyl-1-(toluene-4-sulfonyl)-piperidin-2-yl]-acetic acid
   According to general procedure H, 0.5 g of [(2S,4R,5R)-4-(4-methoxy-phenyl)-5-[4-(3-methoxy-propyl)-3,4-dihydro-2H-benzo[1,4]oxazin-6-ylmethoxy]-1-(toluene-4-sulfonyl)-piperidin-2-yl]-acetonitrile are used to afford the title compound as a green foam. Rf = 0.33 (EtOAc-heptane 5:1); Rt = 4.76.
d) [(2S,4R,5R)-4-(4-Methoxy-phenyl)-5-[4-(3-methoxy-propyl)-3,4-dihydro-2H-benzo[1,4]oxazin-6-ylmethoxy]-1-(toluol-4-sulfonyl)-piperidin-2-yl]-acetonitril
   According to general procedure G, 0.5 g of (2S,4R,5R)-methanesulfonic acid 4-(4-methoxy-phenyl)-5-[4-(3-methoxy-propyl)-3,4-dihydro-2H-benzo[1,4]oxazin-6-ylmethoxy]-1-(toluene-4-sulfonyl)-piperidin-2-ylmethyl ester are used to afford the title compound. Rf = 0.26 (EtOAc-heptane 1:1); Rt = 5.20.
e) (2S,4R,5R)-Methanesulfonic acid 4-(4-methoxy-phenyl)-5-[4-(3-methoxy-propyl)-3,4-dihydro-2H-benzo[1,4]oxazin-6-ylmethoxyl-1-(toluene-4-sulfonyl)-piperidin-2-ylmethyl ester
   According to general procedure F, 225 mg of [(2S,4R,5R)-4-(4-methoxy-phenyl)-5-[4-(3-methoxy-propyl)-3,4-dihydro-2H-benzo[1,4]oxazin-6-ylmethoxy]-1-(toluene-4-sulfonyl)-piperidin-2-yl]-methanol (from example 29g) are used to afford the title compound. Rf = 0.45 (EtOAc-heptane 1:1); Rt = 5.15.

### Example 52

(S)-1-{(2R,4R,SR)-4-(4-Methoxy-phenyl)-5-[4-(3-methoxy-propyl)-3,4-dihydro-2H-benzo[1,4]oxazin-6-ylmethoxy]-piperidin-2-yl}-3-methyl-butan-2-ol According to general procedure A, 0.058 g of (S)-1-[(2R,4R,5R)-4-(4-methoxyphenyl)-5-[4-(3-methoxy-propyl)-3,4-dihydro-2H-benzo[1,4]oxazin-6-ylmethoxy]-1-(toluene-4-sulfonyl)-piperidin-2-yl]-3-methyl-butan-2-ol (diastereomer 1) are used to afford the title compound.

The starting materials are prepared as follows:
a) (S)-1-[(2R,4R,5R)-4-(4-Methoxy-phenyl)-5-[4-(3-methoxy-propyl)-3,4-dihydro-2H-benzo[1,4]oxazin-6-ylmethoxy]-1-(toluene-4-sulfonyl)-piperidin-2-yl]-3-methylbutan-2-ol (diastereomer 1)
   and
   (R)-1-[(2R,4R,SR)-4-(4-Methoxy-phenyl)-5-[4-(3-methoxy-propyl)-3,4-dihydro-2H-benzo[1,4]oxazin-6-ylmethoxyl-1-(toluene-4-sulfonyl)-piperidin-2-yl]-3-methylbutan-2-ol (diastereomer 2)
   Similar to example 29a, 0.16 g of 1-[(2R,4R,5R)-4-(4-methoxy-phenyl)-5-[4-(3-methoxy-propyl)-3,4-dihydro-2H-benzo[1,4]oxazin-6-ylmethoxy]-1-(toluene-4-sulfonyl)-piperidin-2-yl]-3-methyl-butan-2-one are reduced to provide the title compounds.
   Diastereomer 1: colourless oil; Rf = 0.26 (EtOAc-heptan 1:1); Rt = 5.40.
   Diastereomer 2: colourless oil; Rf = 0.19 (EtOAc-heptan 1:1); Rt = 5.50.
b) 1-[(2R,4R,5R)-4-(4-Methoxy-phenyl)-5-[4-(3-methoxy-propyl)-3,4-dihydro-2H-benzo[1,4]oxazin-6-ylmethoxy]-1-(toluene-4-sulfonyl)-piperidin-2-yl]-3-methylbutan-2-one
   A mixture of 0.19 g of 1-[(2R,4R,5R)-4-(4-methoxy-phenyl)-5-[4-(3-methoxy-propyl)-3,4-dihydro-2H-benzo[1,4]oxazin-6-ylmethoxy]-1-(toluene-4-sulfonyl)-piperidin-2-yl]-3-methyl-but-3-en-2-one and 0.044 g of 10% Pd/C in 5 ml of ethanol is hydrogenated at room temperature for 30 minutes. The reaction mixture is clarified by filtration and concentrated to afford the crude title compound as a light brown oil which is used in the next step without any further purification. Rf = 0.30 (EtOAc-heptane 1:1); Rt = 5.54.
c) 1-[(2R,4R,5R)-4-(4-Methoxy-phenyl)-5-[4-(3-methoxy-propyl)-3,4-dihydro-2H-benzo[1,4]oxazin-6-ylmethoxyl-1-(toluene-4-sulfonyl)-piperidin-2-yl]-3-methyl-but-3-en-2-one
   Similar to example 29b, 0.20 g of N-methoxy-2-[(2R,4R,5R)-4-(4-methoxy-phenyl)-5-[4-(3-methoxy-propyl)-3,4-dihydro-2H-benzo[1,4]oxazin-6-ylmethoxy]-1-(toluene-4-sulfonyl)-piperidin-2-yl]-N-methyl-acetamide (from example 29c) are reacted with isopropenyl magnesium bromide (0.5M in tetrahydrofuran) to afford the crude title compound as a yellow-brown oil which is used in the next step without any further purification. Rf = 0.22 (EtOAc-heptane 1:1); Rt = 5.49.

### Example 53

(R)-1-{(2R,4R,5R)-4-(4-Methoxy-phenyl)-5-[4-(3-methoxy-propyl)-3,4-dihydro-2H-benzo[1,4]oxazin-6-ylmethoxy]-piperidin-2-yl}-3-methyl-butan-2-ol According to general procedure A, 0.024 g of (R)-1-[(2R,4R,5R)-4-(4-methoxyphenyl)-5-[4-(3-methoxy-propyl)-3,4-dihydro-2H-benzo[1,4]oxazin-6-ylmethoxy]-1-(toluene-4-sulfonyl)-piperidin-2-yl]-3-methyl-butan-2-ol (diastereomer 2) (from example 52a) are used to afford the title compound.

### Example 110

(R,S)-1-Methoxy-3-{(2R,4R,5R)-4-(4-methoxy-phenyl)-5-[4-(3-methoxy-propyl)-3,4-dihydro-2H-benzo[1,4]oxazin-6-ylmethoxy]-piperidin-2-yl}-2-methyl-propan-2-ol (diastereomeric mixture)
According to general procedure A, 200 mg of (R,S)-1-methoxy-3-{(2R,4R,5R)-4-(4-methoxy-phenyl)-5-[4-(3-methoxy-propyl)-3,4-dihydro-2H-benzo[1,4]oxazin-6-ylmethoxy]-piperidin-2-yl}-2-methyl-propan-2-ol (diastereomeric mixture) are used to afford the title compound.

The starting material is prepared as follows:
a) (R,S)-1-Methoxy-3-{(2R,4R,5R)-4-(4-methoxy-phenyl)-5-[4-(3-methoxy-propyl)-3,4-dihydro-2H-benzo[1,4]oxazin-6-ylmethoxy]-piperidin-2-yl}-2-methyl-propan-2-ol (diastereomeric mixture)
   To a stirred solution of 1.0 g of (R,S)-1-[(2R,4R,5R)-4-(4-methoxy-phenyl)-5-[4-(3-methoxy-propyl)-3,4-dihydro-2H-benzo[1,4]oxazin-6-ylmethoxy]-1-(toluene-4-sulfonyl)-piperidin-2-yl]-propan-2-one (from example 29b) in 15 ml of methanol are added 360 mg of trimethylsulfoxoniumiodide and 273 mg of sodium hydroxide and the reaction mixture is heated to 70°C for 1 day. The reaction mixture is concentrated under reduced pressure and the residue is taken up in water and dichloromethane. The organic phase is dried and concentrated under reduced pressure to afford the crude title compound. The residue is purified by flash chromatography (SiO₂ 60F) to afford the title compound as a yellow oil. Rf = 0.39 (EtOAc-heptane 1:1); Rt = 5.09.

### Example 111

(R,S)-4-Methoxy-1-{(2R,4R,5R)-4-(4-methoxy-phenyl)-5-[4-(3-methoxy-propyl)-3,4-dihydro-2H-benzo[1,4]oxazin-6-ylmethoxyl-piperidin-2-yl}-2-methyl-butan-2-ol (diastereomeric mixture)
According to general procedure A, 65 mg of (R,S)-4-methoxy-1-[(2R,4R,5R)-4-(4-methoxy-phenyl)-5-[4-(3-methoxy-propyl)-3,4-dihydro-2H-benzo[1,4]oxazin-6-ylmethoxy]-1-(toluene-4-sulfonyl)-piperidin-2-yl]-2-methyl-butan-2-ol (diastereomeric mixture) are used to afford the title compound.

The starting materials are prepared as follows:
a) (R,S)-4-Methoxy-1-[(2R,4R,5R)-4-(4-methoxy-phenyl)-5-[4-(3-methoxy-prolpyl)-3,4-dihydro-2H-benzo[1,4]oxazin-6-ylmethoxyl-1-(toluene-4-sulfonyl)-piperid-2-yl]-2-methyl-butan-2-ol (diastereomeric mixture)
   Similar to example 29b, 4-methoxy-1-[(2R,4R,5R)-4-(4-methoxy-phenyl)-5-[4-(3-methoxy-propyl)-3,4-dihydro-2H-benzo[1,4]oxazin-6-ylmethoxy]-1-(toluene-4-sulfonyl)-piperidin-2-yl]-butan-2-one is reacted with methyl magnesium bromide to afford the title compound as a yellow oil. Rf = 0.22 (EtOAc-heptane 1:1); Rt = 5.12.
b) 4-Methoxy-1-[(2R,4R,5R)-4-(4-methoxy-phenyl)-5-[4-(3-methoxy-propyl)-3,4-dihydro-2H-benzo[1,4]oxazin-6-ylmethoxy]-1-(toluene-4-sulfonyl)-piperidin-2-yl]-butan-2-one
   To a stirred solution of 0.5 g of 1-[(2R,4R,5R)-4-(4-methoxy-phenyl)-5-[4-(3-methoxy-propyl)-3,4-dihydro-2H-benzo[1,4]oxazin-6-ylmethoxy]-1-(toluene-4-sulfonyl)-piperidin-2-yl]-but-3-en-2-one in 1.5 ml of methanol is added 38 mg of 2,8,9-tri-sec-butyl-2,5,8,9-tetraaza-1-phospha-bicyclo[3.3.3]undecane and the reaction mixture is heated to 35°C for 1 day. The reaction mixture is diluted with 1 N hydrochloric acid solution and extracted with ethyl acetate. The organic phases are combined, dried and concentrated under reduced pressure to afford the crude title compound. The residue is purified by flash chromatography (SiO₂ 60F) to afford the title compound as a yellow oil. Rf = 0.25 (EtOAc-heptane 2:3); Rt = 5.16.
c) 1-[(2R,4R,5R)-4-(4-Methoxy-phenyl)-5-[4-(3-methoxy-propyl)-3,4-dihydro-2H-benzo[1,4]oxazin-6-ylmethoxy]-1-(toluene-4-sulfonyl)-piperidin-2-yl]-but-3-en-2-one
   Similar to example 29b, using N-methoxy-2-[(2R,4R,5R)-4-(4-methoxy-phenyl)-5-[4-(3-methoxy-propyl)-3,4-dihydro-2H-benzo[1,4]oxazin-6-ylmethoxy]-1-(toluene-4-sulfonyl)-piperidin-2-yl]-N-methyl-acetamide (from example 29c) and vinyl magnesiumbromide to afford the title compound as a yellow oil. Rf = 0.50 (EtOAc-heptane 1:1 ); Rt = 5.23.

### Example 116

N-(2-Methoxy-ethyl)-3-{(2S,4R,5R)-4-(4-methoxy-phenyl)-5-[4-(3-methoxy-propyl)-3,4-dihydro-2H-benzo[1,4]oxazin-6-ylmethoxy]-piperidin-2-yl}-2,2-dimethyl-propionamide
According to general procedure A, 180 mg of N-(2-methoxy-ethyl)-3-[(2S,4R,5R)-4-(4-methoxy-phenyl)-5-[4-(3-methoxy-propyl)-3,4-dihydro-2H-benzo[1,4]oxazin-6-ylmethoxy]-1-(toluene-4-sulfonyl)-piperidin-2-yl]-2,2-dimethyl-propionamide are used to afford the title compound.

The starting material is prepared as follows:
a) N-(2-Methoxy-ethyl)-3-[(2S,4R,5R)-4-(4-methoxy-phenyl)-5-[4-(3-methoxy-propyl)-3,4-dihydro-2H-benzo[1,4]oxazin-6-ylmethoxy]-1-(toluene-4-sulfonyl)-piperidin-2-yl]-2,2-dimethyl-propionamide
   According to general procedure D, 150 mg of 3-[(2S,4R,5R)-4-(4-methoxy-phenyl)-5-[4-(3-methoxy-propyl)-3,4-dihydro-2H-benzo[1,4]oxazin-6-ylmethoxy]-1-(toluene-4-sulfonyl)-piperidin-2-yl]-2,2-dimethyl-propionic acid and 2-methoxyethylamine are used to afford the title compound as a brown oil. Rf = 0.18 (EtOAc-heptane 5:1); Rt = 5.05.
b) 3-[(2S,4R,5R)-4-(4-Methoxy-phenyl)-5-[4-(3-methoxy-propyl)-3,4-dihydro-2H-benzo[1,4]oxazin-6-ylmethoxy]-1-(toluene-4-sulfonyl)-piperidin-2-yl]-2,2-dimethyl-propionic acid
   To a solution of 8.62 g of 3-[(2S,4R,5R)-4-(4-methoxy-phenyl)-5-[4-(3-methoxy-propyl)-3,4-dihydro-2H-benzo[1,4]oxazin-6-ylmethoxy]-1-(toluene-4-sulfonyl)-piperidin-2-yl]-2,2-dimethyl-propionic acid methyl ester in 20 ml tetrahydrofuran and 50 ml ethanol are added 60 ml of a 20% sodium hydroxide solution. The reaction mixture is stirred for 4 hours at 65°C. The organic solvents are evaporated under reduced pressure and the remaining solution is acidified with 6M aqueous hydrochloric acid until pH 2. This mixture is extracted with 200 ml ethyl acetate (3x). The combined organic layers are washed with brine and dried with sodiumsulfate. The organic layer is filtered and evaporated under reduced pressure. The residue is purified by flash chromatography (SiO₂ 60F) to afford the title compound as a colourless foam. Rf = 0.27 (EtOAc-heptane 3:1); Rt = 5.10.
c) 3-[(2S,4R,5R)-4-(4-Methoxy-phenyl)-5-[4-(3-methoxy-propyl)-3,4-dihydro-2H-benzo[1,4]oxazin-6-ylmethoxyl-1-(toluene-4-sulfonyl)-piperidin-2-yl]-2,2-dimethyl-propionic acid methyl ester
   To a solution of 5.75 ml methyl isobutyrate in 28 ml tetrahydrofuran at -78°C are added 100 ml of a 0.5M lithiumdiisopropyl amide solution and the reaction mixture is stirred for 30 minutes at -78°C. Then 17.74 ml hexamethylphosphoramide is added at -78°C and the mixture is stirred for further 30 minutes. A solution of 8.66 g 6-[(3R,4R,6S)-6-bromomethyl-4-(4-methoxy-phenyl)-1-(toluene-4-sulfonyl)-piperidin-3-yloxymethyl]-4-(3-methoxy-propyl)-3,4-dihydro-2H-benzo[1,4]oxazine in 28 ml tetrahydrofuran is then added dropwise to the "enolate" at -78°C and the solution is stirred for 30 minutes. The reaction mixture is allowed to warm to -12°C and stirred at this temperature for additional 40 minutes. The solution is quenched with 1 M aqueous hydrochloric acid and extracted with 200 ml ethyl acetate (3x). The combined organic layers are washed with brine and dried with sodium sulfate. The organic layer is filtered and evaporated under reduced pressure. The residue is purified by flash chromatography (SiO₂ 60F) to afford the title compound as a colourless foam. Rf = 0.29 (EtOAc-heptane 1:1); Rt = 5.65.
d) 6-[(3R,4R,6S)-6-Bromomethyl-4-(4-methoxy-phenyl)-1-(toluene-4-sulfonyl)-piperidin-3-yloxymethyl]-4-(3-methoxy-propyl)-3,4-dihydro-2H-benzo[1,4]oxazine
   To a solution of 11.18 g of methanesulfonic acid (2S,4R,5R)-4-(4-methoxy-phenyl)-5-[4-(3-methoxy-propyl)-3,4-dihydro-2H-benzo[1,4]oxazin-6-ylmethoxy]-1-(toluene-4-sulfonyl)-piperidin-2-ylmethyl ester in 40 ml dimethylformamide is added 13.78 g lithium bromide and the reaction mixture is stirred for 14 hours at 65°C. The reaction mixture is allowed to warm to room temperature and 50 ml water is added. This mixture is extracted with 300 ml tert-butyl methyl ether (3x). The combined organic layers are washed with brine and dried with sodium sulfate. The organic layer is filtered and evaporated under reduced pressure. The residue is purified by flash chromatography (SiO₂ 60F) to afford the title compound as a colourless foam. Rf = 0.39 (EtOAc-heptane 1:1); Rt = 5.68.
e) (2S,4R,5R)-Methanesulfonic acid 4-(4-methoxy-phenyl)-5-[4-(3-methoxy-propyl)-3,4-dihydro-2H-benzo[1,4]oxazin-6-ylmethoxyl-1-(toluene-4-sulfonyl)-piperidin-2-ylmethyl ester
   According to general procedure F, 225 mg of [(2S,4R,5R)-4-(4-methoxy-phenyl)-5-[4-(3-methoxy-propyl)-3,4-dihydro-2H-benzo[1,4]oxazin-6-ylmethoxy]-1-(toluene-4-sulfonyl)-piperidin-2-yl]-methanol (from example 29g) are used to afford the title compound. Rf = 0.45 (EtOAc-heptane 1:1); Rt = 5.15.

### Example 120

(2R,4R,5R)-4-(4-Methoxy-phenyl)-5-[4-(3-methoxy-propyl)-3,4-dihydro-2H-benzo[1,4]oxazin-6-ylmethoxy]-piperidin-2-yl}-methanol
The title compound is prepared according to example 1, starting from 0.126 g (2R,4R,5R)-4-(4-methoxy-phenyl)-5-[4-(3-methoxy-propyl)-3-oxo-3,4-dihydro-2H-benzo[1,4]oxazin-6-ylmethoxy]-piperidine-2-carboxylic acid ethyl ester (from example 119).

The starting materials are prepared as follows:
a) (2R,4R,5R)-4-(4-Methoxy-phenyl)-5-[4-(3-methoxy-propyl)-3-oxo-3,4-diyhdro-2H-benzo[1,4]oxazin-6-ylmethoxy]-piperidine-2-carboxylic acid ethyl ester
   The title compound is prepared according to general procedure B starting from (2R,4R,5R)-1,4-bis-4-(4-methoxy-phenyl)-5-[4-(3-methoxy-propyl)-3-oxo-3,4-dihydro-2H-benzo[1,4]oxazin-6-ylmethoxy]-piperidine-2-carboxylic acid ethyl ester.
b) (2R,4R,5R)-1,4-Bis-4-(4-methoxy-phenyl)-5-[4-(3-methoxy-propyl)-3-oxo-3,4-dihydro-2H-benzo[1,4]oxazin-6-ylmethoxy]-piperidine-2-carboxylic acid ethyl ester
   A solution of 1.000 g (2S,4R,5R)-5-hydroxy-1,4-bis-(4-methoxy-phenyl)-piperidine-2-carboxylic acid ethyl ester (from example 1c) and 0.783 g 6-bromomethyl-4-(3-methoxy-propyl-4H-benzo[1,4]oxazin-3-one in 15 ml dry N,N-dimethylformamide is cooled to -20°C. Sodium hydride 0.100 g, (60% dispersion in oil) is added and the reaction mixture is warmed to room temperature over 8 hours. The reaction is quenched by addition of 1 M aqueous hydrochloric acid solution. The precipitated solid is isolated by filtration and further purified by flash chromatography (SiO₂ 60F). Rf = 0.29 (EtOAc-heptane 1:1); Rt = 5.20.
c) 6-Bromomethyl-4-(3-methoxy-propyl-4H-benzo[1,4]oxazin-3-one
   Bromotrimethylsilane (11.7 ml) is added dropwise to a solution of 15.0 g 6-hydroxymethyl-4-(3-methoxy-propyl-4H-benzo[1,4]oxazin-3-one (from example 1 h) in 150 ml chloroform over 10 minutes. The reaction solution is left to stand at room temperature for 30 minutes and is then concentrated under reduced pressure. The residue is purified by flash chromatography (SiO₂ 60F) to provide the title compound as a grey solid. Rf = 0.55 (EtOAc-heptane 1:1); Rt = 4.09.

### Example 125

4-{(2S,4R,5R)-4-(4-Methoxy-phenyl)-5-[4-(3-methoxy-propyl)-3,4-dihydro-2H-benzo[1,4]oxazin-6-ylmethoxy]-piperidin-2-yl}-2-methyl-butan-2-ol
According to general procedure A, 327 mg of 4-[(2S,4R,5R)-4-(4-methoxy-phenyl)-5-[4-(3-methoxy-propyl)-3,4-dihydro-2H-benzo[1,4]oxazin-6-ylmethoxy]-1-(toluene-4-sulfonyl)-piperidin-2-yl]-2-methyl-butan-2-ol are used to afford the title compound.

The starting materials are prepared as follows:
a) 4-[(2S,4R,5R)-4-(4-Methoxy-phenyl)-5-[4-(3-methoxy-propyl)-3,4-dihydro-2H-benzo[1,4]oxazin-6-ylmethoxy]-1-(toluene-4-sulfonyl)-piperidin-2-yl]-2-methylbutan-2-ol
   Similar to example 29b, 200 mg of 4-[(2S,4R,5R)-4-(4-methoxy-phenyl)-5-[4-(3-methoxy-propyl)-3,4-dihydro-2H-benzo[1,4]oxazin-6-ylmethoxy]-1-(toluene-4-sulfonyl)-piperidin-2-yl]-butan-2-one and 0.5 ml of methylmagnesium bromide (1 N solution in tetrahydrofuran) are used to afford the title compound as a beige oil. Rf = 0.12 (EtOAc-heptane 1:1); Rt = 5.14.
b) 4-[(2S,4R,5R)-4-(4-Methoxy-phenyl)-5-[4-(3-methoxy-propyl)-3,4-dihydro-2H-benzo[1,4]oxazin-6-ylmethoxy]-1-(toluene-4-sulfonyl)-piperidin-2-yl]-butan-2-one
   Similar to example 29b, 1.68 g of N-methoxy-3-[(2S,4R,5R)-4-(4-methoxy-phenyl)-5-[4-(3-methoxy-propyl)-3,4-dihydro-2H-benzo[1,4]oxazin-6-ylmethoxy]-1-(toluene-4-sulfonyl)-piperidin-2-yl]-N-methyl-propionamide and 4.3 ml of of methylmagnesium bromide (1 N solution in tetrahydrofuran) are used to afford the title compound as a colourless oil. Rf = 0.23 (EtOAc-heptane 1:1); Rt = 5.25.
c) N-Methoxy-3-[(2S,4R,5R)-4-(4-methoxy-phenyl)-5-[4-(3-methoxy-propyl)-3,4-dihydro-2H-benzo[1,4]oxazin-6-ylmethoxyl-1-(toluene-4-sulfonyl)-piperidin-2-yl]-N-methyl-propionamide
   According to general procedure D, 1.65 g of 3-[(2S,4R,5R)-4-(4-methoxy-phenyl)-5-[4-(3-methoxy-propyl)-3,4-dihydro-2H-benzo[1,4]oxazin-6-ylmethoxy]-1-(toluene-4-sulfonyl)-piperidin-2-yl]-propionic acid and 271 mg of N,O-dimethylhydroxylamine hydrochloride are used to afford the title compound as a beige oil. Rf = 0.18 (EtOAc-heptane 2:1 ); Rt = 5.09.
d) 3-[(2S,4R,5R)-4-(4-Methoxy-phenyl)-5-[4-(3-methoxy-propyl)-3,4-dihydro-2H-benzo[1,4]oxazin-6-ylmethoxy]-1-(toluene-4-sulfonyl)-piperidin-2-yl]-propionic acid
   According to general procedure H, 1.62 g of 3-[(2S,4R,5R)-4-(4-methoxy-phenyl)-5-[4-(3-methoxy-propyl)-3,4-dihydro-2H-benzo[1,4]oxazin-6-ylmethoxy]-1-(toluene-4-sulfonyl)-piperidin-2-yl]-propionitrile are used to afford the title compound as a brown oil. Rf = 0.25 (EtOAc); Rt = 4.85.
e) 3-[(2S,4R,5R)-4-(4-Methoxy-phenyl)-5-[4-(3-methoxy-propyl)-3,4-dihydro-2H-benzo[1,4]oxazin-6-ylmethoxyl-1-(toluene-4-sulfonyl)-piperidin-2-yl]-2,2-dimethylpropionitrile
   A suspension of 500 mg 3-[(2S,4R,5R)-4-(4-methoxy-phenyl)-5-[4-(3-methoxy-propyl)-3,4-dihydro-2H-benzo[1,4]oxazin-6-ylmethoxy]-1-(toluene-4-sulfonyl)-piperidin-2-yl]-2,2-dimethyl-propionic acid (from example 116b), 111.2 mg ammonium chloride and 0.7 ml triethylamine in 5 ml tetrahydrofuran are cooled to -10°C and 1.21 ml of a T3P^{®} solution (50% in ethyl acetate) is added dropwise. The reaction mixture is warmed to room temperature and stirred for 8 hours. The resulting suspension is stirred at 70°C for 2 days. During this time, the addition of 111.2 mg ammonium chloride, 0.7 ml triethylamine and 1.21 ml of a T3P^{®} solution (50% in ethyl acetate) is repeated twice. The reaction mixture is quenched by addition of 5 ml of water and extracted with 50 ml ethyl acetate (3x). The combined organic layers are washed with brine and dried with sodium sulfate. The organic layer is filtered and evaporated under reduced pressure. The residue is purified by flash chromatography (SiO₂ 60F) to afford the title compound as a colourless foam. Rf = 0.12 (EtOAc-heptane 1:2); Rt=5.42.

### Example 126

6-[(3R,4R,6S)-6-(3(R,S)-Methoxy-butyl)-4-(4-methoxy-phenyl)-piperidin-3-yloxymethyl]-4-(3-methoxy-propyl)-3,4-dihydro-2H-benzo[1,4]oxazine (diastereomeric mixture)
According to general procedure A, 210 mg of 6-[(3R,4R,6S)-6-(3-methoxy-butyl)-4-(4-methoxy-phenyl)-1-(toluene-4-sulfonyl)-piperidin-3-yloxymethyl]-4-(3-methoxy-propyl)-3,4-dihydro-2H-benzo[1,4]oxazine (diastereomeric mixture) are used to afford the title compound.

The starting materials are prepared as follows:
a) 6-[(3R,4R,6S)-6-(3-Methoxy-butyl)-4-(4-methoxy-phenyl)-1-(toluene-4-sulfonyl)-piperidin-3-yloxymethyl]-4-(3-methoxy-propyl)-3,4-dihydro-2H-benzo[1,4]oxazine (diastereomeric mixture)
   Similar to example 30a, 550 mg of 4-[(2S,4R,5R)-4-(4-methoxy-phenyl)-5-[4-(3-methoxy-propyl)-3,4-dihydro-2H-benzo[1,4]oxazin-6-ylmethoxy]-1-(toluene-4-sulfonyl)-piperidin-2-yl]-butan-2-ol (diastereomeric mixture) are used to afford the title compound as a yellow oil. Rf = 0.30 (EtOAc-heptane 1:1); Rt = 5.60.
b) 4-[(2S,4R,5R)-4-(4-Methoxy-phenyl)-5-[4-(3-methoxy-propyl)-3,4-dihydro-2H-benzo[1,4]oxazin-6-ylmethoxy]-1-(toluene-4-sulfonyl)-piperidin-2-yl]-butan-2-ol (diastereomeric mixture)
   Similiar to procedure 29a, 800 mg of 4-[(2S,4R,5R)-4-(4-methoxy-phenyl)-5-[4-(3-methoxy-propyl)-3,4-dihydro-2H-benzo[1,4]oxazin-6-ylmethoxy]-1-(toluene-4-sulfonyl)-piperidin-2-yl]-butan-2-one and 3.0 ml of borane-tetrahydrofuran complex (1 N in tetrahydrofuran) are used to afford the title compound as a yellow oil. Rf = 0.22 (EtOAc-heptane 2:1); Rt = 5.00.
c) 4-[(2S,4R,5R)-4-(4-Methoxy-phenyl)-5-[4-(3-methoxy-propyl)-3,4-dihydro-2H-benzo[1,4]oxazin-6-ylmethoxy]-1-(toluene-4-sulfonyl)-piperidin-2-yl]-butan-2-one
   Similar to example 29b, 1.68 g of N-methoxy-3-[(2S,4R,5R)-4-(4-methoxy-phenyl)-5-[4-(3-methoxy-propyl)-3,4-dihydro-2H-benzo[1,4]oxazin-6-ylmethoxy]-1-(toluene-4-sulfonyl)-piperidin-2-yl]-N-methyl-propionamide in the presence of methyl magnesium bromide are used to afford the title compound after flash chromatography (SiO₂ 60F) as a colourless oil. Rf = 0.23 (EtOAc-heptane 1:1); Rt = 5.25.
d) N-Methoxy-3-[(2S,4R,5R)-4-(4-methoxy-phenyl)-5-[4-(3-methoxy-propyl)-3,4-dihydro-2H-benzo[1,4]oxazin-6-ylmethoxyl-1-(toluene-4-sulfonyl)-piperidin-2-yl]-N-methyl-propionamide
   According to general procedure D, 1.65 g of 3-[(2S,4R,5R)-4-(4-methoxy-phenyl)-5-[4-(3-methoxy-propyl)-3,4-dihydro-2H-benzo[1,4]oxazin-6-ylmethoxy]-1-(toluene-4-sulfonyl)-piperidin-2-yl]-propionic acid are reacted with N,O-dimethylhydroxylamine hydrochloride to afford the title compound as a grey oil. Rf = 0.18 (EtOAc-heptane 5:1); Rt = 5.09.
e) 3-[(2S,4R,5R)-4-(4-Methoxy-phenyl)-5-[4-(3-methoxy-propyl)-3,4-dihydro-2H-benzo[1,4]oxazin-6-ylmethoxy]-1-(toluene-4-sulfonyl)-piperidin-2-yl]-propionic acid
   According to general procedure H, 1.62 g of 3-[(2S,4R,5R)-4-(4-methoxy-phenyl)-5-[4-(3-methoxy-propyl)-3,4-dihydro-2H-benzo[1,4]oxazin-6-ylmethoxy]-1-(toluene-4-sulfonyl)-piperidin-2-yl]-propionitrile are used to afford the title compound as a brown oil. Rf = 0.25 (EtOAc); Rt = 4.85.
f) 3-[(2S,4R,5R)-4-(4-Methoxy-phenyl)-5-[4-(3-methoxy-propyl)-3,4-dihydro-2H-benzo[1,4]oxazin-6-ylmethoxy]-1-(toluene-4-sulfonyl)-piperidin-2-yl]-propionitrile
   According to general procedure G, 2.75 g of methanesulfonic acid 2-[(2R,4R,5R)-4-(4-methoxy-phenyl)-5-[4-(3-methoxy-propyl)-3,4-dihydro-2H-benzo[1,4]oxazin-6-ylmethoxy]-1-(toluene-4-sulfonyl)-piperidin-2-yl]-ethyl ester are used to afford the title compound as a colourless oil.
g) Methanesulfonic acid 2-[(2R,4R,5R)-4-(4-methoxy-phenyl)-5-[4-(3-methoxy-propyl)-3,4-dihydro-2H-benzof 1,4loxazin-6-ylmethoxyl-1-(toluene-4-sulfonyl)-piperidin-2-yll-ethyl ester
   According to general procedure F, 200 mg of 2-[(2R,4R,5R)-4-(4-methoxy-phenyl)-5-[4-(3-methoxy-propyl)-3,4-dihydro-2H-benzo[1,4]oxazin-6-ylmethoxy]-1-(toluene-4-sulfonyl)-piperidin-2-yl]-ethanol are reacted to afford the title compound as a yellow oil. Rf = 0.20 (EtOAc-heptane 1:1); Rt = 5.12.
h) 2-[(2R,4R,5R)-4-(4-Methoxy-phenyl)-5-[4-(3-methoxy-propyl)-3,4-dihydro-2H-benzof 1,4loxazin-6-ylmethoxyl-1-(toluene-4-sulfonyl)-piperidin-2-yll-ethanol
   Similar to example 15a, 0.75 g of [(2R,4R,5R)-4-(4-methoxy-phenyl)-5-[4-(3-methoxy-propyl)-3,4-dihydro-2H-benzo[1,4]oxazin-6-ylmethoxy]-1-(toluene-4-sulfonyl)-piperidin-2-yl]-acetic acid (from example 29d) are reduced to afford the title compound obtained as a colourless foam. Rf = 0.1 (EtOAc-heptane 1:1); Rt = 4.83.

### Example 128

1-{(2S,4R,5R)-4-(4-Methoxy-phenyl)-5-[4-(3-methoxy-phenyl)-3,4-dihydro-2H-benzo[1,4]oxazin-6-ylmethoxy]-piperidin-2-yl}-2-methyl-propan-2-ol
According to general procedure A, 153 mg of 1-[(2S,4R,5R)-4-(4-Methoxy-phenyl)-5-[4-(3-methoxy-propyl)-3,4-dihydro-2H-benzo[1,4]oxazin-6-ylmethoxy]-1-(toluene-4-sulfonyl)-piperidin-2-yl]-2-methyl-propan-2-ol are used to afford the title compound.

The starting materials are prepared as follows:
a) 1-[(2S,4R,5R)-4-(4-Methoxy-phenyl)-5-[4-(3-methoxy-propyl)-3,4-dihydro-2H-benzo[1,4]oxazin-6-ylmethoxyl-1-(toluene-4-sulfonyl)-piperidin-2-yl]-2-methylpropan-2-ol
   Similar to example 29b, 1.56 g of 1-[4-(4-methoxy-phenyl)-5-[(2S,4R,5R)-4-(3-methoxy-propyl)-3,4-dihydro-2H-benzo[1,4]oxazin-6-ylmethoxy]-1-(toluene-4-sulfonyl)-piperidin-2-yl]-propan-2-one are used to afford the title compound as a yellow oil. Rf = 0.18 (EtOAc-heptane 1:1 ); Rt = 5.14.
b) 1-[(2S,4R,5R)-4-(4-Methoxv-ₗphenyl)-5-[4-(3-methoxy-_{ID}ro_{lD}yl)-3,4-dihydro-2H-benzo[1,4]oxazin-6-ylmethoxy]-1-(toluene-4-sulfonyl)-piperidin-2-yl]-propan-2-one
   To a stirred solution of 4.29 g of 1-[(2S,4R,5R)-4-(4-methoxy-phenyl)-5-[4-(3-methoxy-propyl)-3,4-dihydro-2H-benzo[1,4]oxazin-6-ylmethoxy]-1-(toluene-4-sulfonyl)-piperidin-2-yl]-propan-2(R;S)-ol (diastereomeric mixture) in 6.0 ml of dimethylsulfoxide and 30 ml of dichloromethane at 0°C are added 4.80 ml of triethylamine and 3.60 g of pyridine sulfur trioxide complex. The reaction mixture is allowed to stir for an additional 3 hours at this temperature and then allowed to warm to room temperature, diluted with water and acidified by the addition of 1 N potassium bisulfate solution and subsequently extracted with diethyl ether. The organic phases are dried, concentrated under reduced pressure and the residue is purified by flash chromatography (SiO₂ 60F) to afford the title compound as a yellow oil. Rf = 0.14 (EtOAc-heptane 1:1).
c) 1-[(2S,4R,5R)-4-(4-Methoxy-phenyl)-5-[4-(3-methoxy-propyl)-3,4-dihydro-2H-benzo[1,4]oxazin-6-ylmethoxyl-1-(toluene-4-sulfonyl)-piperidin-2-yl]-propan-2(R,S)-ol (diastereomeric mixture)
   Similar to example 29b, 4.0 g of [(2S,4R,5R)-4-(4-methoxy-phenyl)-5-[4-(3-methoxy-propyl)-3,4-dihydro-2H-benzo[1,4]oxazin-6-ylmethoxy]-1-(toluene-4-sulfonyl)-piperidin-2-yl]-acetaldehyde are used to afford the title compound as a yellow oil. Rf = 0.53 (EtOAc-heptane 5:1); Rt = 5.05.
d) [(2S,4R,5R)-4-(4-Methoxy-phenyl)-5-[4-(3-methoxy-propyl)-3,4-dihydro-2H-benzo[1,4]oxazin-6-ylmethoxyl-1-(toluene-4-sulfonyl)-piperidin-2-yl]-acetaldehyde
   To a stirred solution of 8.30 g of (2S,4R,5R)-4-(4-methoxy-phenyl)-5-[4-(3-methoxy-propyl)-3,4-dihydro-2H-benzo[1,4]oxazin-6-ylmethoxy]-1-(toluene-4-sulfonyl)-piperidin-2-yl]-acetonitrile in 80 ml of dichloromethane are added 18.7 ml of a solution of diisobutyl aluminium hydride (1.0M in dichloromethane) at -30°C. The reaction mixture is allowed to warm to -5°C over a period of 5 hours and subsequently cooled again to -30°C overnight. The reaction mixture is diluted with dichloromethane, quenched by the addition of 50 ml of 1 N tartaric acid solution and 50 ml of 1 N Rochelles salt solution. The mixture is filtered, concentrated under reduced pressure and the residue is purified by fash chromatography (SiO₂ 60F) to afford the title compound as a brown oil. Rf = 0.29 (EtOAc-heptane 1:1); Rt = 5.14.
e) [(2S,4R,5R)-4-(4-Methoxy-phenyl)-5-[4-(3-methoxy-propyl)-3,4-dihydro-2H-benzo[1,4]oxazin-6-ylmethoxy]-1-(toluol-4-sulfonyl)-piperidin-2-yl]-acetonitril
   According to general procedure G, 0.5 g of methanesulfonic acid (2S,4R,5R)-4-(4-methoxy-phenyl)-5-[4-(3-methoxy-propyl)-3,4-dihydro-2H-benzo[1,4]oxazin-6-ylmethoxy]-1-(toluene-4-sulfonyl)-piperidin-2-ylmethyl ester (from example 29f) are used to afford the title compound. Rf = 0.26 (EtOAc-heptane 1:1); Rt = 5.20.

### Example 134

(R)-1-{(2R,4R,5R)-4-(4-Methoxy-phenyl)-5-[4-(3-methoxy-propyl)-3,4-dihydro-2H-benzo[1,4]oxazin-6-ylmethoxy]-piperidin-2-ylmethyl}-propyl)-carbamic acid methyl ester
According to general procedure A, {(R)-1-[(2R,4R,5R)-4-(4-methoxy-phenyl)-5-[4-(3-methoxy-propyl)-3,4-dihydro-2H-benzo[1,4]oxazin-6-ylmethoxy]-1-(toluene-4-sulfonyl)-piperidin-2-ylmethyl]-propyl}-carbamic acid methyl ester is used to afford the title compound.

The starting materials are prepared as follows:
a) {(R)-1-[(2R,4R,5R)-4-(4-Methoxy-phenyl)-5-[4-(3-methoxv-propyl)-3,4-dihydro-2H-benzo[1,4]oxazin-6-ylmethoxy]-1-(toluene-4-sulfonyl)-piperidin-2-ylmethyl]-propyl}-carbamic acid methyl ester
   To a solution of 403 mg of (R)-1-[(2R,4R,5R)-4-(4-methoxy-phenyl)-5-[4-(3-methoxy-propyl)-3,4-dihydro-2H-benzo[1,4]oxazin-6-ylmethoxy]-1-(toluene-4-sulfonyl)-piperidin-2-ylmethyl]-propylamine in 4 ml of dichloromethane is added 0.4 ml of triethylamine and 0.145 ml of methylchloroformiate at room temperature. The reaction mixture is stirred for 3 hours, diluted with dichloromethane, washed with water and the organic phase is separated, dried and concentrated under reduced pressure to afford the crude material. The crude material is purified by flash chromatography (SiO₂ 60F) to afford the title compound as yellow oil. Rf = 0.47 (EtOAc-heptane 1:1); Rt = 5.23.
b) (R)-1-[(2R,4R,5R)-4-(4-Methoxy-phenyl)-5-[4-(3-methoxy-propyl)-3,4-dihydro-2H-benzo[1,4]oxazin-6-ylmethoxy]-1-(toluene-4-sulfonyl)-piperidin-2-ylmethyl]-propylamine
   According to general procedure E, 1.7 g of 6-[(3R,4R,6R)-6-(R)-2-azido-butyl)-4-(4-methoxy-phenyl)-1-(toluene-4-sulfonyl)-piperidin-3-yloxymethyl]-4-(3-methoxy-propyl)-3,4-dihydro-2H-benzo[1,4]oxazine are hydrogenated to afford the title compound after flash chromatography (SiO₂ 60F) as brown oil. Rf = 0.14 (dichloromethane-methanol-25% ammonia conc. 200:10:1); Rt = 4.52.
c) 6-[(3R,4R,6R)-6-(R)-2-Azido-butyl)-4-(4-methoxy-phenyl)-1-(toluene-4-sulfonyl)-piperidin-3-yloxymethyl]-4-(3-methoxy-propyl)-3,4-dihydro-2H-benzo[1,4]oxazine
   To a solution of 2.55 g of methanesulfonic acid (S)-1-[(2R,4R,5R)-4-(4-methoxyphenyl)-5-[4-(3-methoxy-propyl)-3,4-dihydro-2H-benzo[1,4]oxazin-6-ylmethoxy]-1-(toluene-4-sulfonyl)-piperidin-2-ylmethyl]-propyl ester in 5.0 ml N,N dimethylformamid is added 1.81 g of sodium azide and the mixture is heated to 80°C overnight. The reaction mixture is dilutred with water and extracted with tert-butyl methyl ether. The organic phase are combined and concentrated under reduced pressure to afford the crude title compound which is purified by fash chromatography (SiO₂ 60F) to afford the title compound as yellow oil. Rf = 0.14 (EtOAc-heptane 1:1); Rt = 4.52.
d) Methanesulfonic acid (S)-1-[(2R,4R,5R)-4-(4-methoxy-phenyl)-5-[4-(3-methoxy-propyl)-3,4-dihydro-2H-benzo[1,4]oxazin-6-ylmethoxy]-1-(toluene-4-sulfonyl)-piperidin-2-ylmethyl]-propyl ester
   According to general procedure F, 100 mg of (S)-1-[(2R,4R,5R)-4-(4-methoxyphenyl)-5-[4-(3-methoxy-propyl)-3,4-dihydro-2H-benzo[1,4]oxazin-6-ylmethoxy]-1-(toluene-4-sulfonyl)-piperidin-2 -yl]-butan-2-ol (diastereomer 2) are used to afford the title compound as a brown oil. Rt = 5.22.
e) (R)-1-[(2R,4R,5R)-4-(4-Methoxy-phenyl)-5-[4-(3-methoxy-propyl)-3,4-dihydro-2H-benzo[1,4]oxazin-6-ylmethoxy]-1-(toluene-4-sulfonyl)-piperidin-2-yl]-butan-2-ol (diastereomer 1) and
   (S)-1-[(2R,4R,5R)-4-(4-Methoxy-phenyl)-5-[4-(3-methoxy-propyl)-3,4-dihydro-2H-benzof 1,4loxazin-6-ylmethoxyl-1-(toluene-4-sulfonyl)-piperidin-2-yll-butan-2-ol (diastereomer 2)
   Similar to example 29a, 1-[(2R,4R,5R)-4-(4-methoxy-phenyl)-5-[4-(3-methoxy-propyl)-3,4-dihydro-2H-benzo[1,4]oxazin-6-ylmethoxy]-1-(toluene-4-sulfonyl)-piperidin-2-yl]-butan-2-one are used to afford the title compounds. The diastereomers are separated by flash chromatography (SiO₂ 60F).
   Diastereomer 1: colourless oil; Rf = 0.60 (EtOAc-heptan 5:1); Rt = 5.11;
   Diastereomer 2: colourless oil; Rf = 0.50 (EtOAc-heptan 1:1); Rt = 5.01;
f) 1-[(2R,4R,5R)-4-(4-Methoxy-phenyl)-5-[4-(3-methoxy-propyl)-3,4-dihydro-2H-benzo[1,4]oxazin-6-ylmethoxy]-1-(toluene-4-sulfonyl)-piperidin-2-yl]-butan-2-one
   Similar to example 29b, 1.0 g of N-methoxy-2-[4-(4-methoxy-phenyl)-5-[4-(3-methoxy-propyl)-3,4-dihydro-2H-benzo[1,4]oxazin-6-ylmethoxy]-1-(toluene-4-sulfonyl)-piperidin-2-yl]-N-methyl-acetamide (from example 29c) in the presence of ethyl magnesium bromide are used to afford the title compound after flash chromatography (SiO₂ 60F) as a colourless oil. Rf = 0.20 (EtOAc-heptane 1:1); Rt = 5.13.

### Example 135

6-[(3R,4R,6S)-6-(2-Methoxy-2-methyl-propyl)-4-(4-methoxy-phenyl)-piperidin-3-yloxymethyll-4-(3-methoxy-propyl )-3,4-d ihydro-2 H-benzof 1,4loxazine
According to general procedure A, 100 mg of 6-[(3R,4R,6S)-6-(2-methoxy-2-methylpropyl)-4-(4-methoxy-phenyl)-1-(toluene-4-sulfonyl)-piperidin-3-yloxymethyl]-4-(3-methoxy-propyl)-3,4-dihydro-2H-benzo[1,4]oxazine are used to afford the title compound.

The starting material is prepared as follows:
a) 6-[(3R,4R,6S)-6-(2-Methoxy-2-methyl-propyl)-4-(4-methoxy-phenyl)-1-(toluene-4-sulfonyl)-piperidin-3-yloxymethyl]-4-(3-methoxy-propyl)-3,4-dihydro-2H-benzo[1,4]oxazine
   To a solution of 60 mg of 1-{(2S,4R,5R)-4-(4-methoxy-phenyl)-5-[4-(3-methoxy-propyl)-3,4-dihydro-2H-benzo[1,4]oxazin-6-ylmethoxy]-piperidin-2-yl}-2-methylpropan-2-ol (from example 128) in N,N-dimethylformamide is added 25 mg of sodium hydride and 0.2 ml of methyl iodide. The reaction mixture is stirred for 2 days at room temperature, acidified with 1 N hydrochloric acid and extracted with tert-butyl methyl ether. The organic phases are combined, dried and concentrated under reduced pressure. The residue is purified by flash chromatography (Si0₂ 60F) to afford the title compound as yellow oil. Rf = 0.21 (EtOAc-heptane 1:1); Rt = 5.70.

### Example 138

N-((S)-2-Hydroxy-propyl)-3-{(2S,4R,5R)-4-(4-methoxymethyl-phenyl)-5-[4-(3-methoxy-propyl)-3,4-dihydro-2H-benzo[1,4]oxazin-6-ylmethoxy]-piperidin-2-yl}-2,2-dimethyl-propionamide According to general procedure A, N-((S)-2-hydroxy-propyl)-3-[(2S,4R,5R)-4-(4-methoxymethyl-phenyl)-5-[4-(3-methoxy-propyl)-3,4-dihydro-2H-benzo[1,4]oxazin-6-ylmethoxy]-1-(toluene-4-sulfonyl)-piperidin-2-yl]-2,2-dimethyl-propionamide is used to afford the title compound, which is identified based on the Rf value.

The starting materials are prepared as follows:
a) N-((S)-2-Hydroxy-propyl)-3-f(2S,4R,5R)-4-(4-methoxymethyl-phenyl)-5-f4-(3-methoxy-propyl)-3,4-d ihydro-2 H-benzof 1,4loxazin-6-yl methoxyl-1-(tol uene-4-sulfonyl)-piperidin-2-yl]-2,2-dimethyl-propionamide
   According to general procedure D, 3-[(2S,4R,5R)-4-(4-methoxymethyl-phenyl)-5-[4-(3-methoxy-propyl)-3,4-dihydro-2H-benzo[1,4]oxazin-6-ylmethoxy]-1-(toluene-4-sulfonyl)-piperidin-2-yl]-2,2-dimethyl-propionic acid and (S)-1-amino-propan-2-ol [2799-17-9]are used to afford the title compound which is identified based on the Rf value.
b) 3-[(2S,4R,5R)-4-(4-Methoxymethyl-phenyl)-5-[4-(3-methoxy-propyl)-3,4-dihydro-2H-benzo[1,4]oxazin-6-ylmethoxy]-1-(toluene-4-sulfonyl)-piperidin-2-yl]-2,2-dimethyl-propionic acid
   According to the procedure described in example 116b-c, 6-[(3R,4R,6S)-6-bromomethyl-4-(4-methoxymethyl-phenyl)-1-(toluene-4-sulfonyl)-piperidin-3-yloxymethyl]-4-(3-methoxy-propyl)-3,4-dihydro-2H-benzo[1,4]oxazine is used to afford the title compound, which is identified based on the Rf value.
c) 6-[(3R,4R,6S)-6-Bromomethyl-4-(4-methoxymethyl-phenyl)-1-(toluene-4-sulfonyl)-piperidin-3-yloxymethyl]-4-(3-methoxy-propyl)-3,4-dihydro-2H-benzo[1 ,4]oxazine
   According to example 116d, methanesulfonic acid (2S,4R,5R)-4-(4-methoxymethylphenyl)-5-[4-(3-methoxy-propyl)-3,4-dihydro-2H-benzo[1,4]oxazin-6-ylmethoxy]-1-(toluene-4-sulfonyl)-piperidin-2-ylmethyl ester is used to afford the title compound, which is identified based on the Rf value.
d) Methanesulfonic acid (2S,4R,5R)-4-(4-methoxymethyl-phenyl)-5-[4-(3-methoxy-propyl)-3,4-dihydro-2H-benzo[1,4]oxazin-6-ylmethoxy]-1-(toluene-4-sulfonyl)-piperidin-2-ylmethyl ester
   According to general procedure F, [(2S,4R,5R)-4-(4-methoxymethyl-phenyl)-5-[4-(3-methoxy-propyl)-3,4-dihydro-2H-benzo[1,4]oxazin-6-ylmethoxy]-1-(toluene-4-sulfonyl)-piperidin-2-yl]-methanol is used to afford the title compound, which is identified based on the Rf value.
e) [(2S,4R,5R)-4-(4-methoxymethyl-phenyl)-5-[4-(3-methoxy-propyl)-3,4-dihydro-2H-benzo[1,4]oxazin-6-ylmethoxy]-1-(toluene-4-sulfonyl)-piperidin-2-yl]-methanol
   According to example 29g, (alternative synthesis) 6-[(3R,4R,6S)-4-(4-methoxymethylphenyl)-1-(toluene-4-sulfonyl)-6-triisopropylsilanyloxymethyl-piperidin-3-yloxymethyl]-4-(3-methoxy-propyl)-3,4-dihydro-2H-benzo[1,4]oxazine is used to afford the title compound, which is identified based on the Rf value.
f) 6-[(3R,4R,6S)-4-(4-Methoxymethyl-phenyl)-1-(toluene-4-sulfonyl)-6-triisopropylsilanyloxymethyl-piperidin-3-yloxymethyl]-4-(3-methoxy-propyl)-3,4-dihydro-2H-benzo[1,4]oxazine
   According to general procedure I, 25.83 g {4-[(2S,4R,5R)-5-[4-(3-methoxy-propyl)-3,4-dihydro-2H-benzo[1,4]oxazin-6-ylmethoxy]-1-(toluene-4-sulfonyl)-2-triisopropylsilanyloxymethyl-piperidin-4-yl]-phenyl}-methanol and methyliodide are used to afford the title compound, which is identified based on the Rf value.
g) {4-[(2S,4R,5R)-5-[4-(3-Methoxy-propyl)-3,4-dihydro-2H-benzo[1,4]oxazin-6-ylmethoxy]-1-(toluene-4-sulfonyl)-2-triisopropylsilanyloxymethyl-piperidin-4-yl]-phenyl}-methanol
   According to general procedure C, 4-[(2S,4R,5R)-5-[4-(3-methoxy-propyl)-3-oxo-3,4-dihydro-2H-benzo[1,4]oxazin-6-ylmethoxy]-1-(toluene-4-sulfonyl)-2-triisopropylsilanyloxymethyl-piperidin-4-yl]-benzoic acid is used to afford the title compound as a yellow oil. Rf = 0.19 (EtOAc-heptane 1:1); Rt = 6.03.
h) 4-[(2S,4R,5R)-5-[4-(3-Methoxy-propyl)-3-oxo-3,4-dihydro-2H-benzo[1,4]oxazin-6-ylmethoxy]-1-(toluene-4-sulfonyl)-2-triisopropylsilanyloxymethyl-piperidin-4-yl]-benzoic acid
   According to example 116b, 4-[(2S,4R,5R)-5-[4-(3-methoxy-propyl)-3-oxo-3,4-dihydro-2H-benzo[1,4]oxazin-6-ylmethoxy]-1-(toluene-4-sulfonyl)-2-triisopropylsilanyloxymethyl-piperidin-4-yl]-benzoic acid methyl ester is used to afford the title compound as a white foam. Rf = 0.20 (EtOAc-heptane 1:1); Rt = 6.30.
i) 4-[(2S,4R,5R)-5-[4-(3-Methoxy-propyl)-3-oxo-3,4-dihydro-2H-benzo[1,4]oxazin-6-ylmethoxy]-1-(toluene-4-sulfonyl)-2-triisopropylsilanyloxymethyl-piperidin-4-yl]-benzoic acid methyl ester
   According to example 29g (alternative Ab), 33.24 g 4-[(2S,4R,5R)-5-hydroxy-1-(toluene-4-sulfonyl)-2-triisopropylsilanyloxymethyl-piperidin-4-yl]-benzoic acid methyl ester are used to afford the title compound as a yellow oil. Rf = 0.43 (EtOAc-heptane 1:1); Rt = 6.79.
j) 4-[(2S,4R,5R)-5-Hydroxy-1-(toluene-4-sulfonyl)-2-triisopropylsilanyloxymethylpiperidin-4-yl]-benzoic acid methyl ester
   According to example 24g (alternative Ac), 24.19 g 4-[(2S,4R,5R)-5-hydroxy-2-hydroxymethyl-1-(toluene-4-sulfonyl)-piperidin-4-yl]-benzoic acid methyl ester are used to afford the title compound as a yellow oil. Rf = 0.45 (EtOAc-heptane 1:1); Rt = 6.46.
k) 4-[(2S,4R,5R)-5-Hydroxy-2-hydroxymethyl-1-(toluene-4-sulfonyl)-piperidin-4-yl]-benzoic acid methyl ester
   To a solution of 6 g of trifluoro-methanesulfonic acid 4-[(2S,4R,5R)-5-hydroxy-2-hydroxymethyl-1-(toluene-4-sulfonyl)-piperidin-4-yl]-phenyl ester in 42 ml of N,N-dimethylformamide and 32 ml of methanol is added 3.1 ml of triethylamin, 245 mg of palladium(li) acetate and 454 mg of 1,3-bisdiphenylphosphinopropane. The reaction mixture is charged into an autoclave and subjected to a pressure of carbon monoxide of 70 bars and subsequently heated to 70°C. The reaction mixture is kept for 5 hours under those reaction conditions followed by concentrating the mixture under reduced pressure. The residue is purified by flash chromatography (SiO₂ 60F) to afford the title compound as a dark yellow oil. Rf = 0.38 (EtOAc-heptane 1:1); Rt = 3.75.
l) Trifluoro-methanesulfonic acid 4-[(2S,4R,5R)-5-hydroxy-2-hydroxymethyl-1-(toluene-4-sulfonyl)-piperidin-4-yl]-phenyl ester
   To a solution of 29.39 g of (3R,4R,6S)-6-hydroxymethyl-4-(4-hydroxy-phenyl)-1-(toluene-4-sulfonyl)-piperidin-3-ol in 170 ml of dichloromethane are added 29.23 g of N-phenyltrifluoromethane sulfonimide and 11.55 ml of triethylamine. The reaction mixture is stirred for 2 hours at room temperature, diluted with sodium bicarbonate solution and extracted with dichloromethane. The organic phases are combined, dried and concentrated under reduced pressure. The residue is purified by flash chromatography (SiO₂ 60F) to afford the title compound white solid. Rf = 0.27 (EtOAc-heptane 1:1); Rt = 4.46.
m) (3R,4R,6S)-6-Hydroxymethyl-4-(4-hydroxy-phenyl)-1-(toluene-4-sulfonyl)-piperidin-3-ol
   According to general procedure J, 25 g (3R,4R,6S)-6-hydroxymethyl-4-(4-methoxyphenyl)-1-(toluene-4-sulfonyl)-piperidin-3-ol (from example 24g Ad) are used to afford the title compound as a white foam. Rf = 0.20 (EtOAc-heptane 2:1); Rt = 3.22.

According to the processes described in example 138, the following compounds are prepared in an analogous manner:
- 139: N-((R)-2-Hydroxy-propyl)-3-[(2S,4R,5R)-4-(4-methoxymethyl-phenyl)-5-[4-(3-methoxy-propyl)-3,4-dihydro-2H-benzo[1,4]oxazin-6-ylmethoxy]-piperidin-2-yl}-2,2-dimethyl-propionamide
using (R)-1-amino-propan-2-ol [2799-16-8] instead of (S)-1-amino-propan-2-ol [2799-17-9].
- 140: N-(2-Methoxy-ethyl)-3-{(2S,4R,5R)-4-(4-methoxymethyl-phenyl)-5-[4-(3-methoxy-propyl)-3,4-dihydro-2H-benzo[1,4]oxazin-6-ylmethoxy]-piperidin-2-yl}-2,2-dimethyl-propionamide
using 2-methoxy-ethylamine [199-87-3] instead of (S)-1-amino-propan-2-ol [2799-17-9].

### Example 156

(S)-4-{(2S,4R,5R)-4-[4-(3-Methoxy-propoxy)-phenyl]-5-[4-(3-methoxy-propyl)-3,4-dihydro-2H-benzo[1,4]oxazin-6-ylmethoxy]-piperidin-2-yl}-butan-2-ol
According to general procedure A, (S)-4-[(2S,4R,5R)-4-[4-(3-methoxy-propoxy)-phenyl]-5-[4-(3-methoxy-propyl)-3,4-dihydro-2H-benzo[1,4]oxazin-6-ylmethoxy]-1-(toluene-4-sulfonyl)-piperidin-2-yl]-butan-2-ol is used to afford the title compound which is identified based on its Rf value.

The starting materials are prepared as follows:
a) (R)-4-[(2S,4R,5R)-4-[4-(3-Methoxy-propoxy)-phenyl]-5-[4-(3-methoxy-propyl)-3,4-dihydro-2H-benzo[1,4]oxazin-6-ylmethoxyl-1-(toluene-4-sulfonyl)-piperidin-2-yl]-butan-2-ol
   and
   (S)-4-[(2S,4 R,SR)-4-[4-(3-Methoxy-propoxy)-phenyl]-5-[4-(3-methoxy-propyl)-3,4-dihydro-2H-benzof 1,4loxazin-6-ylmethoxyl-1-(toluene-4-sulfonyl)-piperidin-2-yll-butan-2-ol
   According to general procedure C, 4-[(2S,4R,5R)-4-[4-(3-methoxy-propoxy)-phenyl]-5-[4-(3-methoxy-propyl)-3,4-dihydro-2H-benzo[1,4]oxazin-6-ylmethoxy]-1-(toluene-4-sulfonyl)-piperidin-2-yl]-butan-2-one is used to afford the title compounds which are identified based on its Rf value.
b) 4-[(2S,4R,5R)-4-[4-(3-Methoxv-ₗproₗpoxy)-_{I}phenyll-5-[4-(3-methoxy-_{ID}ro_{lD}yl)-3,4-dihydro-2H-benzof 1,4loxazin-6-ylmethoxyl-1-(toluene-4-sulfonyl)-piperidin-2-yll-butan-2-one
   According to the procedure described in example 29b-c, 3-[(2S,4R,5R)-4-[4-(3-methoxy-propoxy)-phenyl]-5-[4-(3-methoxy-propyl)-3,4-dihydro-2H-benzo[1,4]oxazin-6-ylmethoxy]-1-(toluene-4-sulfonyl)-piperidin-2-yl]-propionic acid is used to afford the title compound which is identified based on its Rf value.
c) 3-[(2S,4R,5R)-4-[4-(3-Methoxy-propoxy)-phenyl]-5-[4-(3-methoxy-propyl)-3,4-dihydro-2H-benzo[1,4]oxazin-6-ylmethoxyl-1-(toluene-4-sulfonyl)-piperidin-2-yl]-propionic acid
   According to the procedure described in the general procedures H and G methanesulfonic acid 2-[(2R,4R,5R)-4-[4-(3-methoxy-propoxy)-phenyl]-5-[4-(3-methoxy-propyl)-3,4-dihydro-2H-benzo[1,4]oxazin-6-ylmethoxy]-1-(toluene-4-sulfonyl)-piperidin-2-yl]-ethyl ester is used to afford the title compound which is identified based on its Rf value.
d) Methanesulfonic acid 2-[(2R,4R,5R)-4-[4-(3-methoxy-propoxy)-phenyl]-5-[4-(3-methoxy-propyl)-3,4-dihydro-2H-benzo[1,4]oxazin-6-ylmethoxyl-1-(toluene-4-sulfonyl)-piperidin-2-yl]-ethyl ester
   According to general procedure F, 2-[(2R,4R,5R)-4-[4-(3-methoxy-propoxy)-phenyl]-5-[4-(3-methoxy-propyl)-3,4-dihydro-2H-benzo[1,4]oxazin-6-ylmethoxy]-1-(toluene-4-sulfonyl)-piperidin-2-yl]-ethanol is used to afford the title compound which is identified based on its Rf value.
e) 2-[(2R,4R,5R)-4-[4-(3-Methoxy-propoxy)-phenyl]-5-[4-(3-methoxy-propyl)-3,4-dihydro-2H-benzo[1,4]oxazin-6-ylmethoxyl-1-(toluene-4-sulfonyl)-piperidin-2-yl]-ethanol
   According to general procedure C, [(2R,4R,5R)-4-[4-(3-methoxy-propoxy)-phenyl]-5-[4-(3-methoxy-propyl)-3,4-dihydro-2H-benzo[1,4]oxazin-6-ylmethoxy]-1-(toluene-4-sulfonyl)-piperidin-2-yl]-acetic acid is used to afford the title compound which is identified based on its Rf value.
f) [(2R,4R,5R)-4-[4-(3-Methoxy-propoxy)-phenyl]-5-[4-(3-methoxy-propyl)-3,4-dihydro-2H-benzo[1,4]oxazin-6-ylmethoxy]-1-(toluene-4-sulfonyl)-piperidin-2-yl]-acetic acid
   According to example 116b, [(2R,4R,5R)-4-[4-(3-methoxy-propoxy)-phenyl]-5-[4-(3-methoxy-propyl)-3,4-dihydro-2H-benzo[1,4]oxazin-6-ylmethoxy]-1-(toluene-4-sulfonyl)-piperidin-2-yl]-acetic acid 3-methoxy-propyl ester is used to afford the title compound which is identified based on its Rf value.
g) [(2R,4R,5R)-4-[4-(3-Methoxy-propoxy)-phenyl]-5-[4-(3-methoxy-propyl)-3,4-dihydro-2H-benzo[1,4]oxazin-6-ylmethoxy]-1-(toluene-4-sulfonyl)-piperidin-2-yl]-acetic acid 3-methoxy-propyl ester
   According to the procedure described in the general procedures J and K (using 4 equivalents of cesium carbonate and 2.2 equivalents of 3-methoxypropylchloride), 1.17 [(2R,4R,5R)-4-(4-methoxy-phenyl)-5-[4-(3-methoxy-propyl)-3,4-dihydro-2H-benzo[1,4]oxazin-6-ylmethoxy]-1-(toluene-4-sulfonyl)-piperidin-2-yl]-acetic acid methyl ester (from example 51 b) is used to afford the title compound as a yellow oil. Rt = 5.46.

According to the processes described in example 156, the following compound is prepared in an analogous manner:
- 157: (R)-4-{(2S,4R,5R)-4-[4-(3-Methoxy-propoxy)-phenyl]-5-[4-(3-methoxy-propyl)-3,4-dihydro-2H-benzo[1,4]oxazin-6-ylmethoxy]-piperidin-2-yl}-butan-2-ol
starting from (R)-4-[(2S,4R,5R)-4-[4-(3-methoxy-propoxy)-phenyl]-5-[4-(3-methoxy-propyl)-3,4-dihydro-2H-benzo[1,4]oxazin-6-ylmethoxy]-1-(toluene-4-sulfonyl)-piperidin-2-yl]-butan-2-ol (from example 156a).

| **Nr.** | **Structure** | **Appearance** | **R_{f}(System)** | **Rt (Procedure)** |
|---|---|---|---|---|
| 1 | 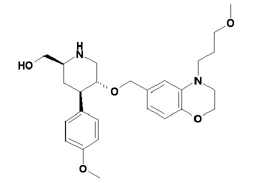 | colourless oil | 0.15 (A) | 3.00 |
| 15 | 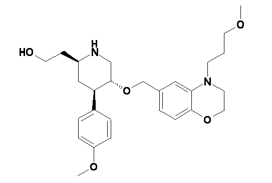 | colourless oil | 0.17 (A) | 3.44 |
| 18 | 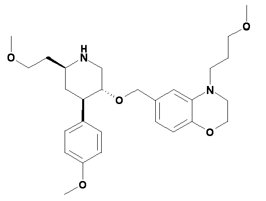 | colourless oil | 0.10 (A) | 3.74 |
| 29 | 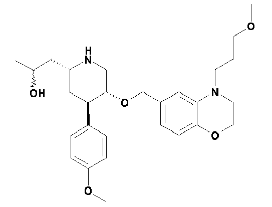 | yellow oil | 0.30 (A) | 3.68/3.74 |
| 30 | 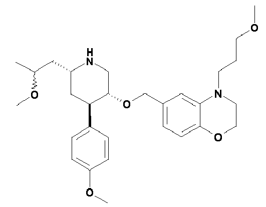 | yellow oil | 0.34 (A) | 4.15 |
| 34 | 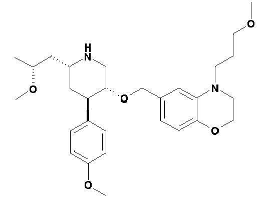 | colourless oil | 0.34 (A) | 4.31 |
| 35 | 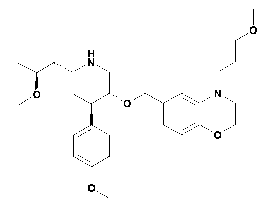 | colourless oil | 0.34 (A) | 4.31 |
| 51 | 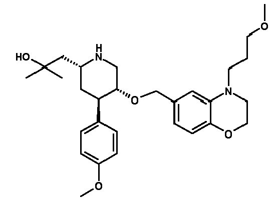 | yellow oil | 0.40 (D) | 3.96 |
| 52 | 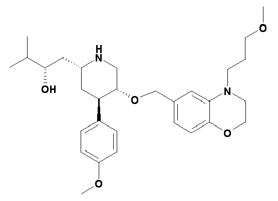 | dark yellow oil | 0.30 (M) | 4.05 |
| 53 | 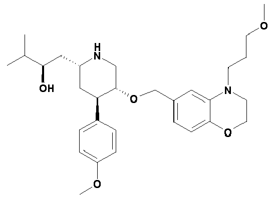 | colorless oil | 0.25 (A) | 4.16 |
| 110 | 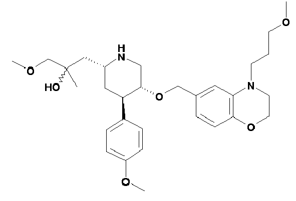 | yellow oil | 0.33 (A) | 3.91 |
| 111 | 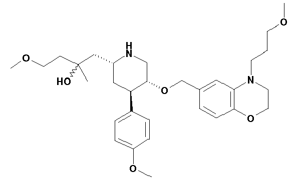 | yellow oil | 0.24 (A) | 4.03 |
| 116 | 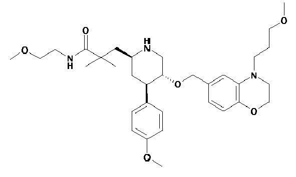 | colourless oil | 0.16 (C) | 3.79 |
| 120 | 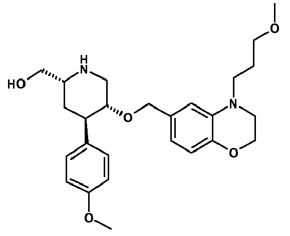 | yellow oil | 0.17 (D) | 3.09 |
| 125 | 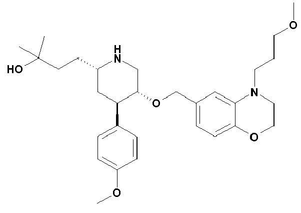 | colourless oil | 0.30 (D) | 3.81 |
| 126 | 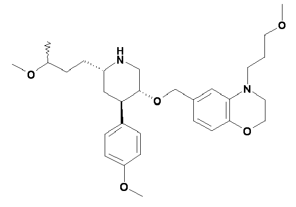 | yellow oil | 0.24 (D) | 4.10 |

### Thin-film chromatography eluent systems:

- A: dichloromethane-methanol = 10:1
- B: EtOAc
- C: dichloromethane-methanol-25% ammonia conc. = 200:10:1
- D: dichloromethane-methanol-25% ammonia conc. = 200:20:1
- E: dichloromethane-methanol-water-acetic acid conc. = 150:54:10:1
- F: dichloromethane-methanol = 20:1
- G: dichloromethane-methanol = 5:1
- H: dichloromethane-methanol = 1:1
- I: EtOAc-heptane = 5:1
- J: dichloromethane-methanol-25% ammonia conc. = 100:10:1
- K: dichloromethane-methanol-25% ammonia conc. = 10:1:0.1
- L: EtOAc-heptane = 1:1
- M: dichloromethane-methanol = 9:1
- N: dichloromethane-methanol-25% ammonia conc. = 40:10:1
- O: dichloromethane-methanol-conc. acetic acid= 100:10:1
- P: dichloromethane-methanol-25% ammonia conc. = 90:10:1
- Q: dichloromethane-methanol-acetic acid conc. = 100:10:2
- R: dichloromethane-methanol = 30:1
- S: dichloromethane-methanol-25% ammonia conc. = 600:20:1
- T: dichloromethane-methanol-25% ammonia conc. = 200:5:1
- U: dichloromethane-methanol-25% ammonia conc. = 5:1:0.1
- V: toluene-EtOAc-triethylamine = 5:3:0.2
- W: dichloromethane-methanol-25% ammonia conc. = 200:15:1
- X: dichloromethane-methanol-25% ammonia conc. = 200:40:1

## Claims

1. Compound of the formula (I) or (II) 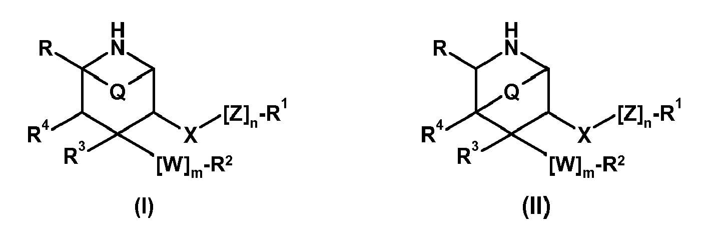 where
R is C₂₋₈-alkenyl, C₁₋₈-alkyl, C₂₋₈-alkynyl, C₀₋₈-alkyl-carbonyl-amino-C₁₋₈-alkyl, C₃₋₈-cycloalkyl-C₀₋₈-alkyl, C₁₋₈-alkyl-sulfonyl-C₁₋₈-alkyl, optionally N-mono- or N,N-di-C₁₋₈-alkylated carbamoyl-C₀₋₈-alkyl, optionally O-C₁₋₈-alkylated carboxyl-C₀₋₈-alkyl, optionally N and/or N' mono-, di- or tri-C₁₋₈-alkylated ureido-C₁₋₈-alkyl or heterocyclylcarbonyl-C₀₋₈-alkyl, each of said radicals being substituted by 1-4 C₁₋₈-alkoxy or hydroxyl;
R¹ is aryl or heterocyclyl;
R² is acenaphthyl, cyclohexyl, diazinyl, furyl, imidazolyl, naphthyl, oxadiazolyl, oxazolyl, phenyl, pyrazinyl, pyridyl, pyrimidinyl, pyrrolyl, oxopyridinyl, tetrazolyl, thienyl, or triazolyl, each of said radicals may be substituted by 1-3 C₁-₈-alkanoyloxy-Cᵢ-₈-al kyl , C₂-₈-alkenyloxy, C₁-₈-alkoxy, C₁₋₈-alkoxy-C₁₋₈-alkoxy, C₁₋₈-alkoxy-C₁₋₈-alkoxy-C₁₋₈-alkyl, C₁₋₈-alkoxy-C₁₋₈-alkyl, C₁₋₈-alkoxycarbonyl, C₁₋₈-alkoxycarbonyloxy-C₁₋₈-alkyl, C₁₋₈-alkyl, carboxy-C₁₋₈-alkyl, C₁₋₈-alkoxy-C₁₋₈-alkylsulfanyl, C₁₋₈-alkylsulfanyl, C₁₋₈-alkylsulfanyl-C₁₋₈-alkoxy, C₁₋₈-alkylsulfanyl-C₁₋₈-alkoxy-C₁₋₈-alkyl, C₁₋₈-alkylsulfanyl-C₁₋₈-alkyl, cyano, cyano-C₁₋₈-alkyl, C₃₋₈-cycloalkyl-C₀₋₆-alkoxy-C₁₋₈-alkoxy, C₁₋₆-alkoxy-C₀₋₆-alkyl-C₃₋₈-cycloalkyl-C₀₋₆-alkoxy-C₁₋₈-alkyl, C₃₋₈-cycloalkyl-C₀-₆-alkoxy-C₁₋₈-alkyl, halo-C₁₋₈-alkyl, halogen, hydroxy-C₁₋₈-alkyl, hydroxyl, oxide, trifluoromethoxy or trifluoromethyl groups, or a C₁₋₈-alkylenedioxy group, and/or by an
L1-T1-L2-T2-L3-T3-L4-T4-L5-U radical;
L1, L2, L3, L4 and L5 are each independently a bond, C₁₋₈-alkylene, C₂₋₈-alkenylene or C₂₋₈-alkynylene, C₃₋₈-cycloalkene or are absent;
T1, T2, T3 and T4 are each independently
(a) a bond, or are absent, or are one of the groups
(b) -CH(OH)-
(c) -CH(OR⁶)-
(d) -CH(NR⁵R⁶)-
(e) -CO-
(f) -CR⁷R⁸-
(g) -O- or -NR⁶-
(h) -S(O)₀₋₂-
(i) -SO₂NR⁶-
(j) -NR⁶SO₂-
(k) -CONR⁶-
(l) -NR⁶CO-
(m) -O-CO-
(n) -CO-O-
(o) -O-CO-O-
(p) -O-CO-NR⁶-
(q) -N(R⁶)-CO-N(R⁶)-
(r) -N(R⁶)-CO-O-
(s) pyrrolidinylene, piperidinylene or piperazinylene
(t) -C(R¹¹)(R¹²)-,
where the bonds starting from (b)-(t) lead to a saturated or aromatic carbon atom of the adjacent group if the bond starts from a heteroatom, and where not more than
two (b)-(f) groups, three (g)-(h) groups and one (i)-(t) group are present;
R³ is hydrogen, hydroxyl, C₁₋₈-alkoxy or C₂-₈-alkenyloxy;
R⁴ is hydrogen, C₂-₈-alkenyl, C₁-₈-alkoxy, C₁-₈-alkoxy-C₁-₈-alkyl, C₁₋₈-alkoxy-C₁₋₈-alkoxy, Cₗ-₈-alkyl, optionally (N-C₁₋₈-alkyl)-C₁₋₈-alkoxycarbonyl-amino-C₁₋₈-alkoxy, optionally (N-C₁₋₈-alkyl)-C₁₋₈-alkylcarbonyl-amino-C₁₋₈-alkoxy, optionally (N-mono- or N,N-di-Cᵢ-C₈-alkyl)-amino-Cᵢ-₈-alkoxy, benzyl, C₃-₈-cycloalkyloxy, C₃₋₈-cycloalkyloxy-C₁₋₈-alkoxy, heterocyclyl-C₀₋₈-alkoxy, heterocydyloxy-C₁₋₈-alkoxy, hydroxy, hydroxy-C₁₋₈-alkoxy-C₁₋₈-alkoxy, hydroxy-C₁₋₈-alkyl, oxo or a
R^{4a}-Z1-X1- group where R^{4a} is
(a) H-
(b) C₁₋₈-alkyl-
(c) C₂₋₈-alkeny-
(d) hydroxy-C₁₋₈-alkyl-
(e) polyhydroxy-C₁₋₈-alkyl-
(f) C₁₋₈-alkyl-O-C₁₋₈-alkyl-
(g) aryl-
(h) heterocyclyl-
(i) arylalkyl-
(j) heterocyclylalkyl-
(k) aryloxyalkyl-
(l) heterocyclyloxyalkyl-
(m) (R⁵,R⁶)N-(CH₂)₁₋₃-
(n) (R⁵,R⁶)N-
(o) C₁₋₈-alkyl-S(O)₀₋₂-
(p) aryl-S(O)₀₋₂-
(q) heterocyclyl-S(O)₀₋₂-
(r) HO-SO₃- or salts thereof
(s) H₂N-C(NH)-NH-
(t) NC-
and the bonds starting from (n)-(t) lead to a carbon atom of the adjacent group and this carbon atom is saturated if the bond starts from a heteroatom;
Z1
(a) is a bond, is absent, or is one of the groups
(b) -C₁₋₈-alkylene-
(c) -C₂₋₈-alkenylene-
(d) -O-, -N(R¹¹)-, -S(O)₀₋₂-
(e) -CO-
(f) -O-CO-
(g) -O-CO-O-
(h) -O-CO-N(R¹¹)-
(i) -N(R¹¹)-CO-O-
(j) -CO-N(R¹¹)-
(k) -N(R¹¹ )-CO-
(l) -N(R¹¹ )-CO-N(R¹¹)-
(m) -CH(_{OR}⁹)₋
and the bonds starting from (d) and (f)-(m) lead to a carbon atom of the adjacent group and this carbon atom is saturated if the bond starts from a heteroatom;
X1
(a) is a bond, is absent, or is one of the groups
(b) -O-
(c) -N(R¹¹)-
(d) -S(O)₀₋₂-
(e) -(CH₂)₁₋₃-;
or R³ and R⁴ in formula (I) together are a bond;
R⁵ and R⁶ are each independently hydrogen, C₁₋₈-alkyl, C₂₋₈-alkenyl, aryl-C₁₋₈-alkyl or acyl, or, together with the nitrogen atom to which they are bonded, are a 5- or 6-membered heterocyclic ring which may contain an additional nitrogen, oxygen or sulfur atom or a -SO- or -SO₂- group, and the additional nitrogen atom may optionally be substituted by C₁₋₈-alkyl radicals;
R⁷ and R⁸, together with the carbon atom to which they are bonded, are a 3-7-membered ring which may contain one or two -O- or -S - atoms or -SO- or -SO₂-groups;
R⁹ is hydrogen, C₁₋₈-alkyl, C₁₋₈-alkoxy-C₁₋₈-alkyl, acyl or arylalkyl;
R¹⁰ is carboxyalkyl, alkoxycarbonylalkyl, alkyl or hydrogen;
R¹¹ is hydrogen or C₁₋₈-alkyl;
R¹² is hydrogen or C₁₋₈-alkyl;
Q is ethylene or is absent (formula I) or is ethylene or methylene (formula II);
U is hydrogen, C₁₋₈-alkyl, cyano, optionally substituted C₃₋₈-cycloalkyl, aryl or heterocyclyl;
W is oxygen or sulfur;
X is a bond, oxygen or sulfur, or is a >CH-R¹¹, >CHOR⁹, -O-CO-, >CO, >C=NOR¹⁰,-O-CHR¹¹- or -O-CHR¹¹-CO-NR⁹- group and the bond starting from an oxygen or sulfur atom leads to a saturated carbon atom of the Z group or to R¹;
Z is C₁₋₈-alkylene, C₂₋₈-alkenylene, hydroxy-C₁₋₈-alkylidene, -O-, -S-, -O-alk-, -S-alk-, - alk-O-, -alk-S- or -alk-NR⁹-, where alk is C₁₋₈-alkylene; and where
(a) if Z is -O- or -S-, X is >CH-R¹¹ and either R² contains an L1-T1-L2-T2-L3-T3-L4-T4-L5-U substituent or R⁴ is a substituent other than hydrogen as defined above;
(b) if Z is -O-alk- or -S-alk-, X is >CH-R¹¹; and
(c) if X is a bond, Z is C₂₋₈-alkenylene, -alk-O- or -alk-S-;
m is 0 or 1;
n is 0 or 1;
or salt or prodrug thereof, or where one or more atoms are replaced by their stable, non-radioactive isotopes.

2. Compound according to Claim 1 of the formula (IA) or (IIA) 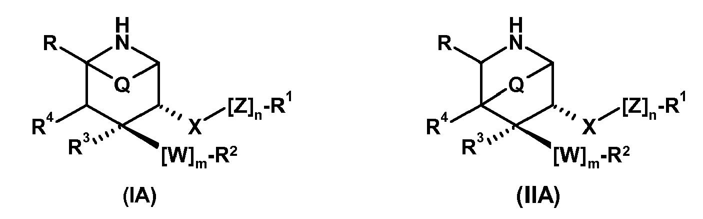 where R, R¹, R², R³, R⁴, Q, W, X, Z, n and m are each as defined for the compounds of the formulae (I) or (II) according to Claim 1.

3. Compound according to Claim 1 or 2 where
R is C₂₋₈-alkenyl, C₁₋₈-alkyl, C₂₋₈-alkynyl, C₀₋₈-alkyl-carbonyl-amino-C₁₋₈-alkyl, C₃₋₈-cycloalkyl-C₀₋₈-alkyl, C₁₋₈-alkyl-sulfonyl-C₀₋₈-alkyl, optionally N-mono- or N,N-di-C₁₋₈-alkylated carbamoyl-C₀₋₈-alkyl, optionally O-C₁₋₈-alkylated carboxyl-C₀₋₈-alkyl, optionally N and/or N' mono-, di- or tri-C₁₋₈-alkylated ureido-C₀₋₈-alkyl or heterocyclylcarbonyl-C₀₋₈-alkyl, each of said radicals being substituted by 1-4 C₁₋₈-alkoxy or hydroxyl;
R¹ is aryl or heterocyclyl;
R² is phenyl, cyclohexyl, tetrazolyl, naphthyl or acenaphthyl, each of said radicals may be unsubstituted or substituted, preferably by 1-3 C₁₋₈-alkanoyloxy-C₁₋₈-alkyl, C₂-₈-alkenyloxy, C₁₋₈-alkoxy, C₁₋₈-alkoxy-C₁₋₈-alkoxy, C₁₋₈-alkoxy-C₁₋₈-alkoxy-C₁₋₈-alkyl, C₁₋₈-alkoxy-C₁₋₈-alkyl, C₁₋₈-alkoxycarbonyl, C₁₋₈-alkoxycarbonyloxy-C₁₋₈-alkyl, C₁₋₈-alkyl, carboxy-C₁₋₈-alkyl, C₁₋₈-alkoxy-C₁₋₈-alkylsulfanyl, C₁₋₈-alkylsulfanyl, C₁₋₈-alkylsulfanyl-C₁₋₈-alkoxy, C₁₋₈-alkylsulfanyl-C₁₋₈-alkoxy-C₁₋₈-alkyl, C₁₋₈-alkylsulfanyl-C₁-₈-alkyl, cyano, cyano-C₁₋₈-alkyl, C₃₋₈-cycloalkyl-C₀₋₆-alkoxy-C₁₋₈-alkoxy, C₁₋₆-alkoxy-C₀-₆-alkyl-C₃₋₈-cycloalkyl-C₀₋₆-alkoxy-C₁₋₈-alkyl, C₃₋₈-cycloalkyl-C₀₋₆-alkoxy-C₁₋₈-alkyl, halo-C₁₋₈-alkyl, halogen, hydroxy-C₁₋₈-alkyl, hydroxyl, oxide, trifluoromethoxy or trifluoromethyl groups, or a C₁₋₈-alkylenedioxy group, and/or by an L1-T1-L2-T2-L3-T3-L4-T4-L5-U radical; or naphthyl or acenaphthyl;
L1, L2, L3, L4 and L5 are each independently a bond, C₁₋₈-alkylene, C₂₋₈-alkenylene or C₂₋₈-alkynylene, C₃₋₈-cycloalkene or are absent;
T1, T2, T3 and T4 are each independently
(a) a bond, or are absent, or are one of the groups
(b) -CH(OH)-
(c) -CH(OR⁶)-
(d) -CH(NR⁵R⁶)-
(e) -CO-
(f) -CR⁷R⁸-
(g) -O- or -NR⁶-
(h) -S(O)₀₋₂-
(i) -SO₂NR⁶-
(j) -NR⁶SO₂-
(k) -CONR⁶-
(l) -NR⁶CO-
(m) -O-CO-
(n) -CO-O-
(o) -O-CO-O-
(p) -O-CO-NR⁶-
(q) -N(R⁶)-CO-N(R⁶)-
(r) -N(R⁶)-CO-O-
(s) pyrrolidinylene, piperidinylene or piperazinylene
(t) -C(R¹¹)(R¹²)-,
where the bonds starting from (b)-(t) lead to a saturated or aromatic carbon atom of the adjacent group if the bond starts from a heteroatom, and where not more than two (b)-(f) groups, three (g)-(h) groups and one (i)-(t) group are present;
R³ is hydrogen, hydroxyl, C₁₋₈-alkoxy or C₂₋₈-alkenyloxy;
R⁴ is hydrogen, C₁₋₈-alkoxy, C₁₋₈-alkoxy-C₁₋₈-alkoxy, C₁₋₈-alkoxy-C₁₋₈-alkyl, C₁₋₈-alkyl, optionally (N-C₁₋₈-alkyl)-C₁₋₈-alkoxycarbonyl-amino-C₁₋₈-alkoxy, optionally (N-C₁₋₈-alkyl)-C₁₋₈-alkylcarbonyl-amino-C₁₋₈-alkoxy, optionally (N-mono- or N,N-di-C₁-C₈-alkyl)-amino-C₁₋₈-alkoxy, C₃₋₈-cycloalkyloxy, C₃₋₈-cycloalkyloxy-C₁₋₈-alkoxy, heterocyclyl-C₀₋₈-alkoxy, heterocyclyloxy-C₁₋₈-alkoxy, hydroxy, oxo or hydroxy-C₁₋₈-alkoxy-C₁₋₈-alkoxy;
R⁵ and R⁶ are each independently hydrogen, C₁₋₈-alkyl or acyl, or, together with the nitrogen atom to which they are bonded, are a 5- or 6-membered heterocyclic ring which may contain an additional nitrogen, oxygen or sulfur atom;
R⁷ and R⁸, together with the carbon atom to which they are bonded, are a 3-7-membered ring which may contain one or two -O- or -S- atoms;
R⁹ is hydrogen, C₁₋₈-alkyl, acyl or arylalkyl;
U is hydrogen, C₁₋₈-alkyl, C₃₋₈-cycloalkyl, cyano, aryl or heterocyclyl;
Q is ethylene or is absent (formula (I)) and is ethylene or methylene (formula (II));
X is a bond, oxygen, sulfur or is a >CHR¹¹, >CHOR⁹, -O-CO-, >CO or -O-CH-R¹¹-CO-NR⁹- group;
W is oxygen or sulfur if R³ is hydrogen;
Z is C₁₋₈-alkylene or -alk-O-;
where, if X is a bond, Z is -alk-O-;
n is 0 or 1;
m is 0 or 1;
or a pharmaceutically useable salt thereof.

4. Compound according to any of Claims 1 to 3 where R² is phenyl or halophenyl each substituted by C₂₋₈-alkenyloxy, C₁₋₈-alkoxy, C₁₋₈-alkoxy-C₁₋₈-alkoxy, C₁₋₈-alkoxy-C₁₋₈-alkoxy-C₁₋₈-alkyl, C₁₋₈-alkoxy-C₁₋₈-alkyl, C₁₋₈-alkoxy-C₁₋₈-alkyl-amino-C₁₋₈-alkyl, C₁₋₈-alkyl, C₁₋₈-alkoxy-C₁₋₈-alkylsulfanyl, C₁₋₈-alkylsulfanyl, C₁₋₈-alkylsulfanyl-C₁₋₈-alkyl, C₁₋₈-alkylsulfanyl-C₁₋₈-alkoxy, C₁₋₈-alkylsulfanyl-C₁₋₈-alkoxy-C₁₋₈-alkyl, C₃₋₈-cycloalkylsulfanyl-C₁₋₈-alkyl, C₁₋₈-alkylsulfonyl-C₁₋₈-alkoxy-C₁₋₈-alkyl, C₃₋₈-cycloalkyl-C₀₋₆-alkoxy, C₃₋₈-cycloalkyl-C₀₋₆-alkoxy-C₁₋₈-alkoxy, C₃₋₈-cycloalkyl-C₀₋₆-alkoxy-C₁₋₈-alkyl, C₁₋₆-alkoxy-C₀₋₆-alkyl-C₃₋₈-cycloalkyl-C₀₋₆-alkoxy-C₁₋₈-alkyl, C₃₋₈-cycloalkyl-C₁₋₈-alkylamino-C₁₋₈-alkyl, halogen, heterocyclyl-C₀₋₆-alkoxy, heterocyclyl-C₀₋₆-alkoxy-C₁₋₈-alkyl, C₁₋₈-alkoxy-C₁₋₈-alkylamino-C₁₋₈-alkyl, N-(halo-phenyl)pyrrolidinyloxy, N-(halophenyl)pyrrolidinyloxy-C₁₋₈-alkyl, C₁₋₈-alkoxybenzyloxy-C₁₋₈-alkoxy, C₁₋₈-alkoxyphenoxy-C₁₋₈-alkoxy, C₁₋₈-alkoxyphenoxy-C₁₋₈-alkyl, C₁₋₈-alkoxyphenyl-C₁₋₈-alkoxy-C₁₋₈-alkoxy, halobenzyloxy-C₁₋₈-alkoxy, halophenoxy-C₁₋₈-alkoxy, halophenoxy-C₁₋₈-alkoxy-C₁₋₈-alkyl, halophenoxy-C₁₋₈-alkyl, halophenyl-C₁₋₈-alkoxy-C₁₋₈-alkoxy or C₁₋₈-alkylbenzyloxy-C₁₋₈-alkoxy, whereby at least one substituent is in the para-position relative to the bond of R² to the rest of the molecule.

5. Compound according to any of Claims 1 to 4 where m is 0.

6. Pharmaceutical preparation comprising a compound of the formula (I), (IA), (II) or (IIA) according to any of Claims 1 to 5.

7. Use of a compound of the formula (I), (IA), (II) or (IIA) according to any of Claims 1 to 5 in the treatment or prevention of hypertension, heart failure, glaucoma, cardiac infarction, kidney failure, restenoses or stroke.

8. Use of a compound of the formula (I), (IA), (II) or (IIA) according to any of Claims 1 to 5 for the preparation of a medicament, preferably a medicament for the treatment or prevention of hypertension, heart failure, glaucoma, cardiac infarction, kidney failure, restenoses or stroke.
